# EUROPÄISCHE PATENTSCHRIFT

(11) **EP 1 817 414 B1**
(45) Veröffentlichungstag und Bekanntmachung des Hinweises auf die Patenterteilung: **14.07.2010**
(21) Anmeldenummer: 05813610.2
(22) Anmeldetag: 29.11.2005
(51) Int. Cl.: C12N 15/11

(54) **VASOPRESSIN BINDENDE L-NUKLEINSÄURE**
VASOPRESSIN-BINDING L-NUCLEIC ACID
ACIDE L-NUCLEIQUE LIANT LA VASOPRESSINE

(30) Priorität: 29.11.2004 DE 102004057523
(43) Veröffentlichungstag der Anmeldung: 15.08.2007
(73) Patentinhaber: Noxxon Pharma AG, 10589 Berlin (DE)
(72) Erfinder: PURSCHKE, Werner, 13359 Berlin (DE); EULBERG, Dirk, 10437 Berlin (DE); KLUSSMANN, Sven, 10709 Berlin (DE); RÖHL, Ingo, 96117 Memmelsdorf (DE); VATER, Axel, 13467 Berlin (DE)
(74) Vertreter: Bohmann, Armin K.
(86) Internationale Anmeldenummer: PCT/EP2005/012744
(87) Internationale Veröffentlichungsnummer: WO 2006/058705

(56) Entgegenhaltungen:
- WO-A-95/07364
- WO-A-96/34879
- WO-A-2004/013274
- WILLIAMS KELLY P ET AL: "Bioactive and nuclease-resistant L-DNA ligand of vasopressin" PROCEEDINGS OF THE NATIONAL ACADEMY OF SCIENCES OF USA, NATIONAL ACADEMY OF SCIENCE, WASHINGTON, DC, US, Bd. 94, Nr. 21, 1997, Seiten 11285-11290, XP002185340 ISSN: 0027-8424 in der Anmeldung erwähnt
- MICHAUD MICKAEL ET AL: "A DNA aptamer as a new target-specific chiral selector for HPLC." JOURNAL OF THE AMERICAN CHEMICAL SOCIETY, Bd. 125, Nr. 28, 16. Juli 2003 (2003-07-16), Seiten 8672-8679, XP002364016 ISSN: 0002-7863
- EULBERG DIRK ET AL: "Spiegelmers: biostable aptamers." CHEMBIOCHEM : A EUROPEAN JOURNAL OF CHEMICAL BIOLOGY. 6 OCT 2003, Bd. 4, Nr. 10, 6. Oktober 2003 (2003-10-06), Seiten 979-983, XP002364015 ISSN: 1439-4227

## Beschreibung

Die vorliegende Erfindung betrifft eine Vasopressin bindende L-Nukleinsäure deren Verwendung zur Herstellung eines Medikamentes sowie diagnostischen Mittels, Komplexe aus der Nukleinsäure und Vasopressin sowie Verfahren zum Screenen von Vasopressin-Antagonisten und Vasopressin-Agonisten.

Humanes Vasopressin, auch bekannt als (Arginin⁸)-Vasopressin (AVP) oder als antidiuretisches Hormon (ADH), sowie Oxytocin sind zyklische, neun Aminosäure lange Peptidhormone. Es handelt sich um Neuropeptide, die nach der Synthese im Hypothalamus aus Vorläuferproteinen prozessiert werden. An ein 10 kDa Carrierprotein gebunden, welches aus demselben Vorläuferprotein prozessiert wird, aus dem das entsprechende Hormon stammt, gelangen sie per intrazellulärem Transport in den Hypophysenhinterlappen, wo sie gespeichert werden und nach entsprechender Stimulation in den Blutstrom ausgeschüttet werden. AVP hat beginnend mit dem Amino-Terminus die Sequenz: Cys-Tyr-Phe-Gln-Asn-Cys-Pro-Arg-Gly-NH₂ (SEQ. ID. NO. 1) mit einem Molekulargewicht von 1085 Da. Oxytocin unterscheidet sich in der Sequenz nur an Position 3 (Ile) und an Position 8 (Lys) vom AVP und hat ein Molekulargewicht von 1007 Da. AVP und Oxytocin sind neben Hypothalamus und Hypophyse auch in Neuronen in anderen Hirnregionen zu lokalisieren. Ihre Rolle als Neurotransmitter steht inzwischen fest (Reghunandanan V et al 1998 Indian. J. Exp. Biol. 36: 635-43, Raggenbass M et al 1998 Prog. Brain. Res. 119: 263-273).

In der Peripherie entfalten die Peptidhormone ihre physiologische Wirkung durch Bindung an spezifische Rezeptoren, die alle aus der großen Familie der G-Protein gekoppelten 7-Transmembranhelix-Rezeptoren stammen, aber deren pharmakologische Profile sowie deren intrazelluläre "second messenger" unterschiedlich sind (Michell RH et al 1979 Biochem. Soc. Trans. 7:861-865; Thibonnier M et al 1998 Adv. Exp. Med. Biol 449 : 251-276).

Die hauptsächliche physiologische Funktion des AVP besteht in der Regulation der freien Wasserresorption in den Nieren und damit der Aufrechterhaltung der Osmolarität der Körperflüssigkeiten und des Blutvolumens. Die grundsätzlich antidiuretische Wirkung wird durch Bindung von AVP an den renalen V₂-Rezeptor vermittelt, welcher auf der basolateralen Membran von Epithelzellen des Sammelrohrs exprimiert wird. Nach Bindung von AVP an den Rezeptor wird intrazellulär das G-Protein G_{S} gebunden, was zur Aktivierung der Adenylyl-Cyklase und zur Synthese des "second messengers" cAMP führt. Daraufhin wird Protein-Kinase A aktiviert, die ihrerseits Proteine phosphoryliert, was wiederum primär zur Insertion von Aquaporin-2 Wasserkanälen (AQP-2) in die dem Innern der Sammelrohre zugewandten Seite der Zellmembran der Epithelzellen führt. Sekundär wird die Transkription der AQP-2 mRNA und die Biosynthese dieses Proteins induziert (Hayashi M et al 1994 J. Clin. Invest. 94: 1778-1783). AQP-2 in der Zellmembran führt zur Resorption des Wassers aus dem Harn in den Organismus. So wird das Harnvolumen verringert und die Osmolarität des Harns steigt an. Ohne den Mechanismus der Resorption würde der Verlust an Wasser innerhalb kürzester Zeit zum Austrocknen und Tod führen. Die Dissoziationskonstante von AVP für die Bindung an den renalen V₂-Rezeptor liegt bei 0,4 nM (Lolait J L et al 1992 Nature 357: 336-339).

Über die Bindung an den V₁-Rezeptor werden eine Vielzahl weiterer physiologischer Funktionen von AVP vermittelt. Der vaskuläre V₁-Rezeptor, der auf der Oberfläche glatter Gefäßmuskelzellen von Blutgefäßen lokalisiert ist, ist an der Regulation des Blutdrucks durch Vasokonstriktion nach Muskelkontraktion der Gefäßmuskelzellen beteiligt. Nach Bindung an den Rezeptor und Aktivierung gekoppelter G Proteine werden die Phospholipasen C, D, und A₂ aktiviert, was zur Bildung der "second messenger" Diacylglycerin und Inositoltriphosphat führt. Simultan werden diverse Proteinkinasen aktiviert und es kommt zur Mobilisierung von intrazellulärem Calcium, zum Einstrom von extrazellulärem Calcium und zur Aktivierung eines Natrium⁺ - H⁺ Kanals (Thibonnier M et al 2000 Am. J. Physiol. 279: H2529-2539).

Die Aktivierung der Kinasen führt zur Aktivierung von Transkriptionsfaktoren im Zellkern, was nach Induktion von "immediate early response"-Genen zur Steigerung des Proteingehalts und zur Zellproliferation führt (Geisterfer A A T et al 1989 Hypertension 14: 413-420). Diese mitogene Wirkung des AVP wurde neben glatten Gefäßmuskelzellen auch für diverse permanente Zellinien und für mesangiale Nierenzellen, Hepatozyten und Glomerulosazellen der Nebennierenrinde gezeigt.

Neben der Lokalisation in den Blutgefäßen findet sich der V₁-Rezeptor in der Leber, wo es nach AVP Aktivierung zur Glykogenolyse kommt, in der Nebennierenrinde, wo nach AVP Aktivierung Aldosteron ausgeschüttet wird, im Pankreas, wo es zur Insulinausschüttung kommt, auf Vorhofmyokardzellen im Herz, wo es zur Freisetzung des atrialen natriuretischen Faktors kommt, auf Thrombozyten, wo es zur Aggregation der Plättchen kommt, in der Milz, in der Niere, im Hirn, in der Gebärmutter, in Adipozyten und in verschiedenen Zellkulturlinien (Jard S 1998 In: Zingg H H et al eds 449: 1-13, Thibonnier M et al 2001 Annu. Rev. Pharmacol. Toxicol. 41: 175-202). Die Dissoziationskonstante für die Bindung von AVP an den vaskulären V₁-Rezeptor liegt bei 1,2 nM (Thibonnier M et al 1994 J. Biol. Chem. 269: 3304-3310). Dieser Rezeptor spricht erst bei AVP Konzentrationen an, bei denen der renale V₂-Rezeptor schon voll aktiviert ist.

Im Hypophysenvorderlappen ist ein dritter Vasopressinrezeptor V₃ lokalisiert. In corticotropen Zellen vermittelt er die Ausschüttung des adrenocorticotropen Hormons (ACTH) nach Aktivierung durch AVP. Der V₃-Rezeptor kann verschiedene Signaltransduktionswege aktivieren, sowohl den Weg über Adenylyl Cyklase mit Bildung von cAMP als "second messenger", als auch den Weg über Phospholipase C, der zur Mobilisierung von intrazellulärem Calcium führt (Thibonnier M 1997 Endocrinology 138: 4109-4122).

Das Oxytocin vermittelt seine Wirkung in der Peripherie durch Bindung an den Oxytocin Rezeptor, der in der Gebärmutter, in den Eierstöcken, in der Brustdrüse und in der Niere lokalisiert ist. Oxytocin aktiviert die Milchausschüttung in der Brust und ist bei der Geburt maßgeblich an der Kontraktion der Gebärmutter beteiligt. Der Oxytocin-Rezeptor induziert nach Bindung des Liganden ebenso wie der AVP V₁-Rezeptor die intrazelluläre Mobilisierung von Calcium.

Die kongestive Herzinsuffizienz (CHF; engl. "congestive heart failure") ist als Endstadium vieler verschiedenen Erkrankungen, haupsächlich verursacht durch Bluthochdruck und durch die koronare Herzkrankeit (Herzinfarkt, Angina pectoris), durch eine schwache Pumpleistung des Herzens gekennzeichnet, die mit zunehmender Schwere der Erkrankung einhergeht mit Kurzatmigkeit bei Anstrengungen, Wasseransammlung in Lunge und Geweben, Hyponatriämie und Atemschwierigkeiten in Ruhelage. Neben alten Menschen sind insbesonders ehemalige Herzinfarktpatienten betroffen. Unbehandelt führt die einmal initiierte Krankeit progressiv innerhalb kurzer Zeit zum Tod, da es durch hypertrophierende Herzmuskelzellen zu einem stetigen bindegewebigen Umbau des Herzens kommt, bei dem die Leistungsfähigkeit des Herzens zunehmend abnimmt. Die einmal initiierte Minderung der Herzfunktion führt zu einer neurohormonalen Gegenregulation, bei der das sympatische Nervensystem (SNS) und das Renin-Angiotensin-Aldosteron-System (RAAS) aktiviert werden, was unmittelbar zu einer Vasokonstriktion führt. Zunächst wird die Herzleistung dadurch erhöht, aber längerfristig überlastet die andauernde Vasokonstriktion den Herzmuskel und der Tod von Herzmuskelzellen und hypertrophierende Zellen führen zur oben bereits erwähnten zunehmenden bindegewebigen Umwandlung des Herzmuskels.

Bei der CHF wird auch ein deutlich gesteigerter AVP Plasmaspiegel festgestellt, wohl verursacht durch das aktivierte Renin-Angiotensin-Aldosteron-System (Angiotensin II stimuliert die AVP-Ausschüttung) und das aktivierte sympatische Nervensystem, das die Synthese von AVP im Hypothalamus stimuliert (Schrier R W et al 1998 Adv. Exp. Med. Biol. 449: 415-426, Schrier R W et al 1999 N. Engl. J. Med. 341: 577-585). Der erhöhte AVP Plasmaspiegel führt zur verstärkten Rückresorption des Wassers in der Niere mit den oben erwähnten Folgen für die Betroffenen.

Damit bietet sich das Peptidhormon als Ziel für neuartige Therapien dieser Krankheit an, da mit AVP-Rezeptorantagonisten diuretische Effekte erzielt werden können, die durch Reduzierung der Wasserlast auch den peripheren Gefäßwiderstand verringern und somit das Herz entlasten. Die direkt gefäßverengende Wirkung von AVP, die im Rahmen von CHF eher einen geringen Anteil ausmachen soll, könnte mit AVP-Antagonisten ebenfalls bekämpft werden. Darüberhinaus gibt es Evidenzen, daß AVP wegen seiner mitogenen Wirkung eine direkte Rolle bei der hypertrophen bindegewebigen Umwandlung des Herzmuskels spielt (Nakamura Y et al 2000 Eur. J. Pharmacol. 391: 39-48). Somit könnte AVP-Antagonismus nicht nur bei der kurzzeitigen Therapie akuter CHF eine Rolle spielen, sondern durch Hemmung AVP vermittelter Hypertrophierung den Status des Herzmuskels langfristig verbessern.

Für verbesserte Therapien besteht dringender Bedarf, da CHF die einzige weitverbreitete myokardiale Erkrankung ist, die an Häufigkeit in den letzten Jahren zunimmt und das Gesundheitssystem enorm belastet. Nachdem der Zusammenhang von CHF mit dem akivierten RAAS und SNS erkannt wurde, besteht die Standardtherapie zur Zeit in der Gabe von Angiotensin-Konverting-Enzym (ACE)-Hemmern, die das aktivierte RAAS herunterregulieren und in der Gabe von β-Blockern, die das aktivierte SNS dämpfen, sowie der Verabreichung von Diuretika, wobei Thiazide, Schleifendiuretika und Kalium-sparende Diuretika eingesetzt werden. Mit der Kombinationstherapie wird zwar die Überlebenswahrscheinlichkeit deutlich erhöht, aber es kommt immer wieder zu Rehospitalisierung, so daß der Bedarf nach verbesserter Behandlung groß ist. Die Diuretika können bei Langzeitgabe die Niere schädigen und zu Störungen im Elektrolythaushalt führen.

Neuartige Therapie-Ansätze zur Behandlung der CHF, die direkt auf die Wirkung des AVP abzielen, beruhen darauf, Rezeptorantagonisten gegen die oben erwähnten V₁- und V₂-Rezeptoren einzusetzen (Lee C R 2003 Am. Heart J. 146: 9-18). Es existiert eine Reihe von nichtpeptidischen Rezeptorantagonisten aus der Gruppe der Benzazepine, die oral verabreicht werden können und mit hoher Affinität (Inhibitionskonstanten Kᵢ von 0,5 nM - 3 nM, Thibonnier M et al 2001 Annu. Rev. Pharmacol. Toxicol. 41: 175-202) entweder spezifisch an einen oder an beide (V₁- und V₂-) Rezeptoren binden. Einige der Rezeptorantagonisten sind nach erfolgreichen Tierstudien und kleineren klinischen Studien in weiterer Entwicklung.

Der V₂-Rezeptorantagonist Tolvaptan zeigte in einer placebokontrollierten Doppelblindstudie an CHF Patienten, die gleichzeitig die Standardtherapie erhielten, eine deutliche Reduktion von Ödemen und des Gewichts bei Patienten mit erhöhter Wasserlast (Gheorghiade M et al 2000 Circulation 102: 592). In Patienten mit Hyponatriämie kam es zur Normalisierung der Natriumkonzentration im Plasma (Gheorghiade M et al 2002 *J. Am. Coll. Cardiol.* 39: 171).

Der am weitesten enwickelte V₁/V₂-Rezeptorantagonist Conivaptan stellte seine Wirksamkeit in CHF Patienten ebenfalls unter Beweis. Die Abnahme des sogenannten "pulmonary capillary wedge pressure" nach einmaliger intravenöser Gabe zeigt eine Entlastung des Herzens. Darüber hinaus wurde ein Anstieg der Urinmenge bei Abnahme der Urin-Osmolarität nach Behandlung mit Conivaptan registriert (Udelson J E et al 2001 Circulation 104: 2417-2423).

Ein möglicherweise positiver Effekt von Conivaptan auf die bei CHF-Patienten auftretende Hypertrophierung des Herzens konnte in Zellkulturstudien bei primären Herzmuskelzellen gezeigt werden, wo die durch AVP per V₁-Rezeptor induzierte Proteinsynthese dosisabhängig inhibiert werden konnte (Tahara A et al 1998 Cardiovasc. Res. 38: 198-205).

Daten und Studien zur Sicherheit, zur Morbidität und Mortalität nach Langzeitbehandlung von CHF Patienten mit den AVP Rezeptorantagonisten stehen noch aus, um den wahren Wert der in den bisherigen Studien positiven Effekte für die Behandlung der CHF beurteilen zu können (Russel S D 2003 Am. J Cardiovasc. Drugs 3:13-20).

Die internationale Patentanmeldung WO 95/07364 offenbart RNA-Aptamere, d.h. D-Nukleinsäuren, die gegen diverse, von Vasopressin verschiedene Zielmoleküle gerichtet sind.

Der vorliegenden Erfindung liegt somit die Aufgabe zugrunde, ein Mittel bereitzustellen zur Behandlung von Erkrankungen, die auf einen erhöhten AVP-Plasmaspiegel zurückgehen oder auf diesem beruhen. Eine weitere Aufgabe der vorliegenden Erfindung besteht darin, als Alternative zu AVP-Rezeptorantagonisten neue AVP-Antagonisten bereitzustellen, die insbesondere als Alternative zu AVP-Rezeptorantagonisten verwendet werden können. Eine noch weitere Aufgabe der vorliegenden Erfindung ist es, Antagonisten mit einer hohen Spezifität für AVP bereitzustellen.

Die Aufgabe wird erfingdungsgemäß gelöst durch den Gegenstand der beigefügten unabhängigen Ansprüche. Bevorzugte Ausführungsformen ergeben sich aus den Unteransprüchen.

In einem ersten Aspekt wird die Aufgabe auch insbesondere gelöst durch eine Vasopressin bindende Nukleinsäure, wobei die Nukleinsäure einen Abschnitt Box 1, einen Abschnitt Box2, einen Abschnitt Helix1 und einen Abschnitt Helix2 umfasst,
wobei Box1 die Sequenz GUGGW aufweist und W = A oder U ist, bevorzugterweise W = U ist, und
wobei Box2 die Sequenz GGGGUAGGGMUUGGAHGGGHA aufweist,
wobei
M jeweils und unabhängig A oder C ist
H jeweils und unabhängig A, C oder U ist, und der Abschnitt Helix1 und der Abschnitt Helix2 zueinander komplementär sind und der Abschnitt Helix1 und der Abschnitt Helix2 jeweils 5 bis 9 Nukleotide umfasst.

In einer bevorzugten Ausführungsform des ersten Aspektes ist vorgesehen, dass U oder C, bevorzugterweise U an den Positionen von H vorhanden ist.

In einer Ausführungsform des ersten Aspektes ist vorgesehen, dass der Abschnitt Helix1 und der Abschnitt Helix2 jeweils 6 bis 7 Nukleotide und bevorzugtererweise 7 Nukleotide umfasst und die Abschnitte Helix1 und Helix2 eine doppelsträngige Helix ausbilden.

In einer bevorzugten Ausführungsform des ersten Aspektes ist vorgesehen, dass die doppelsträngige Helix terminal ausgebildet ist.

In einer Ausführungsform des ersten Aspektes ist vorgesehen, dass die Nukleinsäuren einen W-Abschnitt umfasst, wobei der W-Abschnitt 0 bis 10, bevorzugterweise 6 bis 9 Nukleotide, oder alternativ 0 bis 7 Nukleotide umfasst.

In einer bevorzugten Ausführungsform des ersten Aspektes ist vorgesehen, dass der W-Abschnitt entweder (a) nur aus einem oder mehreren von A und/oder U besteht, oder (b) aus einem oder mehreren von A und/oder U und einem G besteht.

In einer Ausführungsform des ersten Aspektes ist vorgesehen, dass der W-Abschnitt eine PEG-Gruppe umfasst.

In einer bevorzugten Ausführungsform des ersten Aspektes ist vorgesehen, dass die PEG-Gruppe am 5'-Ende des W-Abschnittes oder am 3'-Ende des W-Abschnittes oder zwischen zwei Nukleotiden des W-Abschnittes angeordnet ist.

In einer Ausführungsform des ersten Aspektes ist vorgesehen, dass die Nukleinsäure eine PEG-Gruppe enthält.

In einer Ausführungsform des ersten Aspektes ist die Vasopressin bindende Nukleinsäure eine Nukleinsäure gemäß Formel (I) 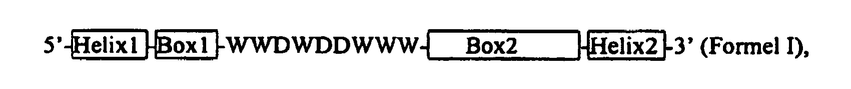 wobei
Helix1 7 Nukleotide umfasst,
Helix2 7 Nukleotide umfasst,
Helix1 und Helix2 zueinander komplementär sind und eine terminale doppelsträngige Helix bilden,
Box1 GUGGW ist, wobei W = A oder U ist, bevorzugterweise U ist,
der W-Abschnitt WWDWDDWWW 6 bis 9 Nukleotide umfasst, wobei
D einzeln und unabhängig A, G oder U sein kann, und wobei bevorzugterweise der W-Abschnitt entweder (a) nur aus einem oder mehrere von A und/oder U besteht, oder (b) aus einem oder mehreren von A und/oder U und einem G besteht.

Wie aus der Formel (I) ersichtlich, weisen die erfindungsgemäßen Nukleinsäuren in einer Ausführungsform eine Konsensus-Struktur auf. Die Konsensus-Struktur umfasst dabei zwei komplementäre Bereiche, genannt Helix1 am 5'-Ende und Helix2 am 3'-Ende, die infolge der Primärstruktur der Nukleinsäure miteinander paaren und eine terminale, doppelsträngige Helix innerhalb der Konsensus-Struktur bilden. Bevorzugterweise weist die Helix eine Länge von sieben gepaarten Nukleotiden auf. In 5'- 3'-Richtung schließt sich dem ersten komplementären Bereich eine Box1 an, die bevorzugterweise aus fünf Nukleotiden der Sequenz GUGGW besteht, wobei W für A oder U steht, wobei bevorzugterweise an dieser Position ein U steht. Dieser Sequenz schließt sich ein sechs bis neun Nukleotide langer A- und U-reicher Bereich an, genannt W-Abschnitt, in dem vorwiegend W, selten G und niemals C vorkommt. Als nächstes folgt eine einundzwanzig Nukleotide lange Box2, die bevorzugterweise die folgende Sequenz aufweist, nämlich GGGGUAGGGMUUGGAHGGGHA, wobei M für A oder C, und H für A oder C oder U steht, wobei an den Positionen, wo H steht, bevorzugterweise U oder C steht, und bevorzugtererweise U steht. Kennzeichnend für Box2 sind vier doppelt bis vierfach vorkommende, strikt konservierte Gs. In 5'-3'-Richtung schließt sich der zweite komplementäre Bereich, Helix2 genannt, an, der mit dem komplementären Bereich am 5'-Ende die terminale, doppelsträngige Helix ausbildet.

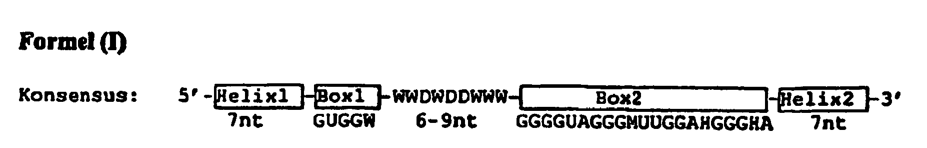

In einer Ausführungsform des ersten Aspektes ist die Vasopressin bindende Nukleinsäure eine Nukleinsäure gemäß Formel (II) 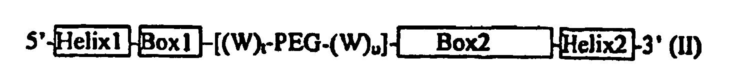 wobei -PEG- für eine PEG-Gruppe steht,
t und u einzeln und unabhängig voneinander 0, 1, 2, 3, 4 oder 5 ist, wobei t + u 0, 1, 2, 3, 4 oder 5 ist; und
wobei
Helix1, Helix2, Box1 und Box2 wie im Zusammenhang mit der im Zusammenhang mit Formel (I) beschriebenen Ausführungsform definiert sind; und
W = A oder U ist.

Wie aus der Formel (II) ersichtlich, weisen die erfindungsgemäßen Nukleinsäuren in einer Ausführungsform eine Konsensus-Struktur wie in Formel (I) auf mit dem Unterschied, daß der A-und U-reiche Abschnitt zwischen Box 1 und Box 2 aus einem PEG-( Hexaethylenglykol)-Spacer in Kombination mit null bis fünf Ws besteht, und die Lage des PEG-Spacers in dem A- und U-reichen Abschnitt frei variabel ist. Dieser Abschnitt wird in den erfindungsgemäßen Nukleinsäuren nach Formel (II) PEG+W genannt.

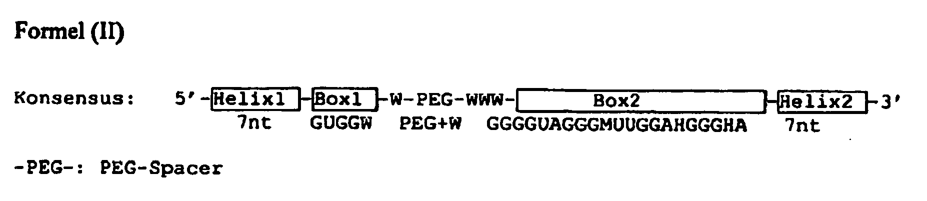

In einer Ausführungsform des ersten Aspektes ist die Vasopressin bindende Nukleinsäure eine Nukleinsäure gemäß Formel (III) 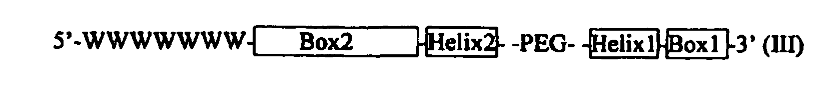 wobei -PEG- für eine PEG-Gruppe steht, und
wobei
Helix1 und Helix2 jeweils 6 oder 7 Nukleotide, bevorzugterweise 6 Nukleotide, umfasst,
Helix1 und Helix2 zueinander komplementär sind und eine doppelsträngige Helix bilden,
Box1 und Box2 wie im Zusammenhang mit der im Zusammenhang mit Formel (I) beschriebenen Ausführungsform definiert sind, wobei der W-Abschnitt WWWWWWW aus 0 bis 7 Ws besteht, wobei W = A oder U ist, bevorzugterweise kein W in Formel (III) vorhanden ist und
die PEG-Gruppe in 5'-3'-Richtung zwischen Helix2 und Helix1 angeordnet ist

Wie aus der Formel (III) ersichtlich, weisen erfindungsgemäße Nukleinsäuren in einer Ausführungsform eine Konsensus-Struktur wie in Formel (II) auf mit den folgenden Unterschieden. Die Lage des 5'- und 3'-Endes ist insoweit verändert, als dass das 5'-Ende der erfindungsgemäßen Nukleinsäuren in dem A- und U-reichen Abschnitt liegt, und das 3'-Ende am Ende von Box 1 lokalisiert ist. Der W-Abschnitt besteht aus null bis sieben Ws, bevorzugterweise aus null W. Box1 und Box2 weisen die in den Formeln (I) und (II) dargestellten Sequenzen auf, bestehend aus fünf und einundzwanzig Nukleotiden. Die komplementären Bereiche Helix1 und Helix2 bestehen aus jeweils sechs bis sieben Nukleotiden, bevorzugterweise aus sechs Nukleotiden, die eine doppelsträngige Helix ausbilden. Der PEG-Spacer liegt nicht mehr in dem A- und U-reichen Abschnitt, sondern er befindet sich in 5'-3'-Richtung gesehen zwischen dem Ende von Helix2 und dem Anfang von Helix1. An Helix1 schließt sich Box1 an, deren 3'-Ende, wie bereits erwähnt, den 3'-Terminus der erfindungsgemäßen Nukleinsäuren bildet.

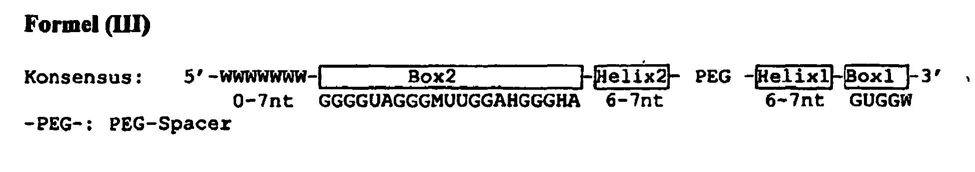

Zu den erfindungsgemäßen Nukleinsäuren gehören auch die in der nachfolgenden Tabelle angeführten Nukleinsäuren, die hierin auch als erfindungsgemäße Nukleinsäuren bezeichnet werden. Die dabei verwendeten Abkürzungen und Symbole entsprechen den hierin definierten.

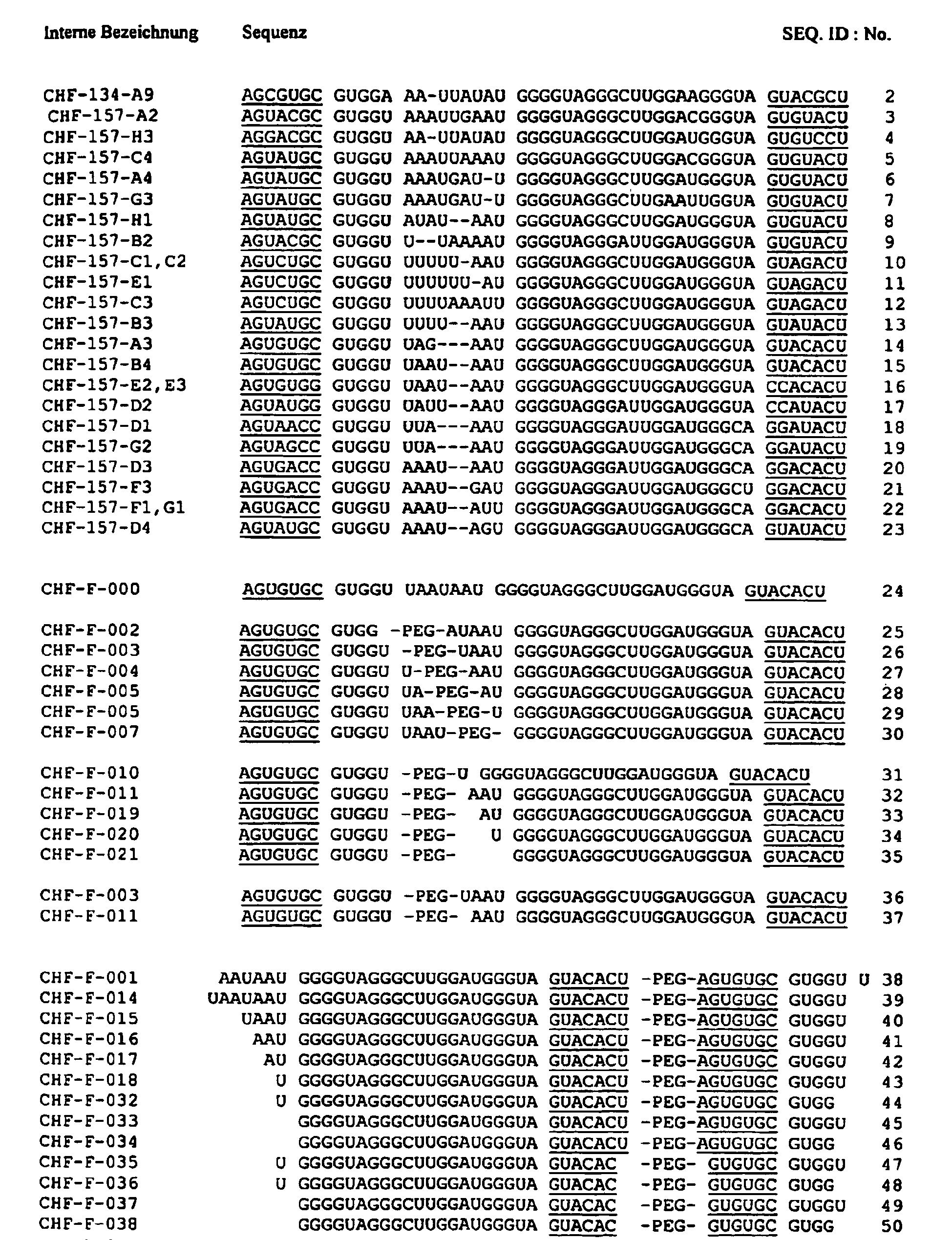

, wobei -PEG- für eine PEG-Gruppe, wie hierin definiert, steht.

In einer Ausführungsform der Nukleinsäure, wie sie in Formel (III) dargestellt ist, ist vorgesehen, dass bei der in 5'-3'-Richtung ersten Gruppe (G)ₙ n = 4 ist, bei der in 5'-3'-Richtung zweiten Gruppe (G)ₙ n = 3 ist, bei der in 5'-3'-Richtung dritten Gruppe (G)ₙ n = 2 ist, und bei der in 5'-3'-Richtung vierten Gruppe (G)ₙ n = 3 ist.

In einer Ausführungsform der erfindungsgemäßen Nukleinsäure insbesondere den in den Formeln (I)-(III) dargestellten Nukleinsäuren ist vorgesehen, dass die PEG-Gruppe ein Molekulargewicht von etwa 172 - 688 Da, bevorzugterweise etwa 344 aufweist.

In einer Ausführungsform der erfindungsgemäßen Nukleinsäure ist vorgesehen, dass die Sequenz ausgewählt ist aus der Gruppe von Sequenzen, die SEQ. ID. NO. 2 bis SEQ. ID. NO. 50 umfasst.

In einer Ausführungsform der in den Formeln (I)-(III) dargestellten Nukleinsäuren ist vorgesehen, dass die Nukleinsäure an Vasopressin bindet.

In einer bevorzugten Ausführungsform ist dabei vorgesehen, dass das Vasopressin humanes Vasopressin ist.

In einer Ausführungsform der erfindungsgemäßen Nukleinsäure ist vorgesehen, dass das Vasopressin eine Aminosäuresequenz gemäß SEQ. ID. NO. 1 aufweist.

In einer Ausführungsform der erfindungsgemäßen Nukleinsäure ist vorgesehen, dass die Nukleinsäure eine Modifikation aufweist.

In einer bevorzugten Ausführungsform ist dabei vorgesehen, dass die Modifikation ausgewählt ist aus der Gruppe, die HESylierung und PEGylierung umfasst.

In einer bevorzugten Ausführungsform ist dabei vorgesehen, dass die PEGylierung durch ein geradkettiges oder verzweigtkettiges PEG erfolgt ist, wobei das Molekulargewicht des PEG etwa 20 bis 120 kDa, bevorzugterweise etwa 30 bis 80 kDa und bevorzugtererweise etwa 40 kDa ist.

In einer bevorzugten Ausführungsform ist dabei vorgesehen, dass die HESylierung durch ein HES erfolgt ist, wobei das Molekulargewicht der HES etwa 10 bis 130 kDa, bevorzugterweise etwa 30 bis 80 kDa und bevorzugtererweise etwa 50 kDa ist.

In einer Ausführungsform der erfindungsgemäßen Nukleinsäure ist vorgesehen, dass die Nukleinsäure vollständig aus L-Nukleotiden besteht.

In einem zweiten Aspekt wird die Aufgabe gelöst durch eine pharmazeutische Zusammensetzung umfassend eine Nukleinsäure nach dem ersten Aspekt und optional einem weiteren Bestandteil, wobei der weitere Bestandteil aus der Gruppe ausgewählt ist, die pharmazeutisch akzeptable Trägermittel umfasst.

In einem dritten Aspekt wird die Aufgabe gelöst durch die Verwendung einer Nukleinsäure nach dem ersten Aspekt zur Herstellung eines Medikamentes.

In einem vierten Aspekt wird die Aufgabe gelöst durch die Verwendung einer Nukleinsäure nach dem ersten Aspekt zur Herstellung eines diagnostischen Mittels.

In einer Ausführungsform des dritten Aspektes ist vorgesehen, dass das Medikament für die Behandlung und/oder Prävention von kongestiver Herzinsuffizienz ist, bevorzugterweise für die kurzzeitige Therapie, bevorzugtererweise zur Behandlung und/oder Prävention akuter dekompensierter kongestiver Herzinsuffizienz.

In einer alternativen Ausführungsform des dritten Aspektes ist vorgesehen, dass das Medikament für die Behandlung und/oder Prävention einer Erkrankung ist, wobei die Erkrankung eine Folgeerkrankung einer Krankheit ist, die ausgewählt ist aus der Gruppe, die Bluthochdruck, koronare Herzerkrankung, Herzinfarkt und Angina pectoris umfasst.

In einer Ausführungsform des dritten Aspektes ist vorgesehen, dass die Patienten ältere Menschen oder Patienten mit einem vorhergegangenen Herzinfarkt sind.

In einer alternativen Ausführungsform des dritten Aspektes ist vorgesehen, dass das Medikament ein Medikament zur Prävention und/oder Behandlung von Bluthochdruck ist.

In einer weiteren alternativen Ausführungsform des dritten Aspektes ist vorgesehen, dass das Medikament ein Medikament zur Behandlung und/oder Prävention von Hypertrophie des Herzmuskels ist, bevorzugterweise durch (Arginin⁸)-Vasopressin Hyptertrophierung des Herzmuskels vermittelte.

In einer Ausführungsform des dritten Aspektes ist vorgesehen, dass das Medikament ein Medikament zur Behandlung und/oder Prävention von Ödemen ist.

In einer Ausführungsform des dritten Aspektes ist vorgesehen, dass das Medikament ein Medikament zur Verringerung des Gewichts bei Patienten mit erhöhter Wasserlast ist.

In einer Ausführungsform des dritten Aspektes ist vorgesehen, dass das Medikament ein Medikament zur Behandlung von Personen mit Hyponatriämie oder zur Prävention von Hyponatriämie ist.

In einer Ausführungsform des dritten Aspektes ist vorgesehen, dass das Medikament ein Medikament zur Behandlung und/oder Prävention des Syndroms der inadäquaten ADH-Sekretion ist.

In einer bevorzugten Ausführungsform des dritten Aspektes ist vorgesehen, dass das Syndroms der inadäquaten ADH-Sekretion einhergeht mit mindestens einem weiteren Symptom, wobei das weitere Symptom ausgewählt ist aus der Gruppe, die Antidiurese, Hyponatriämie und Hypoosmolarität umfasst.

In einer Ausführungsform des dritten Aspektes ist vorgesehen, dass das Syndrom der inadäquaten ADH-Sekretion auf mindestens einer Ursache beruht, wobei die Ursache ausgewählt ist aus der Gruppe, die ektope ADH-Sekretion durch Tumore, medikamentöse Induktion von ADH-Sekretion, Erkrankungen der Lunge und Veränderung der zentralen Osmorezeptoren nach Schädel-Hirn-Trauma oder nach Meningitis umfasst.

In einer alternativen Ausführungsform des dritten Aspektes ist vorgesehen, dass das Medikament ein Medikament für die Prävention und/oder Behandlung der mit Leberzirrhose einhergehenden Hyponatriämie ist.

In einer alternativen Ausführungsform des dritten Aspektes ist vorgesehen, dass das Medikament ein Medikament zur Behandlung und/oder Prävention von Hirnödem, insbesondere nach Schädel-Hirn-Trauma oder nach Schlaganfall ist.

In einer alternativen Ausführungsform des dritten Aspektes ist vorgesehen, dass das Medikament für die Behandlung und/oder Prävention von Schädel-Hirn-Trauma und/oder Schlaganfall ist.

In einer alternativen Ausführungsform des dritten Aspektes ist vorgesehen, dass das Medikament ein Medikament zur Behandlung und/oder Prävention eines Tumors ist.

In einer bevorzugten Ausführungsform des dritten Aspektes ist vorgesehen, dass zumindest einige der den Tumor ausbildenden Zellen mindestens einen Rezeptor exprimieren, wobei der Rezeptor ausgewählt ist aus der Gruppe, die V₁-Rezeptoren, V₂-Rezeptoren und V₃-Rezeptoren umfasst.

In einer weiteren bevorzugten Ausführungsform des dritten Aspektes ist vorgesehen, dass der Tumor ausgewählt ist aus der Gruppe, die ACTH sekretierende Tumoren, kleinzelliges Bronchialkarzinom und Mammakarzinom umfasst.

In einer alternativen Ausführungsform des dritten Aspektes ist vorgesehen, dass das Medikament ein Medikament zur Verhinderung von Frühgeburten ist.

In einer weiteren alternativen Ausführungsform des dritten Aspektes ist vorgesehen, dass das Medikament zur Prävention und/oder Behandlung von primärer Dysmenorrhö ist.

In einem fünften Aspekt wird die Aufgabe gelöst durch einen Komplex umfassend Vasopressin und eine Nukleinsäure nach dem ersten Aspekt.

In einem sechsten Aspekt wird die Aufgabe gelöst durch Verfahren zum Screenen von Vasopressin-Antagonisten oder von Vasopressin-Agonisten umfassend die folgenden Schritte:
- Bereitstellen eines Kandidaten-Vasopressin-Antagonisten und/oder eines Kandidaten-Vasopressin-Agonisten,
- Bereitstellen einer Nukleinsäuren nach dem ersten Aspekt,
- Bereitstellen eines Testsystems, welches eine Signaländerung in Gegenwart eines Vasopressin-Antagonisten und/oder eines Vasopressin-Agonisten ergibt, und
- Bestimmen, ob der Kandidaten-Vasopressin-Antagonist ein Vasopressin-Antagonist ist, und/oder ob der Kandidaten-Vasopressein-Agonist ein Vasopressin-Agonist ist.

In einer Ausführungsform ist vorgesehen, dass das Vasopressin (Arginin⁸)-Vasopressin ist.

In einem siebten Aspekt wird die Aufgabe gelöst durch Verfahren zum Screenen von Vasopressin-Agonisten und/oder Vasopressin-Antagonisten umfassend die folgenden Schritte:
- Bereitstellen von Vasopressin an Phase, bevorzugterweise einer festen Phase
- Bereitstellen einer Nukleinsäure nach dem ersten Aspekt bevorzugterweise einer markierten Nukleinsäure nach dem ersten Aspekt,
- Zugabe eines Kandidaten-Vasopressin-Agonisten und/oder eines Kandidaten-Vasopressin-Antagonisten, und
- Bestimmen, ob der Kandidaten-Vasopressin-Agonist ein Vasopressin-Agonist ist und/oder ob der Kandidaten-Vasopressin-Antagonist ein Vasopressin-Antagonist ist.

In einer Ausführungsform ist vorgehen, dass das Bestimmen dadurch erfolgt, dass festgestellt wird, ob die Nukleinsäure durch den Kandidaten-Vasopressin-Agonisten verdrängt wird.

In einem achten Aspekt wird die Aufgabe gelöst durch Kit für den Nachweis von Vasopressin, bevorzugterweise Arginin-Vasopressin, umfassend eine Nukleinsäure nach dem ersten Aspekt.

Die vorliegenden Erfinder haben überraschend festgestellt, dass es möglich ist, spezifisch an (Arg⁸)-Vasopressin, bindende Nukleinsäuren und insbesondere L-Nukleinsäuren zu screenen. Bei den erfindungsgemäßen Nukleinsäuren handelt es sich bevorzugterweise um Ribonükleinsäuren und bevorzugtererweise um L-Ribonukleinsäuren. Die erfindungsgemäßen (Arg⁸)-Vasopressin bindenden Nukleinsäuremoleküle stellen gegenüber dem im Stand der Technik beschriebenen Vasopressin bindenden DNA-Molekül (Williams K. P. et al. 1997 Proc. Natl. Acad. Sci USA 94: 11285-11290) insoweit eine überraschende Verbesserung dar, als dass das im Stand der Technik beschriebene DNA-Molekül nur eine Dissoziationskonstante von 0,9 µM *in vitro* aufwies. In Zellkulturversuchen zeigte dieses DNA-Molekül des Standes der Technik einen IC50 von ca. 3 µM und wurde insoweit für einen erfolgversprechenden Einsatz als Therapeutikum wegen der zu schwachen Bindung an Vasopressin verworfen. Schließlich war das (Arg⁸)-Vasopressin bindende DNA-Molekül nach dem Stand der Technik verglichen mit den erfindungsgemäßen Nukleinsäuren auch insoweit nicht vorteilhaft, als dass es eine Länge von 55 Nukleotiden aufweist, was einen erheblichen Herstellungsaufwand mit sich gebracht hätte. Demgegenüber sind die erfindungsgemäßen Nukleinsäuren um mehrere Größenordnungen affiner (Dissoziationskonstante: 1,5 nM, gemessen durch isothermale Kalorimetrie; IC50: 1 nM im cAMP-Zellkulturtest), um bis zu 17 Nukleotide kürzer und, wie in dem Beispiel näher dargelegt, auch *in vivo* und bei 37 °C aktiv. Insoweit haben sich die vorliegenden Erfinder von der im Stand der Technik begründeten Auffassung der Fachwelt gelöst, dass (Arg⁸)-Vasopressin bindende Nukleinsäuren für therapeutische Zwecke nicht geeignet seien.

Weiterhin haben die vorliegenden Erfinder überraschenderweise festgestellt, dass alle positiven Effekte, die bisher mit AVP-Rezeptorantagonisten gezeigt werden konnten, wie beispielsweise gesteigerte Diurese ohne Störung im Elektrolythaushalt zu erzeugen, Entlastung des Herzens, oder möglicherweise Inhibition der Hypertrophie des Herzmuskels, auch mit den erfindungsgemäßen Molekülen erreicht werden können.

Ein weiterer Vorteil der erfindungsgemäßen Nukleinsäuren besteht darin, dass sie gegenüber AVP-Rezeptorantagonisten nach dem Stand der Technik für eine Dauermedikation vorteilhaft sind. Bei der Medikation mit AVP-Rezeptorantagonisten nach dem Stand der Technik kann es zu einer Gegenregulation mit einem einhergehenden Anstieg der AVP-Konzentration im Plasma kommen, was bei Dauermedikation letztlich die Effekte der Antagonisten erniedrigen kann. Selbst wenn diese Reaktion auch bei Verwendung der erfindungsgemäßen Nukleinsäuren auftreten sollte, so kann eine entsprechend hohe Dosierung selbst unphysiologisch hohe AVP-Plasmaspiegel neutralisieren.

Die erfindungsgemäßen Nukleinsäuren umfassen in einer bevorzugten Ausführungsform auch solche Nukleinsäuren, die im Wesentlichen homolog zu den spezifisch hierin offenbarten Sequenzen sind. Der Begriff "im Wesentlichen homolog" soll hierin bevorzugterweise so verstanden werden, dass die Homologie wenigstens 75 %, bevorzugterweise 85 %, bevorzugtererweise 90 % und am bevorzugtesten mehr als 95, 96, 97, 98 oder 99 % beträgt.

Bei den erfindungsgemäßen Nukleinsäuren handelt es sich bevorzugterweise um solche, die aus Ribonukleotiden bestehen. Es ist jedoch auch im Rahmen der vorliegenden Erfindung, dass einzelne Nukleotide als 2'-Desoxy-ribonukleotide oder andere Varianten wie z.B. 2'-O-Methylribonukleotide, oder LNA-Nukleotide im Molekül vorhanden sind. Am bevorzugtesten ist jedoch, wenn die Nukleinsäure vollständig aus Ribonukleotiden besteht.

Die erfindungsgemäßen Nukleinsäuren binden bevorzugterweise an Vasopressin bei 37° C in Lösung mit einer Dissoziationskonstante Kd <10 nM, gemessen mit der isothermalen Kalorimetrie.

Es wird offenbart, dass die erfindungsgemäßen Nukleinsäuren in einer bevorzugten Ausführungsform auch an Oxytocin binden können.

Im Rahmen der vorliegenden Erfindung umfassen in einer Ausführungsform die erfindungsgemäßen Nukleinsäuren auch solche, die Teil einer längeren Nukleinsäure sind, wobei diese längeren Nukleinsäuren mehrere Teile umfassen können, wobei wenigstens ein Teil eine Nukleinsäure gemäß der vorliegenden Erfindung oder ein Teil davon ist. Der andere Teil oder die anderen Teile dieser längeren Nukleinsäuren können entweder eine D-Nukleinsäure oder eine L-Nukleinsäure sein. Eine jegliche Kombination kann in Verbindung mit der vorliegenden Erfindung und zu den Zwecken bzw. Verwendungen verwendet werden, wie sie für die erfindungsgemäßen Nukleinsäuren hierin offenbart sind. Dieser andere Teil bzw. diese anderen Teile der längeren Nukleinsäure können eine Funktion aufweisen, die vom Binden und insbesondere vom Binden an Vasopressin verschieden ist. Eine mögliche Funktion besteht darin, eine Wechselwirkung mit anderen Molekülen, z. B. zu Zwecken der Immobilisierung, Kreuzvernetzung, des Nachweises, der Amplifikation oder Modifikation oder Molekulargewichtserhöhung zu erlauben.

In einem weiteren Aspekt betrifft die vorliegende Erfindung eine pharmazeutische Zusammensetzung, die aus mindestens einer der erfindungsgemäßen Nukleinsäuren in Kombination mit einer oder mehreren anderen Nukleinsäuren besteht, wobei die andere(n) Nukleinsäuren bevorzugterweise an von (Arg⁸)-Vasopressin verschiedene Zielmoleküle bindet/binden oder eine von den erfindungsgemäßen Nukleinsäuren verschiedene Funktion ausübt/ausüben.

In einer Ausführungsform können die erfindungsgemäßen Nukleinsäuren modifiziert vorliegen. Besonders bevorzugte Formen der Modifizierung sind die PEGylierung oder HESylierung. Dabei handelt es sich um die Modifizierung der erfindungsgemäßen Nukleinsäuren durch Bindung von Polyethylenglycol (PEG), Hydroxyethylstärke (HES), oder anderer Gruppen, die in der europäischen Patentanmeldung EP 1 306 382 beschrieben wurden und deren Offenbarung hierin durch Bezugnahme aufgenommen wird.

Bevorzugterweise beträgt das Molekulargewicht der solchermaßen modifizierten, d. h. PEGylierten erfindungsgemäßen Nukleinsäure etwa 2.000 bis 200.000 Da, bevorzugterweise 40.000 bis 120.000 Da.

Die HESylierung von Nukleinsäuren ist beispielsweise in der deutschen Patentanmeldung DE 1 2004 006 249.8 beschrieben. Die dabei verwendete Hydroxyethylstärke (HES) weist bevorzugterweise ein Zahlenmittel des mittleren Molekulargewichtes von 3 bis 100.000 Da und bevorzugterweise von 5.000 bis 60.000 Da auf.

Der Vorteil der Modifikation der erfindungsgemäßen Nukleinsäure unter Verwendung von hochmolekularen Polymeren, die physiologisch akzeptabel sind, wie beispielsweise Hydroxyethylstärke (HES) oder Polyethylenglycol (PEG), besteht darin, dass die Ausscheidungskinetik der erfindungsgemäßen Nukleinsäuren verändert wird. Ohne darauf festgelegt sein zu wollen, scheint dieses Verhalten der solchermaßen modifizierten erfindungsgemäßen Nukleinsäuren darin begründet zu liegen, dass infolge des erhöhten Molekulargewichtes der solchermaßen modifizierten erfindungsgemäßen Nukleinsäuren und der fehlenden Metabolisierbarkeit derselben, insbesondere wenn diese als L-Nukleinsäuren vorliegen, deren Ausscheidung aus einem Organismus, insbesondere einem Säugetierorganismus, verlangsamt ist. Da die Ausscheidung typischerweise über die Nieren erfolgt, wird derzeit davon ausgegangen, dass die glomeruläre Filtrationsrate der Nieren hinsichtlich der solchermaßen modifizierten erfindungsgemäßen Nukleinsäuren gegenüber derjenigen der nicht-modifizierten erfindungsgemäßen Nukleinsäuren signifikant verringert ist, was zu einer erhöhten Verweildauer, d. h. biologischen Halbwertszeit der modifizierten erfindungsgemäßen Nukleinsäuren, insbesondere der modifizierten erfindungsgemäßen L-Nukleinsäure, verglichen mit der Verweildauer der korrespondierenden, aber nicht modifizierten erfindungsgemäßen Nukleinsäuren, insbesondere der nicht modifizierten L-Nukleinsäuren, führt. Besonders beachtlich ist in diesem Zusammenhang die Tatsache, dass trotz der in einer bevorzugten Ausführungsform vorliegenden Modifizierung die solchermaßen modifizierten erfindungsgemäßen Nukleinsäuren an ihrer Spezifität offensichtlich nichts einbüßen. Damit weisen die erfindungsgemäßen Nukleinsäuren insbesondere in ihrer modifizierten Form vollkommen überraschend eine Eigenschaft auf, wie man sie ansonsten bei anderen pharmazeutisch aktiven Verbindungen normalerweise nicht realisieren kann, dass nämlich auf umfangreiche galenische Formulierungen, beispielsweise in Form von Depotpräparaten, die sukzessive den Wirkstoff abgeben, verzichtet und vielmehr eine direkte Modifizierung des infragestehenden Wirkstoffes herbeigeführt werden kann, ohne dass dabei dessen biologische Aktivität, insbesondere ausgedrückt als Spezifität der Reaktion oder Komplexbildung mit dem jeweiligen Zielmolekül, nachteilig beeinflusst wird. Es ist im Rahmen der vorliegenden Erfindung, die modifizierten erfindungsgemäßen Nukleinsäuren in Form von Depotpräparaten anzuwenden.

Es ist auch im Rahmen der vorliegenden Erfindung, dass die erfindungsgemäßen Nukleinsäuren in nicht-modifizierter Form vorliegen, d. h. keine Modifikation aufweisen, was den mit ihrer Herstellung verbunden Aufwand verringert. Insbesondere bei der Behandlung von akuter dekompensierter kongestiver Herzinsuffizienz in der Intensivmedizin mit intravenöser Infusion ist diese Ausführungsform der erfindungsgemäßen Nukleinsäuren insoweit besonders vorteilhaft, als dass sie besser dosierbar ist bzw. dort Anwendung findet, wo eine schnelle Ausscheidung durch Diurese bzw. eine kurze Halbwertszeit im Plasma erwünscht ist.

Infolge der hohen Stabilität der erfindungsgemäßen Nukleinsäuren, insbesondere in der Ausführungsform, dass diese als L-Nukleinsäuren vorliegen, ist eine direkte Gabe der erfindungsgemäßen Nukleinsäuren zur Behandlung eines Patienten, der einer solchen Behandlung bedarf, möglich. Bevorzugterweise werden die erfindungsgemäßen Nukleinsäuren als physiologische Lösung zur lokalen oder systemischen Verabreichung bereitgestellt. Bevorzugterweise ist die pharmazeutische Zusammensetzung für die intravenöse Verabreichung. Es ist jedoch auch im Rahmen der vorliegenden Erfindung, dass derartige pharmazeutische Zusammensetzungen intramuskulär, intraperitoneal, oder subkutan verabreicht werden können. Die erfindungsgemäßen Nukleinsäuren sind bevorzugterweise in einem pharmazeutisch akzeptablen Lösungsmittel enthalten oder gelöst. Derartige Lösungsmittel sind insbesondere jene, die aus der Gruppe ausgewählt sind, die physiologische Kochsalzlösung, PBS, oder eine Glukoselösung, insbesondere eine 5% GlukoseLösung umfasst.

Die erfindungsgemäßen Nukleinsäuren können zur Herstellung eines Medikamentes ebenso wie zur Herstellung eines diagnostischen Mittels verwendet werden. Die diagnostische Anwendung oder Verwendung beruht dabei auf der spezifischen Wechselwirkung zwischen den erfindungsgemäßen Nukleinsäuren und Vasopressin, insbesondere (Arg⁸)-Vasopressin die hierin auch in ihrer Gesamtheit oder einzeln als Zielmolekül bezeichnet werden. Infolge der Beteiligung von Vasopressin an den verschiedenen, hierin im Zusammenhang mit dem Aspekt der Verwendung der erfindungsgemäßen Nukleinsäuren zur Herstellung eines Medikamentes beschriebenen Erkrankungen, ist eine grundsätzliche Diagnose derartiger Erkrankungen unter Verwendung der erfindungsgemäßen Nukleinsäuren im Rahmen der vorliegenden Erfindung. Im Rahmen eines entsprechenden Diagnoseverfahrens wird bevorzugterweise die Konzentration des Zielmoleküls über die Wechselwirkung mit einer oder mehrerer der erfindungsgemäßen Nukleinsäuren ermittelt. Eine derartige Wechselwirkung kann beispielsweise dadurch nachgewiesen werden, dass in einem kompetitiven Testansatz zur Bestimmung der Konzentration von (Arg⁸)-Vasopressin in Körperflüssigkeiten die Bindung eines markierten Vasopressin-Tracers an die erfindungsgemäße Nukleinsäure durch das aus der Körperflüssigkeit stammende (Arg8)-Vasopressin kompetitiert wird.

Die Erkrankungen, zu deren Behandlung die erfindungsgemäßen Nukleinsäuren verwendet werden können, stehen direkt oder indirekt im Zusammenhang mit einem gegenüber normalen physiologischen Bedingungen erhöhten Vasopressingehalt in dem zu behandelnden Lebewesen, insbesondere einem Säugetier und bevorzugterweise einem Menschen. Bevorzugterweise ist der erhöhte Vasopressingehalt ein erhöhter Titer an Vasopressin in einer Körperflüssigkeit, wobei die Körperflüssigkeit bevorzugterweise Blut ist.

Eine Erkrankung, für deren Behandlung die erfindungsgemäßen Nukleinsäuren verwendet werden können, ist die kongestive Herzinsuffizienz (CHF). Dabei ist es im Rahmen der vorliegenden Erfindung, dass die erfindungsgemäßen Nukleinsäuren für die kurzzeitige Therapie von kongestiver Herzinsuffizienz verwendet werden. Eine weitere Ausführungsform der mittels der erfindungsgemäßen Nukleinsäuren zu behandelnden oder zu verhindernden Herzinsuffizienz ist die akute dekompensierte kongestive Herzinsuffizienz.

Es ist auch im Rahmen der vorliegenden Erfindung, dass die erfindungsgemäßen Nukleinsäuren für die Behandlung von Bluthochdruck, koronarer Herzerkrankung, Herzinfarkt und Angina pectoris verwendet werden. In einer besonders bevorzugten Ausführungsform sind Bluthochdruck, koronare Herzerkrankung, Herzinfarkt und Angina pectoris Erkrankungen, die letztendlich zu kongestiver Herzinsuffizienz führen, die insoweit das Endstadium dieser Erkrankungen darstellen kann. In einer bevorzugten Ausführungsform ist die kongestive Herzinsuffizienz in den hierin beschriebenen Ausführungsformen ebenso wie die anderen erwähnten Erkrankungen, insbesondere koronare Herzkrankheiten, Herzinfarkt, Angina pectoris und Bluthochdruck eine solche, die insbesondere bei älteren Menschen auftritt oder bei Patienten mit einem oder mehreren Herzinfarkten. Ältere Patienten sind in einer bevorzugten Ausführungsform insbesondere solche, deren körperliche Leistungsfähigkeit altersbedingt gegenüber der maximalen Leistungsfähigkeit verringert ist.

Weitere Erkrankungen, bei denen die erfindungsgemäßen Medikamente zur Behandlung und/oder Prävention verwendet werden können, sind Hypertrophie des Herzmuskels, insbesondere durch Arginin-Vasopressin vermittelte Hypertrophie des Herzmuskels, Ödeme, und Hyponatriämie.

Eine weitere Erkrankung, die unter Verwendung der erfindungsgemäßen Nukleinsäuren behandelt bzw. verhindert werden kann, ist das Syndrom der inadäquaten ADH-Sekretion.

Beim Syndrom der inadäquaten ADH-Sekretion (SIADH, Schwartz-Bartter Syndrom) ist bedingt durch verschiedenste Ursachen wie beispielsweise ektope ADH-Sekretion durch Tumore, medikamentöse Induktion durch verschiedene Psychotropika wie Carbamazepin, Neuroleptika, trizyklische Antidepressiva oder selektive Serotonin Wiederaufnahme-Hemmer, Erkrankungen der Lunge z.B. Tuberkulose oder Bronchialkarzinom, oder Veränderung der zentralen Osmorezeptoren nach Schädel-Hirn-Trauma oder Meningitis, die AVP Konzentration im Plasma dauerhaft erhöht, was zur Antidiurese und zur Hyponatriämie und Hypoosmolarität führt (Baylis P H 2003 Int. J. Biochem. Cell. Biol. 35:1495-1499). Da die Symptome der Krankheit ursächlich mit der erhöhten AVP Konzentration im Plasma zusammenhängen, können die erfindungsgemäßen Nukleinsäuren zur Behandlung und/oder Prävention der inadäquaten ADH-Sekretion bzw. des Syndroms der inadäquaten ADH-Sekretion verwendet werden. In Tierstudien und bei SIADH Patienten konnte die Wirksamkeit eines V₂-Rezeptorantagonisten bei der Behandlung dieser Krankheit gezeigt werden (Saito T et al 1997 J.Clin. Endocrinol. Metab. 82: 1054-1057).

In einem weiteren Aspekt betrifft die Erfindung die Verwendung der erfindungsgemäßen Nukleinsäuren zur Herstellung eines Medikamentes zur Behandlung und/oder Prävention von Leberzirrhose und Hyponatriämie.

Bei der Leberzirrhose führt eine periphere Vasodilatation zu einer deutlich erhöhten Plasmakonzentration an AVP, was wegen der darauf folgenden verringerten Wasserausscheidung zu Hyponatriämie und Hypoosmolarität führt. Da hier im Stand der Technik mit Diuretika therapiert wird, die die Hyponatriämie noch verstärken können, sind die erfindungsgemäßen Nukleinsäuren von Vorteil, da sie so wie die V₂-Rezeptorantagonisten aufgrund des Wirkmechanismus von AVP spezifisch nur die Wasserausscheidung verstärken und eine bestehende Hypoosmolarität und Hyponatriämie normalisieren. In Patienten mit Leberzirrhose konnte nach Einmalgabe eines V₂-Rezeptorantagonisten eine deutliche Zunahme des Urinvolumens und Abnahme der Urinosmolalität nachgewiesen werden. Allerdings zeigte sich dosisabhängig eine Zunahme des AVP Plasmaspiegels, der bei dauerhafter Medikation möglicherweise die diuretischen Effekte des Rezeptorantagonisten wieder aufhebt. (Inoue T et al 1998 Clin. Pharmacol. Ther. 63: 561-570). Die vorliegenden Erfinder gehen derzeit davon aus, dass die erfindungsgemäßen Nukleinsäuren bei der Behandlung von Hyponatriämie bei der Leberzirrhose keinen Anstieg des AVP-Plasmaspiegels verursachen, so daß sie gegenüber dem V₂-Rezeptorantagonisten deutlich im Vorteil wären.

In einem weiteren Aspekt können die erfindungsgemäßen Nukleinsäuren für die Prävention und/oder Behandlung von Hirnödem eingesetzt werden. Dabei ist es im Rahmen der vorliegenden Erfindung, dass die erfindungsgemäßen Nukleinsäuren auch zur Behandlung von Schädel-Hirn-Trauma oder zur Behandlung von Schlaganfall verwendet werden. Es ist im Rahmen der vorliegenden Erfindung, dass die Verwendung der erfindungsgemäßen Nukleinsäuren zur Behandlung und/oder Prävention von Hirnödem verwendet werden, wobei dieses insbesondere aus einem Schädel-Hirn-Trauma oder einem Schlaganfall resultiert.

Bei der Behandlung von Hirnödemen nach Schädel-Hirn-Trauma oder Schlaganfall werden im Stand der Technik unterstützend Schleifendiuretika wie Furosemid eingesetzt. Diese Medikamente können jedoch aufgrund ihrer Wirkungsweise, die mit einem vermehrten Verlust an Na⁺, K⁺, und Cl⁻-lonen einhergeht, zu einer Störung des Elektrolytgleichgewichts der Körperflüssigkeiten und damit zu Hyponatriämie und/oder Hypokaliämie führen. Bei der Verwendung der erfindungsgemäßen Nukleinsäuren, die ebenfalls diuretisch wirken, treten Störungen im Elektrolythaushalt jedoch nicht auf, da es durch die Hemmung der Wasserrückresorption im Sammelrohr der Niere nicht zum Ionenverlust, sondern nur zu einer vermehrten Wasserausscheidung kommt. In einem Tiermodel für das Hirnödem nach Schädel-Hirn-Trauma konnte mit einem V₂-Rezeptor Antagonisten die Ausbildung des Hirnödems dosisabhängig unterbunden werden (Lazlo F F 1999 Eur. J. Pharmacol. 364: 115-122).

In einem weiteren Aspekt betrifft die Erfindung die Verwendung der erfindungsgemäßen Nukleinsäuren zur Behandlung und/oder Prävention eines Tumors. Bei dem Tumor handelt es sich insbesondere um einen solchen, der einen oder mehrere Rezeptoren exprimiert, die aus der Gruppe ausgewählt sind, die V₁-Rezeptoren, V₂-Rezeptoren und V₃-Rezeptoren umfasst.

Bei Tumoren der Hypophyse, die ACTH ausschütten und auch beim ektopen ACTH Syndrom, ist der V3-Rezeptor überexprimiert (De Keyser Y 1996 J. Clin. Invest. 97: 1311-1318). Da die mitogene Wirkung von AVP für eine ganze Reihe von Zelltypen (s.o.) bekannt ist, allerdings vermittelt durch den V₁-Rezeptor, ist AVP per Stimulation via V₃-Rezeptor an der Tumorgenese ACTH sekretierender Tumoren beteiligt. Infolge dieses Wirkmechanismus sowie dem der erfindungsgemäßen Nukleinsäuren können diese inhibierend bei der Tumorgenese verwendet werden.

Beim kleinzelligen Bronchialkarzinom, einer Unterform des Bronchialkarzinoms, das in der westlichen Welt die Hauptursache der Krebstodesfälle bei Männern und die zweithäufigste Ursache bei Frauen ausmacht, werden alle drei bekannten AVP Rezeptoren und AVP selbst exprimiert. Das gleiche gilt für das Mammakarzinom, das bei Frauen eines der häufigsten Malignome darstellt (North W G 2000 Exp. Physiol. 85: 27-40). Das Vorkommen von AVP und aller drei AVP Rezeptoren in den genannten Tumoren begründet das Verständnis der vorliegenden Erfinder, dass AVP eine wesentliche Rolle bei der Physiologie dieser Tumore spielt. Infolge dessen können die erfindungsgemäßen Nukleinsäuren auch zur Inhibierung der Tumoren und speziell der vorstehend genannten Tumoren verwendet werden.

In einem weiteren Aspekt betrifft die vorliegende Erfindung die Verwendung der erfindungsgemäßen Nukleinsäuren zur Verhinderung von Frühgeburten.

AVP und Oxytocin stimulieren über ihre jeweiligen Rezeptoren in der Gebärmutter die Uteruskontraktion und sind entscheidend für die Einleitung der Geburt. Bei Frühgeburten, die einerseits enorme Gesundheitsrisiken für die Zufrühgeborenen bergen und andererseits das öffentliche Gesundheitssytem enorm belasten, sind die beiden Hormone ebenfalls beteiligt, denn durch das Oxytocin-Analogon Atosiban, welches den V₁-Rezeptor sowie den Oxytocin Rezeptor blockiert, können Frühgeburten effektiv verhindert werden (Akerlund M 2002 Prog. Brain. Res. 139: 359-365). Dies begründet die Verwendung der erfindungsgemäßen Nukleinsäuren zur Verhinderung von Frühgeburten.

In einem weiteren Aspekt betrifft die vorliegende Erfindung die Verwendung der erfindungsgemäßen Nukleinsäuren zur Behandlung und/oder Prävention der primären Dysmenorrhö.

Bei der primären Dysmenorrhö kommt es bedingt durch starke Uteruskontraktionen während und zum Teil schon vor der Menstruation zu kolikartigen Unterleibsschmerzen. Frauen, die daran leiden, weisen einen erhöhten AVP-Plasmaspiegel auf. Mit dem synthetischen Oxytocin-Analogon Atosiban und dem nichtpeptidalen Rezeptorantagonisten SR 49059, die beide gleichermaßen den V₁-Rezeptor und den Oxytocin Rezeptor blockieren, lassen sich therapeutisch positive Effekte erzielen (Akerlund M 2002 Prog. Brain. Res. 139: 359-365). Dies begründet die Verwendung der erfindungsgemäßen Nukleinsäuren zur Behandlung und/oder Prävention von primärer Dysmenorrhö.

Die erfindungsgemäßen Nukleinsäuren können weiterhin als Ausgangsmaterial für das Design von pharmazeutischen Wirkstoffen (engl. *drug design*) verwendet werden. Grundsätzlich gibt es hierzu zwei mögliche Ansätze. Ein Ansatz besteht in dem Screening von Bibliotheken von Verbindungen, wobei derartige Bibliotheken von Verbindungen bevorzugterweise Bibliotheken von niedermolekularen Verbindungen (engl. *low* oder *small molecules*) sind. Derartige Bibliotheken sind den Fachleuten auf diesem Gebiet bekannt. Alternativ können gemäß der vorliegenden Erfindung die Nukleinsäuren für das rationale Design von Wirkstoffen verwendet werden.

Das rationale Design von Wirkstoffen kann dabei seinen Ausgangspunkt von irgendeiner der Nukleinsäuren gemäß der vorliegenden Erfindung nehmen und umfaßt eine Struktur, insbesondere eine dreidimensionale Struktur, die ähnlich der Struktur der erfindungsgemäßen Nukleinsäure(n) ist oder identisch ist zu dem Teil der Struktur der erfindungsgemäßen Nukleinsäure(n), die die Bindung an Vasopressin vermittelt. In einem jeden Fall zeigt eine derartige Struktur noch das gleiche oder ein zumindest ähnliches Bindungsverhalten, wie die erfindungsgemäße(n) Nukleinsäure(n). In entweder einem weiteren Schritt oder als ein alternativer Schritt wird bei dem rationalen Design von Wirkstoffen die bevorzugterweise dreidimensionale Struktur jener Teile der an Vasopressin bindenden Nukleinsäuren durch chemische Gruppen nachgeahmt, die bevorzugterweise verschieden sind von Nukleotiden und Nukleinsäuren. Durch dieses Nachahmen, auch als Mimikri bezeichnet, kann eine Verbindung konstruiert werden, die von der Nukleinsäure bzw. den Nukleinsäuren verschieden ist, die als Ausgangsmaterialien für das rationale Design des Wirkstoffes verwendet wurde. Eine derartige Verbindung oder Wirkstoff ist bevorzugterweise ein kleines Molekül (engl. *small molecule*) oder ein Peptid.

Im Falle des Screenings von Verbindungsbibliotheken unter Verwendung kompetitiver Tests, die den Fachleuten auf dem Gebiet bekannt sind, können geeignete Vasopressin-Analoga, Vasopressin-Agonisten oder Vasopressin-Antagonisten, gefunden werden. Derartige kompetitive Assays können wie folgt aufgebaut sein. Die erfindungsgemäße Nukleinsäure, bevorzugterweise ein Spiegelmer, d. h. eine das Zielmolekül bindende L-Nukleinsäure, wird an eine bevorzugterweise feste Phase gekoppelt. Um Vasopressin-Analoga zu identifizieren, wird mit einer Markierung versehenes Vasopressin zu dem Testsystem hinzugegeben. Alternativ könnte auch das Vasopressin an eine feste Phase gekoppelt werden und die erfindungsgemäße Nukleinsäure könnte markiert sein. Ein potentielles Analogon bzw. ein potentieller Agonist oder Antagonist würde mit den Vasopressin-Molekülen, die an das Spiegelmer binden, kompetitieren, was mit einer Abnahme des Signals, das von der entsprechenden Markierung erhalten wird, einhergehen würde. Das Screenen auf Agonisten oder Antagonisten kann die Verwendung eines Zellkultur-Testsystems umfassen, das den Fachleuten auf dem Gebiet bekannt ist.

In einem weiteren Aspekt können die erfindungsgemäßen Nukleinsäuren infolge ihres charakteristischen Bindungsverhaltens an Vasopressin für die Targetvalidierung verwendet werden. Die erfindungsgemäßen Nukleinsäuren können in einem *ex vivo*-Organmodell verwendet werden, um die Funktion von Vasopressin zu studieren. Grundsätzlich existieren *ex vivo* Modelle, in denen Vasopressin Agonisten/Antagonisten getestet werden können, z.B. isolierte, perfundierte Nieren oder isolierte Aorten im Organbad.

Ein Kit gemäß der vorliegenden Erfindung kann wenigstens eine oder mehrere der erfindungsgemäßen Nukleinsäuren umfassen. Zusätzlich kann der Kit wenigstens eine oder mehrere Positiv- oder Negativkontrollen umfassen. Als Positivkontrollen kann z. B. Vasopressin verwendet werden, gegen das die erfindungsgemäße Nukleinsäure gescreent wurde, oder an die diese bindet, bevorzugterweise in flüssiger Form. Als Negativkontrolle kann unter anderem ein Peptid verwendet werden, welches sich hinsichtlich seiner biophysikalischen Eigenschaften ähnlich wie Vasopressin verhält, welches jedoch nicht durch die erfindungsgemäßen Nukleinsäuren erkannt wird oder ein Peptid mit gleicher Aminosäurezusammensetzung aber von Vasopressin verschiedener Sequenz.

Weiterhin kann der Kit einen oder mehrere Puffer umfassen. Die verschiedenen Bestandteile können in dem Kit in trockener oder lyophilisierter Form, oder gelöst in einer Flüssigkeit vorliegen. Der Kit kann eine oder mehrere Behältnisse umfassen, die wiederum ein oder mehrere der Bestandteile des Kits enthalten können. Bevorzugterweise enthalten die Gefäße Reaktionsansätze, wie sie für eine einmalige Durchführung eines Experimentes unter Verwendung einer oder mehrerer Bestandteile des Kits erforderlich sind.

Es ist weiter im Rahmen der vorliegenden Erfindung, dass die erfindungsgemäßen Nukleinsäuren zum Nachweis des Zielmoleküls wie Vasopressin verwendet werden können. Hierzu aber auch generell können die erfindungsgemäßen Nukleinsäuren direkt oder indirekt markiert werden. Bevorzugterweise ist die Markierung ausgewählt aus der Gruppe, die radioaktive Markierungen, Fluoreszenzmarkierungen oder für die Magnetresonanz-Spektroskopie geeignete Markierungen, wie beispielsweise Europium, umfasst.

Die vorliegende Erfindung wird weiter anhand der folgenden Figuren und Beispiele erläutert, aus denen sich weitere Merkmale, Ausführungsformen und Vorteile ergeben. Dabei zeigt
- Fig. 1: das Schema der automatischen RNA-Selektion;
- Fig. 2: eine schematische Darstellung des Arbeitsbereiches des verwendeten *in vitro* Se- lektionsroboters;
- Fig. 3: die Sequenz von AVP-bindenden Klonen nach der automatischen *in vitro* Selekti- on;
- Fig. 4: Sequenzen von AVP-bindenden Klonen und die Konsensus-Sequenz nach *in vitro* Selektion;
- Fig. 5: einen Vergleich des Bindungsverhaltens der Sequenzen CHF-157-B4 und CHF- 134-A9, dargestellt als Bindung an AVP in Prozent als Funktion der Nukleinsäu- rekonzentration;
- Fig. 6: Sequenzvarianten nach zielgerichteter Modifizierung und Verkürzung mit inter- nen PEG-Spacerbausteinen sowie eine hieraus abgeleitete Konsensus-Sequenz, wobei die Varianten unveränderte 5'- und 3'-Enden aufweisen;
- Fig. 7: Sequenzvarianten nach zielgerichteter Modifizierung und Verkürzung mit inter- nen PEG-Spacerbausteinen sowie eine hieraus abgeleitete Konsensus-Sequenz, wobei die Varianten veränderte 5'- und 3'-Enden aufweisen;
- Fig. 8: eine Darstellung des Sekundärstrukturmodells des AVP-bindenden Spiegelmers CHF-F-037;
- Fig. 9: ein Chromatogramm des 3'-aminomodifizierten Spiegelmers CHF-F-037-3'NH₂, das mittels IEX-HPLC erhalten wird;
- Fig. 10: ein Massenspektrum des 3'-aminomodifizierten Spiegelmers CHF-F-037-3'NH₂, das mittels MALDI-TOF erhalten wird;
- Fig. 11: ein Elektropherogramm des 3'-aminomodifizierten Spiegelmers, das mittels CGE (engl; *capillary gel electrophoresis*) erhalten wird;
- Fig. 12: ein Chromatogramm des mit einem 40 kDa PEG modifizierten Spiegelmers CHF- F-037-3'PEG, das mittels RP-HPLC erhalten wird;
- Fig. 13: die Inhibierung der AVP-V₂-Rezeptor-Wechselwirkung durch die Spiegelmere CHF-F-037 und CHF-F-037-3'PEG, dargestellt als Abnahme der Biosynthese von cAMP als Funktion der Konzentration des Spiegelmers in cAMP/Napf bei Stimu- lation mit 1 nM AVP;
- Fig. 14A: die Inhibierung der AVP-V₁-Rezeptor-Wechselwirkung durch CHF-F-037, darge- stellt als prozentuale Abnahme der intrazellulären Ca-Freisetzung als Funktion der Konzentration des Spiegelmers in Prozent von 100% Freisetzung bei Stimulation mit 5 nM AVP;
- Fig. 14B: die Inhibierung der AVP-V₁-Rezeptor-Wechselwirkung durch CHF-F-037- 3'PEG, dargestellt als prozentuale Abnahme der intrazellulären Ca-Freisetzung als Funktion der Konzentration des Spiegelmers in Prozent von 100% Freisetzung bei Stimulation mit 5 nM AVP;
- Fig. 15: Diagramme, die die Urinmenge verschiedener Gruppen Ratten nach unterschied- lichen Zeitspannen nach Substanzgabe darstellen;
- Fig. 16: Diagramme, die den Wasserverbrauch verschiedener Gruppen von Ratten nach unterschiedlichen Zeitspannen nach Substanzgabe darstellen;

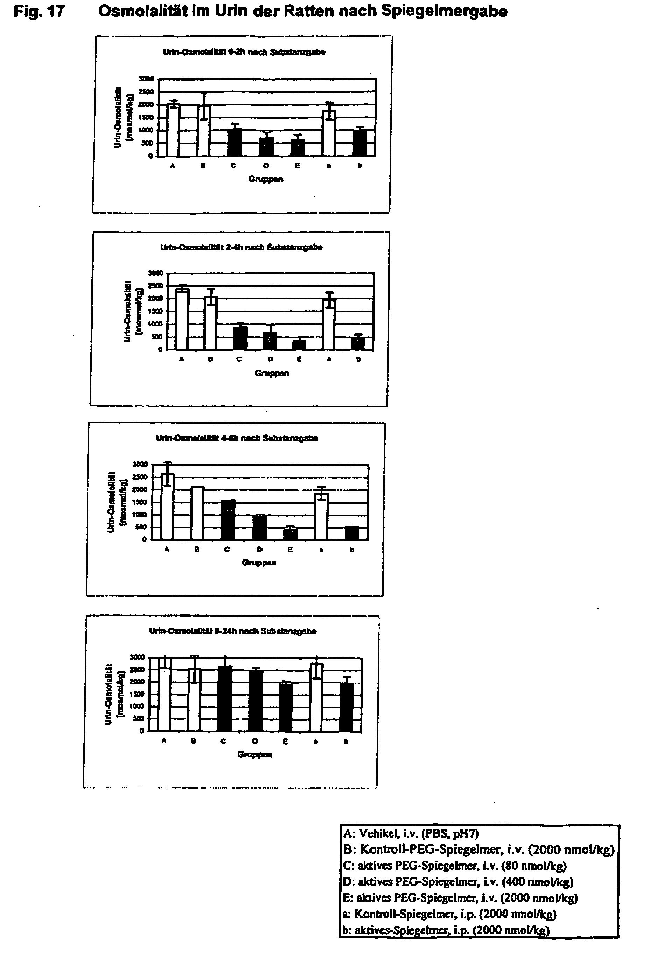

- Fig. 17: Diagramme, die die Osmolalität im Urin verschiedener Gruppen von Ratten nach unterschiedlichen Zeitspannen nach Substanzgabe darstellen; und
- Fig. 18: Diagramme, die den Na-Gehalt im Urin verschiedener Gruppen von Ratten nach unterschiedlichen Zeitspannen nach Substanzgabe darstellen.

### Beispiel 1: Automatische D-AVP-Selektion

### A. Materialien

### Feinchemikalien und Enzyme

NTPs und dNTPs wurden von Larova, Berlin bezogen. Die T7 RNA-Polymerase (50 U/µl) stammte von Stratagene, Heidelberg; DNase I von Sigma-Aldrich, Taufkirchen; thermostabile Vent exo⁻ DNA-Polymerase (2 U/µl) von New England Biolabs; SuperScript II Reverse Transkriptase (200 U/µl), sowie RNase Out RNase-Inhibitor (40 U/µl) von Invitrogen. Die Reagenzien für die RT-PCR im Rahmen der automatischen Selektion wurden von Qiagen, Hilden bezogen.

### Zielmolekül humanes D-AVP [D-(Arg⁸)-Vasopressin]

Das Nonapeptid D-AVP (Aminosäuresequenz: Cys-Tyr-Phe-Gln-Asn-Cys-Pro-Arg-Gly) wurde von BACHEM, Heidelberg synthetisiert. Das Molekül trägt am Carboxy-Terminalen Gly über ein zusätzlich eingefügtes Lysin eine Biotingruppe, um mittels der Biotin-Streptavidin-Wechselwirkung mit Affinitätschromatographie-Materialien wie NeutrAvidin-Agarose oder Streptavidin-UltraLink (Pierce) gebundene von ungebundenen Nukleinsäuren trennen zu können.

### Nukleinsäurebibliotheken und Oligonukleotidprimer

Der verwendete Startpool DE5-7.34 wurde bei der NOXXON Pharma AG, die für die RT-PCR verwendeten Oligonukleotid-Primer DE5.T7 bzw. DE5.R bei der IBA GmbH, Göttingen, mit Standard-Phosphoramiditchemie synthetisiert:
Bibliothek DE5-7.34; synthetisierter Reverse Strang
   5'-GTGGAACCGACTCACCTGAGCG-N₃₄-CGCTGCTGTTGTCTAAGCTCC-3'
Forward Primer DE5.T7
   5'-TCTAATACGACTCACTATAGGAGCTTAGACAACAGCAG-3'
Reverse Primer DE5.R
   5'-GTGGAACCGACTCACCTGAG-3'

Klonierung und Sequenzierung von angereicherten Pools wurde von AGOWA, Berlin im Auftrag durchgeführt.

### Herstellung des Startpools

Für die Generierung eines Startpools für die erste Selektionsrunde wurden 4 nmol synthetisierter einzelsträngiger DNA (ssDNA; DE5-7.34 initialer Pool, Reverse Strang), entsprechend einer Komplexität von ungefähr 2 x 10¹⁵ Molekülen in einem Volumen von 20 x 100 µl Taq Polymerase-Puffer mit 3-fachem Überschuss an DE5.T7 Primer durch Inkubation mit 400 U Taq Polymerase (5 U/µl) für 2 h bei 63°C zu doppelsträngiger DNA (dsDNA) aufgefüllt. Ausgehend von dem entstandenen doppelsträngigen T7-RNA-Polymerase-Promotor wurde in 2 ml Reaktionsvolumen 4 nmol dsDNA in T7 Transkriptionspuffer (80 mM HEPES pH 7,5; 22 mM MgCl₂; 1 mM Spermidin; 10 mM Dithiothreitol; je 4 mM GTP, ATP, CTP und UTP; 120 µg/ml BSA) in den entsprechenden RNA-Startpool transkribiert. Nach der Transkriptionsreaktion (Details s. Abschnitt C - Enzymatische Reaktionen) wurde die Template-DNA mit DNAse I verdaut, die RNA über ein 8%-iges denaturierendes Polyacrylamidgel gereinigt, mit Alkohol gefällt und getrocknet.

### B. Selektionsschritte

### Denaturierung und Faltung der RNA

Alle nicht-enzymatischen Schritte der Selektion mit Ausnahme der Denaturierung der RNA vor dem Kontaktieren mit dem Zielmolekül D-AVP wurden in Selektionspuffer (20 mM Tris-HCl, pH 7,4; 150 mM NaCl; 5 mM KCI; 1 mM MgCl₂; 1 mM CaCl₂; 0,1% [w/vol] Tween-20) durchgeführt. Die Denaturierung erfolgte für 5 Minuten bei 95°C in Selektionspuffer ohne CaCl₂ und MgCl₂. Nach der Denaturierung wurde die RNA auf Eis abgekühlt, MgCl₂ und CaCl₂ wurden zugegeben und weitere 5-15 Minuten bei 37°C inkubiert. In den ersten, manuellen Runden, wurden 4-8 nmol RNA-Pool verwendet, in den späteren automatischen 0,2 nmol.

### Anbindung und Abtrennung von Matrix-bindenden RNA-Spezies

Im Anschluss an die Faltung wurde die RNA zunächst bei 37°C für 15 Minuten ohne Peptid mit der Matrix (Streptavidin-UltraLink Plus oder NeutrAvidin-Agarose; beide Matrices von Pierce) unter Schütteln inkubiert. Diese sogenannte Präselektion diente der Entfernung von potentiellen Matrixbindern. Nach diesem Inkubationsschritt wurde die ungebundene RNA von der Matrix mittels Zentrifugation getrennt, der Überstand abgenommen und die Matrix mit zwei Säulenvolumen Selektionspuffer gewaschen. Der Überstand und die Waschfraktionen wurden vereinigt und für die weitere Selektion verwendet. Bei automatischer Durchführung wurde die Matrix durch einfaches Sedimentierenlassen abgetrennt und nur der Überstand weiterverwendet.

### Selektion von D-AVP-bindenden Ribonukleinsäuren

Für die Bindungsreaktion an D-AVP wurden die verbliebenen Nukleinsäurespezies mit verschiedenen Konzentrationen von biotinyliertem D-AVP versetzt und für 1-3 Stunden bei 37°C inkubiert. Daraufhin wurde dem Bindungsansatz 10-80 µl der Biotin-bindenden Matrix zugesetzt und nochmals unter Schütteln für 10-15 min bei 37°C inkubiert. Anschließend wurde die Matrix mit Selektionspuffer gewaschen, um nicht-bindende RNA-Spezies von bindenden zu entfernen. Das hierbei verwendete Waschvolumen lag in den ersten drei manuellen Runden beim 2-fachen Volumen der Matrix, in späteren Runden beim bis zu 135-fachen Festphasenvolumen.

### Elution

Während der ersten drei, manuell durchgeführten Selektionsrunden wurde die gebundene RNA durch zweimaliges Inkubieren der Matrixpartikel mit angebundener RNA in 150 µl in 4 M Guanidin-Thiocyanat (Roth) für 15 min bei 37°C unter denaturierenden Bedingungen eluiert. Diese Prozedur wurde nach Abnahme des Üerstandes nochmals bei 65°C wiederholt. Die eluierte RNA in den vereinigten Überständen wurde einmal mit wassergesättigtem Phenol/Chloroform/Isoamylalkohol (25:24:1) sowie zweimal mit Chloroform/Isoamylalkohol (24: 1) extrahiert, für 30 Minuten mit 1 Volumen Isopropanol bei -20°C gefällt, mit 70% EtOH gewaschen und getrocknet.

Im Zuge der weiteren automatischen Runden wurde die Elution durch Erhitzen der Matrixpartikel in RT-PCR-Puffer (Qiagen) auf 95°C für 10 min erreicht.

### C. Enzymatische Reaktionen

### Transkription - Herstellung von RNA zur Verwendung in der Selektion

Transkriptionen wurden mit 100 U T7 RNA-Polymerase und 25-40 U RNase Out RNase-Inhibitor in T7-Reaktionspuffer (80 mM HEPES pH 7,5; 22 mM MgCl₂; 1 mM Spermidin; 10 mM Dithiothreitol; je 4 mM GTP, ATP, CTP und UTP; 120 µg/ml BSA; 0,8 M Betain) in 100 µl Volumen durchgeführt. Pro 100 µl-Reaktion wurden 50-100 pmol RT-PCR-Produkt (manuelle Selektionsrunden) bzw. 10-30 µl RT-PCR Reaktion mit darin entstandener, doppelsträngiger DNA (automatischen Selektionsrunden) als Transkriptionstemplate eingesetzt.

Die Reaktionen wurden 3-12 Stunden bei 37°C inkubiert und anschließend mit DNase 1 versetzt, um die Template-DNA zu verdauen. Die erzeugte RNA wurde daraufhin entweder manuell unter denaturierenden Bedingungen über ein 8% Polyacrylamidgel mit 8 M Harnstoff oder aber automatisch mittels Ultrafiltration mit Ultrafiltrationseinheiten von nicht eingebauten NTPs abgetrennt. Ultrafiltrationsgereinigte RNA wurde vom Filter gespült, gelgereinigte RNA aus den ausgeschnittenen Gelstückchen eluiert, mit Ethanol gefällt, getrocknet und in Wasser aufgenommen.

### Reverse Transkription - manuelle Runden 1-3

Die gefällte RNA wurde mittels reverser Transkription in einzelsträngige DNA umgeschrieben. Höchstens 8 pmol pro 40 µl-Reaktion wurden mit 1 µM DE5.R Primer denaturiert und der Primer durch Abkühlen an die RNA hybridisiert. Anschließend wurden 8 µl 5 x RT-Puffer (First Strand Buffer, Invitrogen), 10mM DTT, 0,5 mM dNTP, 0,8 M Betain zugegeben und 2 min bei 48°C inkubiert. Dann wurde mit 5 U Superscript II Reverse Transkriptase gestartet und für 30 min bei 48°C; 20 min bei 50°C; 10 min bei 55°C und 15 min bei 70°C im Thermocycler inkubiert. Die dabei entstandene cDNA diente als Template für die nachgeschaltete PCR.

### Reverse Transkription - automatische Runden 4-16

Die gewaschenen Matrixpartikel mit Zielmolekül und daran gebundenen RNA-Spezies wurden in 120 µl Qiagen OneStep RT-PCR-Puffer (Primerkonzentration, je 1 µM) resuspendiert und 10 min bei 95°C inkubiert. Nach langsamem Abkühlenlassen zunächst auf 63°C und schließlich auf 50°C wurde die Reaktion durch Zugabe von jeweils 4 µl Enzyme Mix (Bestandteil des OneStep RT-PCR Kits von Qiagen) gestartet. Das Temperaturprofil bei der RT war wie folgt: 20 min 50°C; 2 min 53,3°C; 2 min 56,6°C; 10 min 60°C.

### PCR - Manuelle Runden 1-3

Jeweils 10 µl der RT-Reaktion wurden als Template für 3 PCRs ä 100 µl Volumen eingesetzt (10 µl 10x Vent-Puffer [New England Biolabs]; 1 µl 50 mM MgSO₄; 2 µl 10 mM dNTP-Mix; 3 µl 100 µM DE5.T7 Primer; 3 µl 100 µM DE5.R Primer; 16 µl 5 M Betain; 2,5 µl Vent exo⁻ DNA-Polymerase). Die Amplifizierung erfolgte über ein Thermocylerprogramm mit dem folgenden Profil:. 95°C,1 min; 63°C, 1 min; 72°C, 1 min; im Ganzen 6-10 Zyklen. Der Amplifikationsfortschritt wurde nach der Reaktion auf einem nativen Polyacrylamidgel kontrolliert. Die PCR Reaktion wurde anschließend mit Alkohol gefällt, das Pellet mit 70% Ethanol gewaschen und getrocknet. Die DNA wurde in H₂O gelöst und 50-100 pmol davon als TranskriptionsTemplate für die nächste Selektionsrunde eingesetzt.

### PCR - Automatische Runden 4-16

Die PCR wurde durch Inkubation der OneStep RT-PCR-Ansätze für 15 min bei 95°C gestartet. Es folgten 7-16 Thermozyklen (95°C, 30 s; 63°C, 30 s; 72°C, 30 s).

Während der automatischen Runden wurde der Verlauf der PCR mittels Fluoreszenzmessung von Aliquots aus den PCR-Reaktionen überwacht und die PCR nach Erreichen des erforderlichen Schwellenwertes abgebrochen (s. Abschnitt D, Kontrolle des PCR-Fortschrittes bei der automatischen Selektion)

### D. Kontrolle des PCR-Fortschritts bei der automatischen Selektion

Die Zunahme an doppelsträngiger DNA während der PCR wurde halbquantitativ verfolgt. Dies diente dem Zweck, die Anzahl an PCR-Zyklen möglichst klein zu halten. Dadurch werden nur so viele PCR-Zyklen durchgeführt, wie nötig sind, um genügend Template für die T7-Reaktion zu erhalten. Während der PCR wurden daher ab einer gewissen Zyklenzahl Aliquots aus den PCR-Ansätzen entnommen und zu 90 µl einer PicoGreen-Lösung (verdünnt 1:400 in TE [10 mM Tris-HCl, pH 8; 1 mM EDTA]) gegeben. PicoGreen ist ein Fluoreszenzfarbstoff, der frei in Lösung kaum, an doppelsträngige DNA gebunden jedoch stark fluoresziert (Ex: 485 nm; Em: 520 nm). Messung der Fluoreszenz im Vergleich zu einer nicht gecycelten Kontrolle ohne thermostabile Polymerase erlaubt recht genaue Abschätzung des PCR-Fortschritts. Nach Erreichen des Schwellenwerts (Fluoreszenz gecycelt / Fluoreszenz ungecycelt > 2) kann ein Aliquot der RT-PCR-Reaktion als Template für die in vitro-Transkription dienen.

### E. Automatische Manipulationen

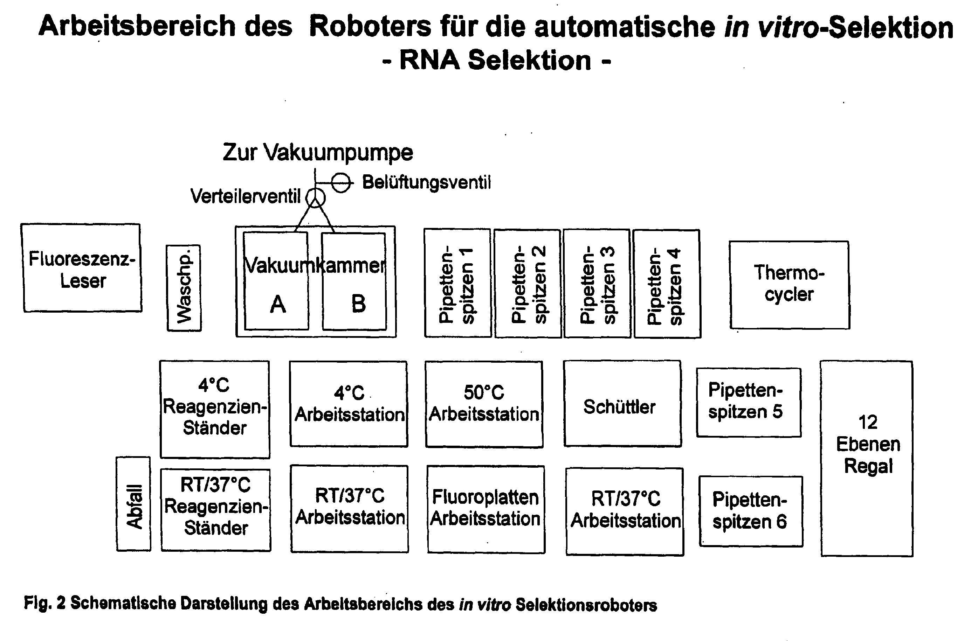

Ab der dritten Runde wurden alle Manipulationen außer unregelmäßig durchgeführten Gelreinigungsschritten auf einem Pipettierroboter vollautomatisch durchgeführt. Die Anordnung der einzelnen dabei verwendeten Module auf dem Arbeitsbereich des Roboters geht aus Fig. 2 hervor. Folgende Module wurden eingesetzt:
- Fluoreszenzleser zur Kontrolle des Amplifikationsfortschrittes während der PCR. Proben, in denen bereits ausreichend DNA erzeugt worden ist, werden vollautomatisch zwischengelagert und keinen weiteren Thermozyklen unterworfen
- Doppelvakuumkammer mit Kammer A für die Trennung von an die Matrix gebundenen und ungebundenen RNA-Spezies und Kammer B für die Aufreinigung von Transkriptionsreaktionen vor dem Beginn der jeweils nächsten Runde
- Halterungen für Pipettenspitzen
- Thermocycler zur Durchführung der PCR-Programme sowie für diverse Inkubationsschritte
- Schüttler zur Suspendierung von Matrix in Bindungs- oder Reaktionspuffer
- 50°C Arbeitsstation, um enzymatische Reaktionen direkt bei dieser Temperatur starten zu können ("hot start") bzw. um PCR-Reaktionen während der Probennahme zur Fluoreszenzkontrolle nicht zu sehr abkühlen zu lassen
- 4°C Arbeitsstation zur Zwischenlagerung von PCR- bzw. Transkriptionsreaktionen
- 4°C Reagenzienständer zur Aufbewahrung von wärmeempfindlichen Reagenzien wie Enzyme oder vorbereitete Reaktionsgemische
- RT/37°C Reagenzienständer zur Aufbewahrung von Waschpuffer
- RT/37°C Arbeitsstation zur Durchführung der meisten Manipulationen
- Fluoroplatten Arbeitsstation zur Vorbereitung der Proben zur Fluoreszenzmessung
- Hotel zur Lagerung von momentan nicht bearbeiteten Platten
- Abfallposition für Deponieren von gebrauchten Pipettenspitzen

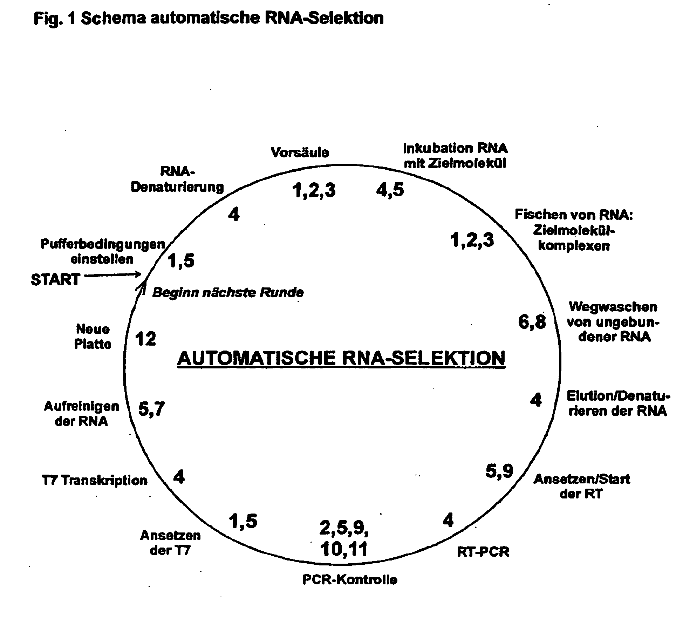

Die Beteiligung der einzelnen Module am im Rahmen dieses Beispiels beschriebenen Selektionsprozess sowie die Reihenfolge ihrer Benutzung geht aus Fig. 1 hervor.

### F. Resultate

### Selektionsverlauf

In jeder Selektionsrunde ab Runde 4 wurden drei unterschiedlich stringente Ansätze sowie eine Leersäule ohne D-AVP als Zielmolekül gefahren. Die Stringenz wurde sowohl durch Variation des verwendeten Waschvolumens als auch durch Verwendung von geringeren D-AVP-Konzentrationen erhöht.

Runden 1 bis 3 wurden manuell durchgeführt, weil die großen Mengen an Matrix, die für die Bindung des D-AVP in den (notwendigermassen hohen) Konzentrationen dieser initialen Runden erforderlich sind, nicht mehr sicher von Pipettierautomaten bearbeitet werden können. Ab Runde 4 wurde vollautomatisch selektiert.

Generell wurde die selektierte RNA des Stranges mit der höchsten Stringenz, d.h. der geringsten Peptidkonzentration bzw. des höchsten Waschvolumens, der bei der Amplifikation noch ein signifikantes Signal über der Nullkontrolle ergab, in die nächste Runde eingesetzt. Als Maß für dieses Signal galt die Anzahl an Zyklen, die notwendig war, um den Schwellenwert (s. "Kontrolle des PCR-Fortschritts") zu erreichen. Insgesamt wurden 16 Selektionsrunden durchgeführt.

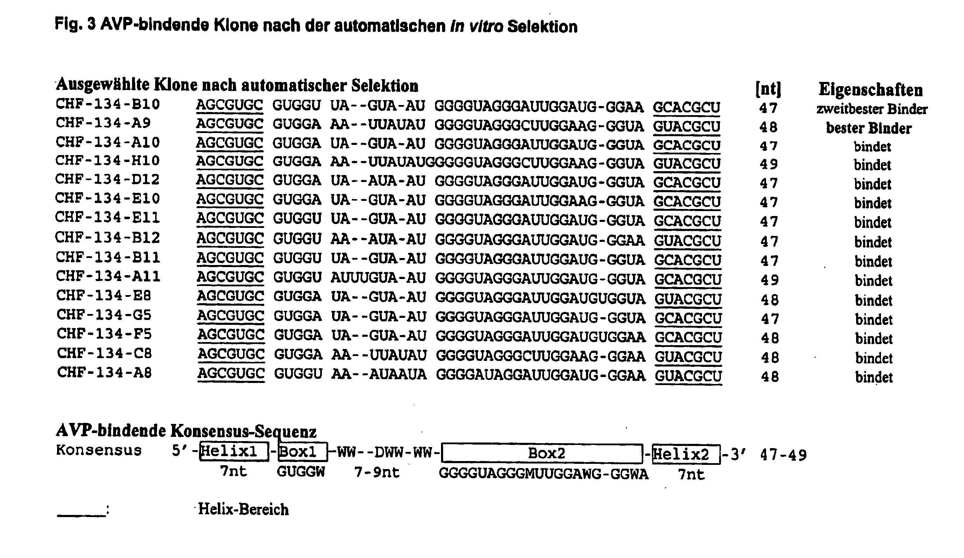

Die Populationen an dsDNA-Molekülen aus Runde 14 (D-AVP-Konzentration: 30 nM) und aus Runde 16 (D-AVP-Konzentration: 10 nM) wurden kloniert, und es wurden insgesamt 48 Klone sequenziert, die nach Ausrichtung (engl; *Alignment*) der Sequenzen zu mehreren Familien gruppiert werden konnten. In Fig. 3 ist die Familie der Moleküle mit den besten Bindungseigenschaften dargestellt. Alle Klone dieser Familie weisen die in Fig. 3 unterhalb des Sequenzalignments dargestellte allgemeine Konsensus-Sequenz mit den folgenden Elementen auf. Erstens zwei komplementäre Bereiche (Helix1 und Helix2) am 5'- und am 3'-Ende, welche eine sieben Basenpaare lange doppelsträngige Helix ausbilden; zweitens ein fünf Nukleotide langer Abschnitt (Box1) der Sequenz GUGGW; drittens ein sieben bis neun Nukleotide langen A- und U-reicher W-Abschnitt in dem niemals C und maximal ein G pro Abschnitt vorkommt; und viertens einem 21 Nukleotide langen Abschnitt (Box2) der Sequenz GGGGUAGGGMUUGGAWGGGWA. Hauptkennzeichen von Box2 sind vier doppelt bis vierfach vorkommende, strikt konservierte Gs. Alle dargestellten Moleküle binden an AVP und der beste Binder ist das Molekül CHF-134-A9. Neben den in Fig. 3 dargestellten AVP-bindenden Molekülen wurden weitere Moleküle mit völlig unterschiedlichen Sequenzen während der automatischen Selektion angereichert (Nicht gezeigt). Diese Moleküle wiesen deutlich schlechtere Bindungseigenschaften auf.

### Beispiel 2: Sreening von D-AVP-bindenden Aptameren im Anschluß an die automatische Selektion durch manuelle, mutagene Hochstringenzselektion

### A. Materialien

### Feinchemikalien und Enzyme

siehe Beispiel 1

### Zielmolekül humanes D-AVP [D-(Arg⁸)-Vasopressin]

Das Nonapeptid D-AVP (Aminosäuresequenz: Cys-Tyr-Phe-Gln-Asn-Cys-Pro-Arg-Gly, SEQ.ID. No.: 1) wurde in zwei Varianten von BACHEM, Heidelberg synthetisiert. Beide Varianten tragen eine Biotingruppe am Carboxyterminus, um mittels der Biotin-Avidin bzw. Streptavidin-Wechselwirkung mit den Affinitätschromatographie-Matrices NeutrAvidin-Agarose (NAag) und Streptavidin-UltraLink+ (SAul+), beide von Pierce, an das Peptid gebundene von ungebundenen Nukleinsäuren trennen zu können. Das Biotin ist am Carboxy-terminalen Gly über ein zusätzlich eingefügtes Lysin und für die beiden Varianten unterschiedliche Linker mit dem Peptid-Teil verbunden. Eine hydrophile PEG-Brücke, bestehend aus Amino-Ethoxy-Ethoxy-Acetylamino-Ethoxy-Ethoxy-Acetyl (AEEA-AEEA-Linker), führt zum biotinylierten D-AVP(C-Lys). Die hydrophobere ε-Aminohexyl-ε-Aminohexyl-Brücke führt zum biotinylierten D-AVP(C-LC-LC).

Grund für die Auswahl der unterschiedlichen Zielmolekül-Varianten war die Tatsache, daß die physikochemischen Eigenschaften bei kleinen Peptiden wie AVP durch Modifizierung mit chemischen Bausteinen wie Biotin relativ stärker verändert werden als bei großen Molekülen. Die Abweichung vom natürlichen Zielmolekül kann im Verlauf der Selektion zu unerwünschten Aptameren führen, die den Linker für hochaffine Bindung benötigen. Daher wurde bei der hochstringenten Selektion mit den zwei verschiedenen AVP-Derivaten im dauernden Wechsel und in wechselnder Kombination mit den beiden oben erwähnten Avidin Matrices gearbeitet, um die Anreicherung von Molekülen zu vermeiden, die für die Bindung an das Peptid die Linkerregion benötigen und die Anreicherung der erwünschten Moleküle verhindern, die hochaffin nur am reinen Peptid anbinden.

### Nukleinsäurebibliotheken und Oligonukleotidprimer

siehe Beispiel 1

### Startpool

Die manuelle, mutagene Hochstringenzselektion wurde mit doppelsträngiger Pool-DNA aus Runde 16 der automatischen Selektion gestartet. Dazu wurden 10 pmol Pool DNA aus Runde 16 durch mutagene PCR mit Vent exo⁻ thermostabiler DNA Polymerase amplifiziert, und mit T7 RNA Polymerase *in vitro* transkribiert. Die RNA wurde unter denaturierenden Bedingungen über ein 8% Polyacrylamidgel mit 8 M Harnstoff gereinigt, eluiert, mit Alkohol gefällt und in Wasser zur Selektion aufgenommen (Details siehe enzymatische Reaktionen).

### B. Selektionsschritte

### Denaturierung und Faltung der RNA; verwendete Menge an RNA

siehe Beispiel 1. Zu Beginn der Selektion mit mutagener Amplifikation wurden 500 pmol mutagenisierte Pool RNA in Runde 17 eingesetzt. Im Verlauf der Selektion wurde mit steigender Stringenz immer weniger RNA eingesetzt (5 pmol in Runde 29)

### Anbindung und Abtrennung von Matrix bindenden RNA-Spezies

siehe Beispiel 1

### Selektion von D-A VP-bindenden Ribonukleinsäuren

Für die Bindungsreaktion an D-AVP wurden die verbliebenen RNA-Spezies mit verschiedenen, im Laufe der Selektion immer geringer werdenden Konzentrationen von biotinyliertem D-AVP versetzt und für 30 min bei 37 °C inkubiert. Daraufhin wurde dem Bindungsansatz je nach eingesetzter Peptidmenge und verwendeter Matrix 3 - 20 µl Matrix zugesetzt und nochmals unter Schütteln für 10-15 min bei 37 °C inkubiert. Anschließend wurde die Matrix mit Selektionspuffer gewaschen, um nicht-bindende RNA-Spezies von bindenden zu entfernen. Das hierbei verwendete Waschvolumen lag zwischen dem 50- und 200-fachen Matrixvolumen.

### Elution

siehe Beispiel 1. Die Elution wurde wie bei den ersten drei, manuell durchgeführten Selektionsrunden der automatischen Selektion durchgeführt.

### C. Enzymatische Reaktionen

### Transkription - Herstellung von RNA zur Verwendung in der Selektion

siehe Beispiel 1. Die erzeugte RNA wurde ausschließlich manuell unter denaturierenden Bedingungen per Gelelektrophorese präpariert.

### Reverse Transkription

siehe Beispiel 1; *Reverse Transkription - manuelle Runden 1-3*

### PCR - nichtmutagen

Die revers transkribierte RNA wurde grundsätzlich zuerst unter nichtmutagenen Bedingungen amplifiziert (siehe Beispiel 1; *PCR - manuelle Runden 1-3*), um dann entweder direkt *in vitro* transkribiert zu werden oder vorher unter mutagenen PCR-Bedingungen amplifiziert zu werden.

### PCR - mutagen

Die DNA wurde mit einem zweistufigen PCR-Protokoll unter Reaktionsbedingungen amplifiziert, unter denen thermostabile DNA Polymerasen eine hohe Fehlerrate aufweisen, und die DNA mutieren. (Fromant et al 1995 Anal. Biochem. 224 : 347-353). 1 pmol der unter Standardbedingungen amplifizierten DNA wurde in 100 µl Reaktionsvolumen (10 mM Tris, pH 8,7; 50 mM KCI; 5 µg/ml BSA; 4,82 mM MgCl₂; 0,8 M Betain; 0,5 mM MnCl₂; 3,85 mM dTTP; 0,55 mM dGTP; 0,12 mM dATP; 0,1 mM dCTP; 2 µM DE5.R Primer; 2 µM DE5.T7 Primer) mit 8 U Taq DNA Polymerase bis zur Sättigung mit folgendem Temperaturprofil amplifiziert: 95°C, 1 min; 63°C, 1 min; 72°C, 3 min; insgesamt 10-16 Zyklen. Der Amplifikationsfortschritt wurde nach der Reaktion auf einem nativen 8% Polyacrylamidgel kontrolliert. 1 pmol der unter mutagenen Bedingungen amplifizierten DNA wurde erneut unter den oben genannten Bedingungen amplifiziert. Die DNA wurde nach der zweiten mutagenen PCR-Amplifikation mit Alkohol gefällt und *in vitro* transkribiert.

### D. Selektionsverlauf

Die manuelle Hochstringenzselektion mit mutagener PCR wurde für 13 Runden durchgeführt (Runde 17 bis 29 im Kontext der automatischen Selektion). Die Parameter hierfür sind in Tabelle 1 dargestellt.

**Tabelle 1**

| **Runde** | **Avidin-Matrix** | **D-AVP(C-Lys)** | **D-AVP(C-LC-LC)** | **RNA pool mutiert** | **RNA pool nicht mutiert** | **Signal/Kontroll Verhältnis** |
|---|---|---|---|---|---|---|
| 17 | NAag | | 5 nM | 10 nM | | 38 |
| 18 | NAag | 5 nM | | 10 nM | | 45 |
| 19 | SAul+ | 1 nM | | 10 nM | | 25 |
| 20 | SAul+ | | 200 pM | 10 nM | | 3 |
| 21 | NAag | | 40 pM | | 10 nM | 10 |
| 22 | NAag | 20 pM | | 10 nM | | 18 |
| 23 | SAul+ | 5 pM | | | 4 nM | 1,8 |
| 24 | SAul+ | | 4 pM | 2 nM | | 1,3 |
| 25 | NAag | | 1 pM | | 2 nM | größer 1 |
| 26 | NAag | 1 pM | | | 100 pM | größer 1 |
| 27 | NAag | | 1 pM | 100 pM | | größer 1 |
| 28 | SAul+ | | 0,4 pM | | 100 pM | größer 1 |
| 29 | NAag | 0,4 pM | | | 333 pM | größer 1 |

Dabei wurde im Laufe der Selektion die Peptidkonzentration als Hauptstringenzparameter bis auf 0,4 pM erniedrigt. Die RNA wurde im Überschuß eingesetzt, um durch Kompetition unter den Bindern nur die besten Moleküle zu selektieren. In jeder Runde wurden durch die gewählten Selektionsparameter und durch die Waschprozedur, (die Matrix wurde mit dem 50-200-fachen Festbettvolumen nach der Immobilisierung der Peptid-RNA-Komplexe gewaschen) Abreicherungsfaktoren von größer 10000 erreicht.

Das Ziel der hochstringenten Selektion bestand darin, aus dem D-AVP-bindenden RNA-Pool, der nach 16 Runden automatischer Selektion vorlag, weiter optimierte Binder durch Mutation und Selektion zu erzeugen. Generell wurden in jeder Runde Bindungsreaktionen mit unterschiedlichen Peptidkonzentrationen (Signal) und im Vergleich dazu eine Reaktion ganz ohne Peptid durchgeführt (Kontrolle). Die Reaktion, die bei niedrigster Peptidkonzentration noch ein Signal/Kontroll Verhältnis größer 1 aufwies wurde in die folgende Selektionsrunde weitergeführt. Die Tabelle zeigt nur die Parameter dieser Bindungsreaktionen.

Die DNA wurde in jeder der ersten vier Selektionsrunden mutagenisiert, um möglichst viel Variabilität im bindenden Pool zu erzeugen. In den folgenden Runden wurde abwechselnd nicht mutagen und mutagen amplifiziert, um verbesserte Binder vor weiterer Mutagenese jeweils anzureichern. Die letzten drei Runden wurden ohne weitere Mutagenese durchgeführt. Nach der 29. Runde wurde die DNA kloniert und die Sequenz von 24 Klonen bestimmt.

### Sequenzen

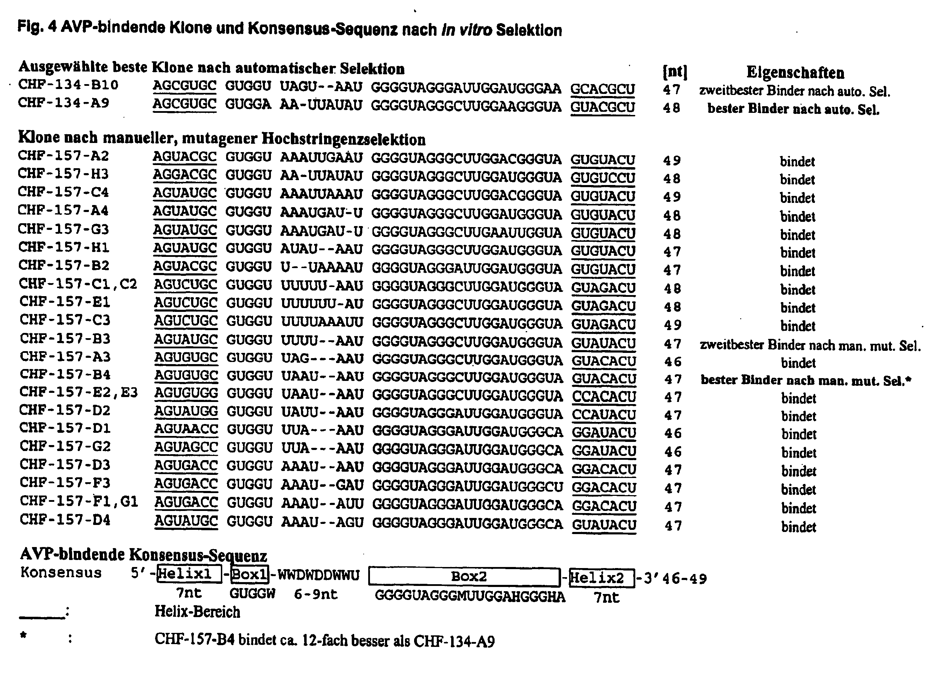

Das Ergebnis der Sequenzanalyse ohne die funktionell für die Bindung nicht notwendigen konstanten Bereiche beider Primer ist in Fig. 4 als Alignment dargestellt. Von den 24 Klonen, deren Länge ohne die flankierenden Regionen zwischen 46 und 49 nt liegt, sind drei komplett identisch. Alle anderen weisen eine sehr hohe Sequenzhomologie auf, die sie als Vertreter der Familie der besten Binder nach der automatischen Selektion ausweist. Die übrigen Familien, die nach der automatischen Selektion noch existierten, sind unter den Selektionsbedingungen der mutagenen Hochstringenzselektion verschwunden. Alle selektierten Moleküle weisen die typische bereits in Fig. 3 dargestellte Konsensus-Sequenz auf mit den vier Elementen Helix1 und Helix2 am 5'- bzw. 3'-Ende, Box1, A-und U-reicher W-Abschnitt und Box2. Unter den hochstringenten Selektionsbedingungen haben sich die vier Elemente wie folgt verändert. Helix1 und Helix2 weisen veränderte und unterschiedliche aber weiterhin komplementäre Sequenzen auf. Die fünfte Position von Box1 ist nur noch U statt vorher W (also kein A mehr). Im A- und U-reichen W-Abschnitt ist das Vorkommen von maximal einem G pro Abschnitt verringert. Nach der automatischen Selektion enhielten noch 9 von 15 Klonen, also 60% ein G pro Abschnitt, während es nach der hochstringenten Selektion nur noch 6 von 24 Klonen, also 25% sind. In Box2, wo nach der automatischen Selektion an zwei Positionen ein W vorkam, kommt nach der hochstringenten Selektion an diesen Positionen bei keinem Molekül mehr ein A vor, sondern neben U findet sich bei einigen Molekülen an diesen Positionen ein C. Weil insgesamt gesehen an diesen Positionen niemals ein G steht, wird in der Konsensus-Sequenz an diese beide Positionen ein H gesetzt. Somit ergibt sich aus der Sequenzanalyse der Klone von Runde 29 nach der manuellen Hochstringenzselektion und von Runde 16 nach der automatischen Selektion die in Fig. 4 gezeigte Konsensus-Sequenz für eine L-(Arg⁸)-Vasopressin-bindende L-RNA.

### Ranking

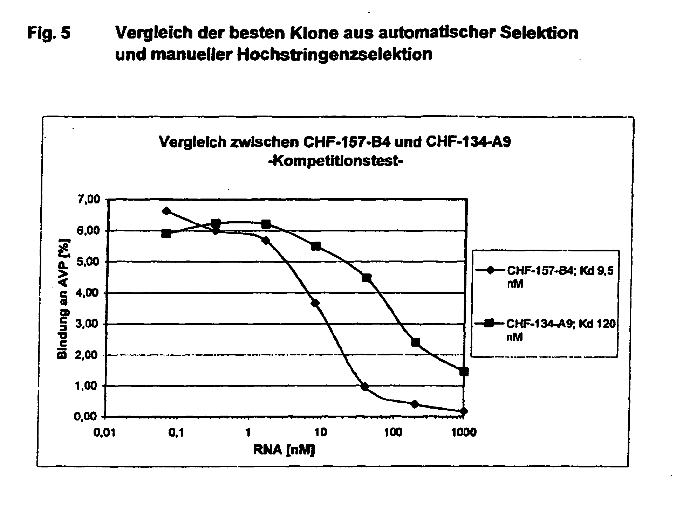

Die Klone wurden hinsichtlich ihrer Bindung an AVP getestet. Alle Klone zeigten Bindung an das Peptid. Der Klon CHF-157-B4 zeigte im Vergleich die beste Bindung. In einem Kompetitionstest wurde CHF-157-B4 im Vergleich zum besten Klon (CHF-134-A9 ) aus der 16. Runde nach automatischer Selektion gemessen. In diesem Test lag die Dissoziationskonstante von CHF-157-B4 für die Bindung an biotinyliertem AVP bei 9,5 nM während sie für CHF-134-A9 bei 120 nM lag, wie auch in Fig. 5 dargestellt. Durch die mutagene Hochstringenzselektion wurde die Güte der Bindung um etwa den Faktor 12 verbessert.

### Bespiel 3: Weiterentwicklung und Verkürzung von AVP-bindenden Aptameren durch Einbau interner PEG-Spacer-Bausteine

### Methodik

Nach dem zweistufigen Selektionsprozeß (automatische Selektion und manuelle, mutagene Hochstringenzselektion) wurden auf Basis des besten Binders gezielt Varianten konstruiert und chemisch synthetisiert, bei denen durch Einbau eines PEG-Spacerbausteins oder PEG-Gruppe (Hexaethylenglykol) intern Nukleosidpositionen ersetzt wurden. Dabei sollte das Molekül verkürzt und seine Bindungseigenschaften weiter optimiert werden. Das Aptamer CHF-157-B4 (für die weitere Entwicklung CHF-F-000 genannt) hatte sich als der beste Binder von allen selektierten Molekülen erwiesen. Ausgehend von diesem 47 nt langen Aptamer wurden die synthetisierten Varianten im kompetitiven Rankingsassay miteinander und mit dem Ausgangsmolekül verglichen und das beste, verkürzte Molekül wurde als Spiegelmer, d. h. als L-Nukleinsäure, synthetisiert.

### Synthese der PEG-Spacervarianten

Die konstruierten und getesteten Varianten wurden bei der NOXXON Pharma AG mit Standard-Phosphoramiditchemie unter Verwendung von Hexaethylenglykol-Phosphoramidit synthetisiert und nach der Entschützung durch preparative, denaturierende Gelelektroporese (12% PAA, 8 M Harnstoff, Tris-Borat-EDTA-Puffer) und anschließende Elektroelution gereinigt.

### Kompetitiver Rankingassay

Der Test basiert auf der Kompetition der AVP-Bindung eines radioaktiv markierten Aptamers durch nichtmarkierte Aptamere. Dabei dient die Stärke der Kompetition des markierten Aptamers durch sich selbst (nichtmarkiert) als Referenz, an dem die Güte der Varianten gemessen werden. Das Molekül, welches beim Vergleich am stärksten und besser als die Referenz kompetitiert, wird bei der Analyse weiterer Varianten als neue Referenz benutzt.

Das Referenzaptamer wurde durch T4 Polynukleotidkinase (Invitrogen) am 5'-Ende mit [y-³²P]ATP (400 Ci/mmol; Hartmann Analytic, Braunschweig) zu einer spezifischen Radioaktivität von 60000 - 120000 cpm/pmol markiert. Anschließend wurde es (0,2 nM) mit biotinyliertem D-AVP (0,5 nM) zusammen mit nichtmarkierten Aptameren im Überschuß oder ohne unmarkierte Aptamere als Kontrolle für 1 Stunde in Selektionspuffer inkubiert. Nach Immobilisierung an 1 µl SAul+-Matrix, Abnahme des Überstandes und Waschen der Matrix mit 50 - 100 µl Selektionspuffer wurden die Bindungsreaktionen im Szintillationszähler (Beckman, USA) gemessen.

### PEG-Spacervarianten des A VP-bindenden Aptamers CHF-F-000

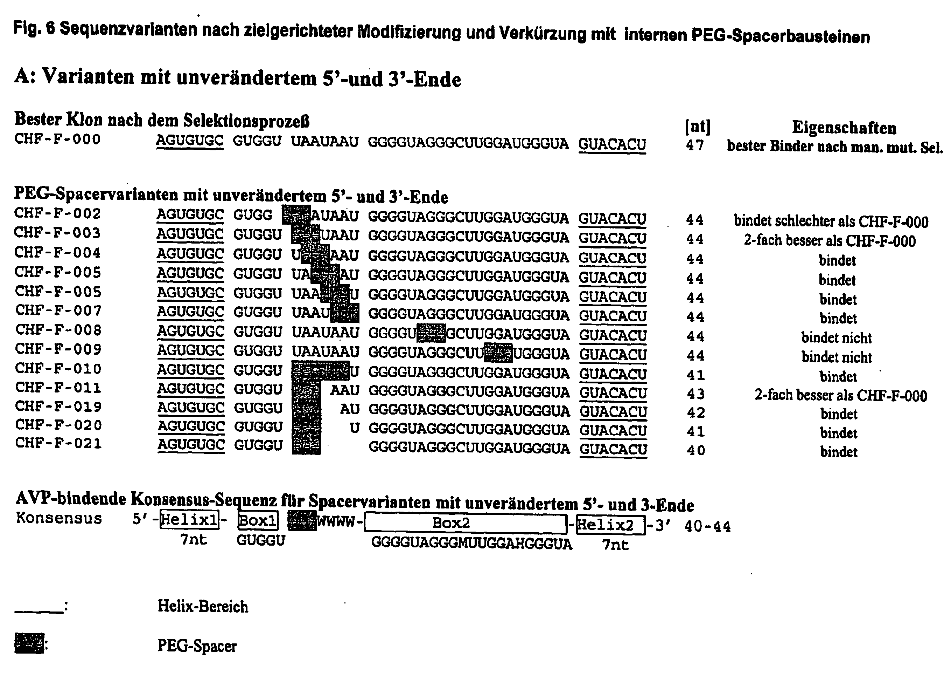

Anhand der Sequenzanalyse aller Klone aus Runde 16 nach der automatischen Selektion und Runde 29 nach der anschließenden Hochstringenzselektion kristallisierte sich der Adenosin/Uridin-reiche W-Abschnitt zwischen Box1 und Box2 als der Bereich heraus, in dem der Ersatz von Nukleosiden durch den internen PEG-Spacer am wahrscheinlichsten möglich war, um das Molekül zu verkürzen ohne an Funktionalität einzubüßen. Fig. 6 zeigt die getesteten Varianten, bei denen das 5'- und 3'-Ende unverändert blieb. Die Varianten CHF-F-008 und CHF-F-009 zeigten im Unterschied zu allen anderen getesteten Varianten keinerlei Bindung, da hier der PEG-Spacer in der für das Molekül offensichtlich essentiellen Box2 liegt. Am besten erwies sich die 44 Nukleotide lange Variante CHF-F-003, die um drei Nukleoside intern verkürzt ähnlich gut wie das 47 Nukleotide lange Ausgangsmolekül CHF-F-000 an AVP bindet.

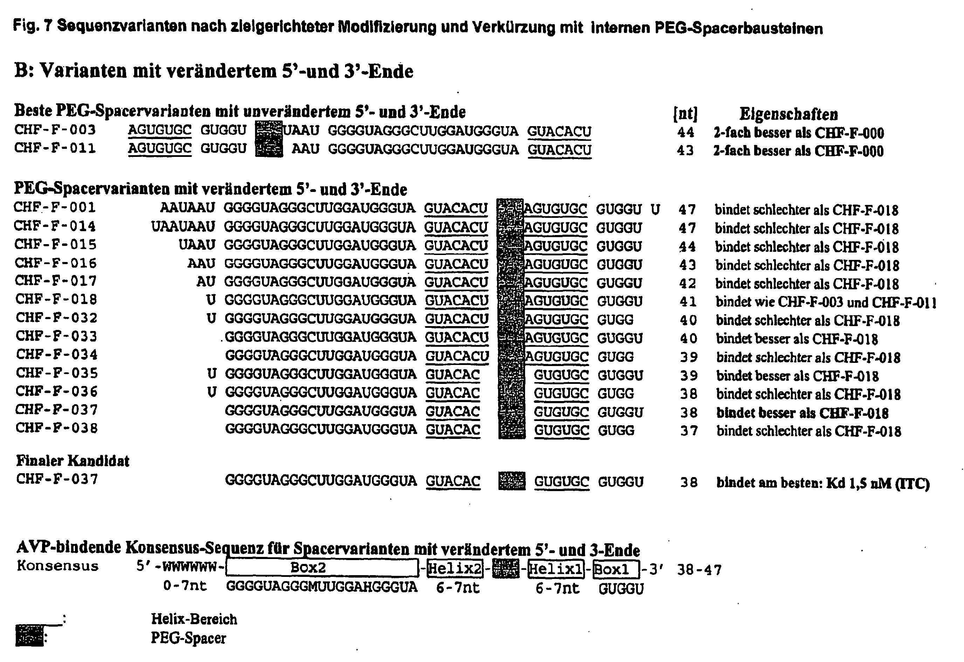

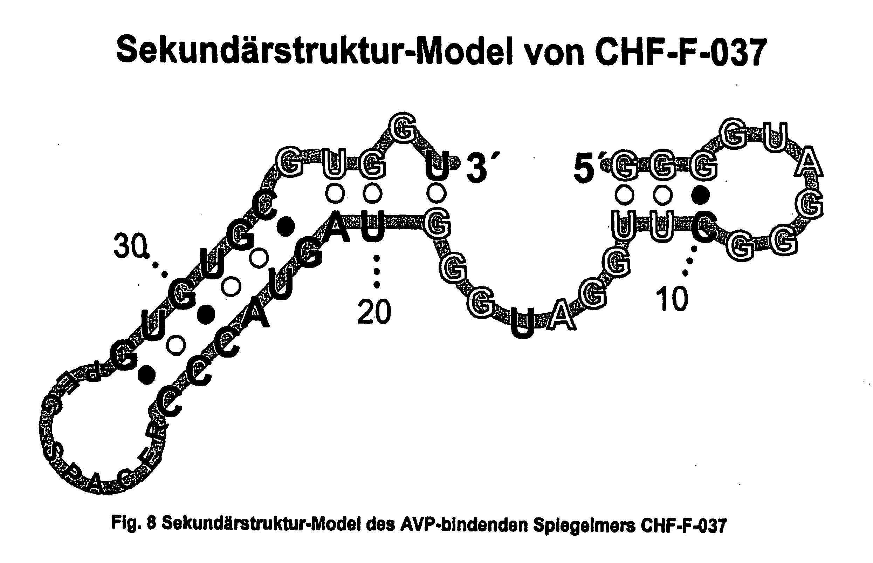

Neben den Varianten mit unveränderten Molekülenden wurde auch eine Reihe von Varianten getestet, bei denen das 5'- und das 3'-Ende in den Adenosin/Uridin-reichen W-Abschnitt gelegt wurde und der PEG-Spacer an die Basis der Helix verlagert wurde, wie in Fig. 7 dargestellt. Der jetzt offene Adenosin/Uracil-reiche W-Abschnitt wurde sukzessive verkürzt und parallel wurde die Helix um das äußerste Basenpaar verkürzt. Von den getesteten Molekülen, die alle noch an AVP binden, erwiesen sich CHF-F-033 (40 nt) und CHF-F-037 (38 nt) als am besten. Sie binden ähnlich gut, wie die Referenzmoleküle CHF-F-003 und CHF-F-000. Die vier Varianten, bei denen das U am 3'-Ende von Box1 entfernt wurde, binden signifikant schlechter, was die Bedeutung dieser Box1 unterstreicht und zeigt, dass für eine volle Funktionalität dieser Abschnitt aus GUGGU bestehen muß. Im Gegensatz dazu kann das U vom 3'-Ende des A- und U-reichen Abschnittes (s. CHF-F-033 und CHF-F-037) entfernt werden, ohne daß die Funktion darunter leidet. Da CHF-F-037, dessen Sekundärstruktur-Modell in Fig. 8 dargestellt ist mit 38 Nukleotiden von den getesteten Molekülen am kürzesten ist, ohne an Bindungsgüte zu verlieren, wurde es als Spiegelmer hergestellt, biophysikalisch und im Rahmen von Zellkultur-Tests analysiert, um schließlich die Wirksamkeit im Tierversuch zu demonstrieren.

### Beispiel 4: Chemische Synthese und Präparation des 3'-terminal PEGylierten Spiegelmers CHF-F-037-3'PEG

Das Spiegelmer wurde am 3'-Ende kovalent an 40 kDa großes Polyethylenglykol (PEG) gekoppelt, um die Auscheidung des Spiegelmers über die Nieren nach Applikation bei Tierstudien zu verzögern. Mit dem solchermaßen vergrößerten Spiegelmer erzielt man eine deutlich längere Verweildauer und damit eine verlängerte Wirksamkeit des solchermaßen vergrößerten Spiegelmers im Plasma.

### Chemische Festphasensynthese

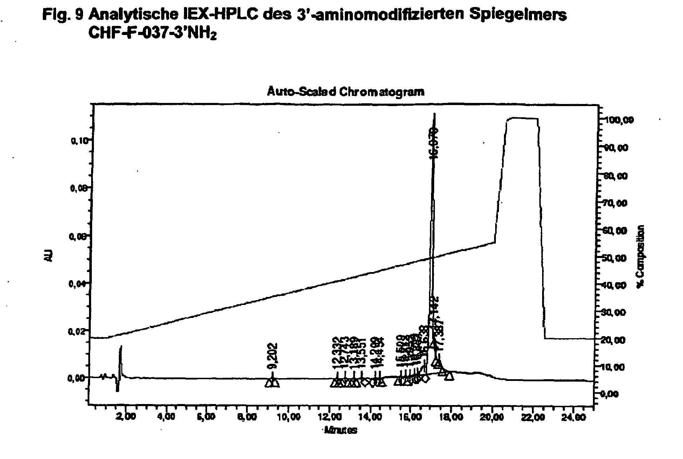

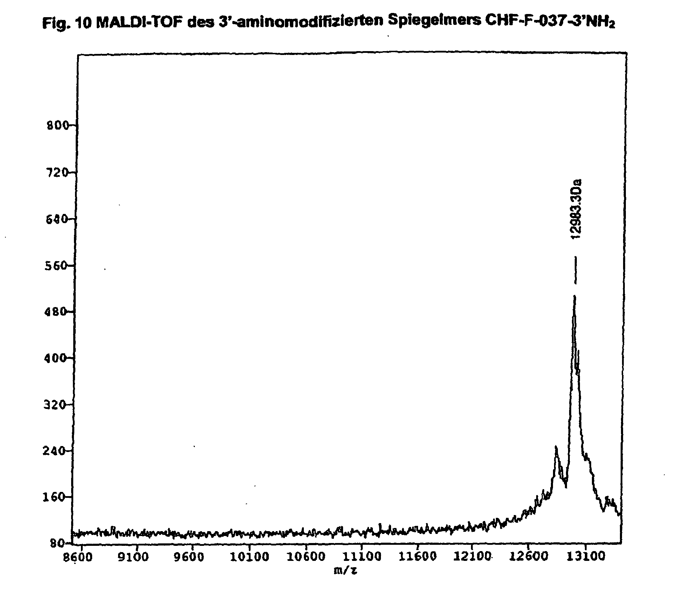

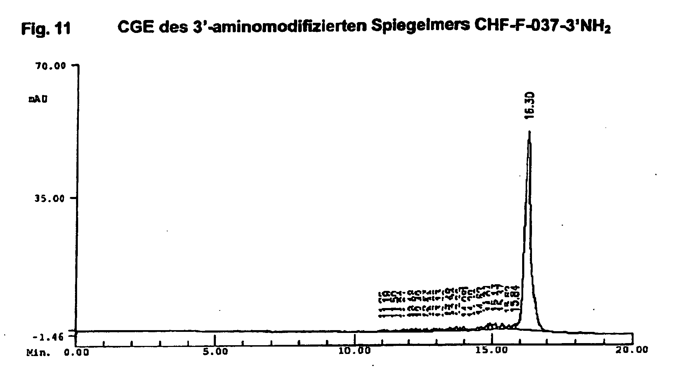

Das Spiegelmer wurden mittels Festphasen-Synthese auf einem ÄktaPilot100 Synthesiser, Amersham Biosciences (General Electric Healthcare, Freiburg), über 2'TBDMS RNA Phosphoramidit Chemie hergestellt (M.J. Damha, K.K. Ogilvie, Methods in Molecular Biology, Vol. 20 Protocols for oligonucleotides and analogs, ed. S. Agrawal, p. 81-114, Humana Press Inc. 1993). L-rA(N-Bz)-, L-rC(Ac)-, L-rG(N-ibu)-, L-rU- und Hexaethylenglycol-Phosphoramidit wurden von ChemGenes Corp., Wilmington, MA (USA) bezogen. Die Synthese wurde auf 3'-aminomodifiziertem C-6 CPG mit einer Porengröße von 1000Å, ChemGenes Corp., Wilmington, MA (USA), durchgeführt. Für die Kopplung wurde eine 0,3M Lösung von Benzylthiotetrazol, CMS-Chemicals, Abingdon (UK), in Acetonitril, 3,5eq. der entsprechenden 0,1M Amiditlösung in Acetonitril bei 15min Kopplungszeit verwendet. Es wurde ein Oxidations-Capping Zyklus benutzt. Weitere Standard-Lösungsmittel und Reagenzien, die bei der Oligonukleotidesynthese benutzt werden, wurden von Biosolve (Valkenswaard, NL) bezogen. Die Spiegelmere wurden DMT-ON synthetisiert und nach der Entschützung mittels präparativer RP-HPLC (Wincott F et al 1995 Nucleic Acids Res. 23: 2677) über Source15RPC Medium, Amersham Biosciences, gereinigt. Die 5'DMT-Gruppe wurde mit 80% Essigsäure (30min, RT) entfernt. Nach Zugabe von wässriger 2M NaOAc Lösung wurde das Spiegelmer über Tangential-Fluß-Filtration mit einer 5K regenerierten Zellulose Membran, Millipore Corp. Bedford, MA (USA), entsalzt. Die Ausbeute an gereinigtem 3'-aminomodifiziertem Spiegelmer war im Bereich von 60-75 OD/µmol. Die IEX-HPLC-, MALDI-TOF- and CGE-Analytik des gereinigten Spiegelmers ist in den Figs. 9, 10 und 11 gezeigt.

### PEGylierung

Zur PEGylierung (vgl. Europäische Patent Anmeldung EP 1 306 382) wurde das gereinigte 3'-aminomodifizierte Spiegelmer (18µmol) in einer Mischung von Wasser (2,5ml), DMF (5ml) and 5ml Puffer A gelöst. Zubereitung von Puffer A: Zu einer Mischung von Zitronensäure-Monohydrat (7,00g), Borsäure (3,54g), Phosphorsäure (2,26ml) und 1 M Natronlauge (343ml) wurde Wasser gegeben, so dass 11 der Stammlösung erhalten wurde. Diese Stammlösung wurde durch Zugabe von 1M Salzsäure auf pH 8,4 eingestellt).

Der pH-Wert der Spiegelmerlösung wurde durch Zugabe von 1M Natronlauge auf 8,5 eingestellt. Bei 37°C wurde 40kDa PEG-NHS Ester (Nektar Therapeutics, Huntsville, AL (USA)) in Portionen von 0,6 Äquivalenten/30min zugegeben bis bei 2,4 Äquivalenten ein maximaler Umsatz von 75-85% erreicht wurde. Während der Zugabe von PEG-NHS Ester wurde der pH der Reaktionsmischung durch Zugabe von 1M Natronlauge bei einem Wert von 8,0 bis 8,5 gehalten.

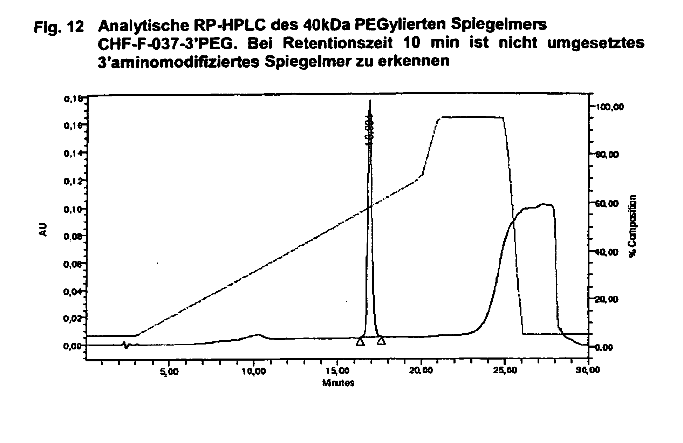

Das PEGylierte Spiegelmer wurde mittels RP-HPLC über Source15RPC Medium, Amersham Biosciences, und einem 0,1M Triethylammoniumacetat Gradienten in Wasser (Puffer B) und Acetonitril (Puffer C) gereinigt. Vor der Injektion auf die HPLC Säule wurde die Reaktionsmischung mit 8 M Harnstoff (4ml), Puffer A (4ml) und Puffer B (4ml) versetzt und für 15min auf 95°C erhitzt. Überschüssiges PEG wurde bei 5% Puffer C eluiert. PEGyliertes Spiegelmer eluierte bei 10-15% Puffer C. Produktfraktionen mit einer HPLC-Reinheit >95% wurden vereinigt und mit 2 M NaOAc (40ml) versetzt. Nach Entsalzung über Tangential-Fluß-Filtration mit einer 5K regenerierten Zellulose Membran, Millipore Corp. Bedford, MA (USA), wurde das gereinigte PEGylierte Spiegelmer in einer Ausbeute von 35-50% erhalten. Es wurden 390mg PEGyliertes Spiegelmer ausgehend von 240mg 3'-aminomodifiziertem Spiegelmer erhalten. Die IEX-HPLC Analytik des gereinigten 40kDa PEGylierten Spiegelmers CHF-F-037-3'PEG ist in Fig. 12 dargestellt.

### Beispiel 5: Biophysikalische Charakterisierung des Spiegelmers CHF-F-037 durch isothermale Kalorimetrie (ITC)

### Methodik

Kalorimetrische Messungen zur Bestimmung von Dissoziationskonstanten wurden mit einem VP-ITC Mikrokalorimeter (MicroCal, Northampton, MA) durchgeführt. Die zu messende Spiegelmer- bzw. Ligandenlösung wurde in Messpuffer (20 mM Tris-HCl, pH 7,4; 150 mM NaCl; 5 mM KCl; 1 mM MgCl₂; 1 mM CaCl₂) bei 37°C unter Vakuum entgast. Das Spiegelmer wurde in die Messzelle des Instrument zu 2 - 6 µM eingefüllt (Zellenvolumen: 1,43 ml), und der Ligand wurde zu 25 µM in die Injektionsspritze aufgezogen (Spritzenvolumen: 0,25 ml). Die Ligandenlösung wurde nach einer initialen 3 µl-Injektion in 7,5 µl-Aliquots über jeweils 6 s in die Messzelle injiziert. Die Messtemperatur betrug grundsätzlich 37°C, die Zeit zwischen zwei Injektionen war jeweils 5 min.

### Resultate

Die Ergebnisse der Messungen von nicht-PEGyliertem Spiegelmer CHF-F-037 und PEGyliertem Spiegelmer CHF-F-037-3'PEG gegen L-AVP sowie von CHF-F-037-3'PEG gegen Oxytocin sind in Tabelle 2 zusammengefasst.

**Tabelle 2**

| **Messung** | **Spiegelmer** | **Peptid** | **K_{d}** | **Aktivität** |
|---|---|---|---|---|
| 1 [110] | CHF-F-037 | L-AVP | 1,7 nM | 46,8% |
| 2 [120] | CHF-F-037-3'PEG | L-AVP | 1,3 nM | 59,1% |
| 3 [193] | CHF-F-037-3'PEG | L-Oxytocin | 16 nM | 69,8% |

Die Dissoziationskonstante liegt für das Spiegelmer sowohl in PEGylierter als auch in nicht-PEGylierter Form bei 1,5 nM. Damit bindet das Spiegelmer mit einer sehr hohen Affinität an AVP, was einen Einsatz bei der Medikation von Krankheitsbildern mit ursächlicher Beteiligung von AVP erlaubt. Das ähnliche Oxytocin wird mit etwa 10fach geringerer Affinität gebunden. Aber auch diese Affinität ist ausreichend, um mit dem Spiegelmer bei Kranheitsbildem mit Oxytocin-Beteiligung günstige Effekte zu erzielen. Die Dissoziationskonstante von 1,5 nM für das AVP-bindende Spiegelmer ist im Vergleich zur Dissoziationskonstante des nach dem Stand der Technik bereits bekannten DNA-Spiegelmers (Williams K P et al 1997 Proc. Natl. Acad. Sci USA 94: 11285-11290) um etwa 600fach besser.

### Beispiel 6: Biologische Charakterisierung des Spiegelmers CH-F-037 in Zellkultur

### A. Methodik

### Analyse der Hemmung der Bindung von (Arg⁸)-Vasopressin an den V₂-Rezeptor durch (Arg⁸)-Vasopressin-bindende Spiegelmere

Die Grundlage dieser Methode ist die Hemmung der AVP-V₂-Rezeptor-Interaktion und der darauffolgenden Bildung von cAMP. Zellen der Schweine-Nierenepithel-Zelllinie LLC-PK1 (ATCC-CL-101), die den renalen V₂-Rezeptor exprimieren, wurden in einer Zahl von 6 x 10⁴ pro Napf einer 96 Napf -Mikrotiterplatte ausgesät und über Nacht bei 37°C und 5% CO₂ in Medium 199 (Invitrogen, Karlsruhe), das zusätzlich 10% hitzeinaktiviertes fötales Kälberserum (FCS), 4 mM L-Alanyl-L-Glutamin (GLUTAMAX ), 50 Einheiten /ml Penicillin, 50 µg/ml Streptomycin enthält, kultiviert.

Die Spiegelmere wurden zusammen mit 1 nM L-AVP (Calbiochem) in Hank's balanced salt solution (HBSS) + 1 mg/ml BSA für 15 - 60 min bei RT oder 37°C in einer 0,2 ml "low profile 96-tube"-Platte inkubiert. Kurz vor Zugabe der Bindungsansätze zu den Zellen wurden 2 µl 50 mM 3-Isobutyl-1-Methylxanthin (IBMX-Lösung von Calbiochem) zugegeben. Die Zellen wurden 20 min vor Zugabe der AVP/Spiegelmer-Ansätze mit 1 mM IBMX vorbehandelt.

Zur Stimulation wurde das Medium von den Zellen abgesaugt und die vorinkubierten Bindungsansätze wurden zugegeben. Nach Inkubation für 30 min bei 37°C wurden die Zellüberstände abgesaugt und die Zellen mit 50 µl/Napf Lysis-Puffer für 30 min bei 37°C lysiert. Der Lysis-Puffer ist Bestandteil des "cAMP-Screen^{™} System"-kits (Applied Biosystems, Darmstadt), mit dem der cAMP-Gehalt der Extrakte bestimmt wird. Jeweils 10 µl der Extrakte wurden in den Test eingesetzt. Die Durchführung des Tests erfolgte wie vom Hersteller beschrieben:

In einer Assay-Platte (beschichtet mit Ziegen anti-Kaninchen IgG) wurden zu 50 µl des Lysis-Puffers 10 µl/ Napf des Lysats gegeben und mit 30 µl/ Napf des nach Herstellerangaben verdünnten cAMP-Alkalische Phosphatase-Konjugats vermischt. Danach wurden 60 µl/ Napf des im Kit mitgelieferten cAMP-Antikörpers hinzugefügt. Es folgte eine Inkubationszeit von 1 h unter Schütteln bei RT. Danach wurden die Lösungen aus den Näpfen entfernt und diese 6 mal mit dem mitgelieferten Wasch-Puffer gewaschen. Zur Detektion wurden 100 µl/Napf CSPD/Sapphire-II RTU Substrat zugegeben, 30 min bei RT inkubiert und die Lumineszenz in einem POLARstar Galaxy Multidetektions-Plattenlesegerät (BMG LABTECH, Offenburg) gemessen.

### Analyse der Hemmung der Bindung von (Arg⁸)-Vasopressin an den V₁-Rezeptor durch (Arg⁸)-Vasopressin-bindende Spiegelmere

Die Grundlage dieser Methode ist die Hemmung der AVP-V₁-Rezeptor-Interaktion und der darauffolgenden intrazellulären Freisetzung von Calcium. Zellen der Linie A7r5 (aus glatten Muskelzellen der Rattenaorta, ATCC-CRL-144), die den vaskulären V₁-Rezeptor exprimieren, wurden in einer Zahl von 4 x 10⁴ pro Napf einer schwarzen 96 Napf -Mikrotiterplatte mit klarem Boden (Greiner Bio-One, Frickenhausen) ausgesät und über Nacht bei 37°C und 5% CO₂ in DMEM, das zusätzlich 10 % fötales Kälberserum, 4 mM L-Alanyl-L-Glutamin (GLUTAMAX), 50 Einheiten /ml Penicillin und 50 µg/ml Streptomycin enthielt, kultiviert.

Die Spiegelmere wurden zusammen mit 5 nM L-AVP (Calbiochem, Schwalbach) in "Hanks balanced salt solution" (HBSS) in einer 0,2 ml "low profile 96-tube"-Platte für 15 - 60 min bei RT oder 37°C in einer 0,2 ml "low profile 96-tube"-Platte inkubiert. HBSS war mit 1 mg/ml BSA, 5 mM Probenecid und 20 mM HEPES supplementiert (HBSS+).

Vor der Beladung mit dem Calcium-Indikatorfarbstoff Fluo-4 wurden die Zellen 1 x mit je 200 µl HBSS+ gewaschen. Dann wurden 50 µl der Indikatorfarbstofflösung (10 µM Fluo-4 (Molecular Probes, Eugene, OR), 0.08% Pluronic 127 (Molecular Probes) in HBSS+) zugegeben und für 60 min bei 37°C inkubiert. Danach wurden die Zellen 3 x mit je 180 µl HBSS+ gewaschen. Pro Napf wurden anschließend 90 µl HBSS+ zugegeben.

Die Messung der Fluoreszenzsignale erfolgte bei einer Anregungungswellenlänge von 485 nm und einer Emissionswellenlänge von 520 nm in einem POLARstar Galaxy Multidetektions-Plattenlesegerät (BMG LABTECH).

Für die parallele Messung mehrerer Proben wurden jeweils die Näpfe einer (senkrechten) Reihe einer 96 well Platte gemeinsam vermessen. Dazu wurden zunächst im Abstand von 4 s drei Messwerte zur Feststellung der Basislinie registriert. Dann wurde die Messung unterbrochen, die Platte aus dem Lesegerät ausgefahren und mit einer Mehrkanalpipette 10 µl der Stimulationslösung aus der "low profile 96-tube"-Platte, in der die Vorinkubation durchgeführt wurde, zu den Näpfen der zu messenden Reihe zugegeben. Dann wurde die Platte wieder in das Gerät eingefahren und die Messung fortgesetzt (insgesamt 20 Messungen mit je 4 s Abstand).

Aus den erhaltenen Messkurven wurde für jeden einzelnen Napf die Differenz zwischen maximalem Fluoreszenzsignal und Fluoreszenzsignal vor der Stimulation bestimmt und gegen die Konzentration an Spiegelmer aufgetragen.

### B. Ergebnisse

### Inhibition der AVP-V₂-Rezeptor-Interaktion

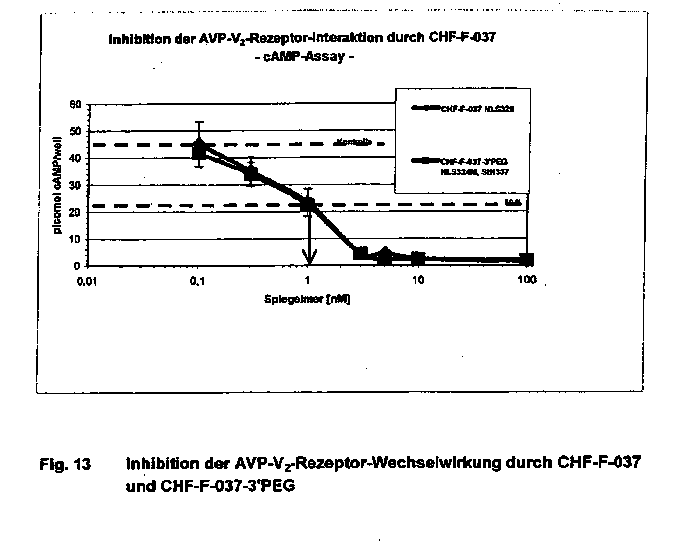

Die Inhibition der Interaktion von AVP mit dem V₂-Rezeptor auf LLC-PK1-Zellen durch das PEGylierte und das nicht-PEGylierte Spiegelmer CHF-F-037 ist in Fig. 13 gezeigt. Die gebildete Menge an cAMP ist gegen die Konzentration an Spiegelmer aufgetragen. Die graphische Auswertung ergibt als Konzentration an Spiegelmer, bei der nur noch 50% der in der Kontrolle vorhandenen cAMP-Menge gebildet wird, in beiden Fällen einen IC50 von ca. 1 nM. Damit ergibt sich im Vergleich zur Wirksamkeit des nach dem Stand der Technik bereits bekannten AVP-bindenden DNA Spiegelmers (Williams K P et al 1997 Proc. Natl. Acad. Sci USA 94: 11285-11290), für das im gleichen Test ein IC50 von ca. 3 µM bestimmt worden ist, eine Verbesserung um etwa den Faktor 3000.

### Inhibition der AVP-V₁-Rezeptor-Interaktion

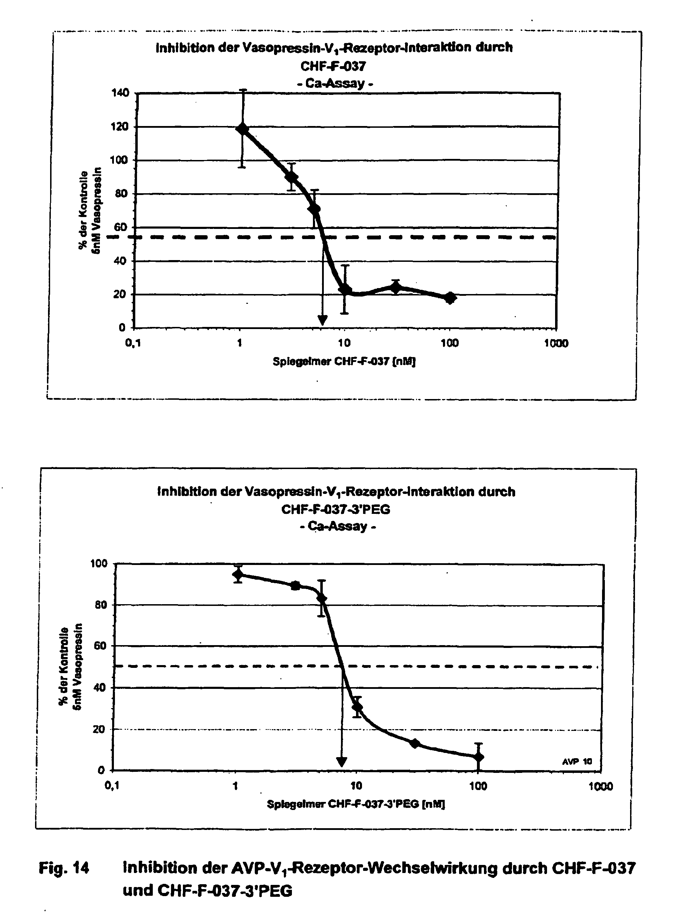

Die Inhibition der Interaktion von AVP mit dem V₁-Rezeptor auf A7r5-Zellen durch das PEGylierte und das nicht-PEGylierte Spiegelmer CHF-F-037 ist in Fig. 14 gezeigt. A7r5-Zellen wurden mit 5 nM AVP oder AVP, das zusammen mit verschiedenen Konzentrationen an Spiegelmer bei 37°C vorinkubiert worden war, stimuliert und die dadurch induzierte, intrazelluläre Calciumfreisetzung gemessen. Die Fig. zeigt die Abnahme des Prozentsatzes des Signals in Abhängigkeit steigender Konzentrationen von Spiegelmer. Zu 100% wurde das Signal gesetzt, das in Abwesenheit von Spiegelmer gemessen wurde (% der Kontrolle). Das nicht-PEGylierte Spiegelmer hemmt die AVP-induzierte, intrazelluläre Calciumfreisetzung mit einer halbmaximal wirksamen Konzentration (IC50) von ca. 6 nM. Das PEGylierte Spiegelmer hemmt mit einem IC50-Wert von ca. 7 nM.

### Beispiel 7: Studie zur Diurese in Ratten nach Verabreichung des Spiegelmers CHF-F-037

### A. Versuchsaufbau

Um den Effekt des Spiegelmers CHF-F-037 und seiner mit einem 40 kDa PEG-Rest versehenen Variante CHF-F-037-3'PEG *in vivo* zu testen, wurde eine Diurese-Studie mit männlichen Sprage Dawley-Ratten (Elevage Janvier, Le Genest St. Isle, Frankreich) von 239 - 290 g Körpergewicht bei der aurigon GmbH (Tutzing) durchgeführt.

Im Versuchsaufbau wurden sieben verschiedene Gruppen zu je 5 Tieren gebildet, die verschiedene Konzentrationen von PEGyliertem und nicht-PEGyliertem Spiegelmer sowie unwirksames Kontroll-Spiegelmer und reine Pufferlösung (Vehikel) als Kontrollen intravenös bzw. intraperitoneal erhalten sollten, wie in Tabelle 3 dargestellt.

**Tabelle 3**

| **Gruppe** | **Gruppengröße** | **Substanz** | **Dosis [mg/kg]** | **Dosis [nmol/kg]** | **Volumen [ml/kg]** | **Administration** |
|---|---|---|---|---|---|---|
| A | 5 | PBS (pH 7.4) | -- | -- | 2 | i.v. |
| B | 5 | Kontroll-SPM-5'PEG | 105,9 | 2000 | 2 | i.v. |
| C | 5 | CHF-F-037-3'PEG | 4,2 | 80 | 2 | i.v. |
| D | 5 | CHF-F-037-3'PEG | 21,2 | 400 | 2 | i.v. |
| E | 5 | CHF-F-037-3'PEG | 106 | 2000 | 2 | i.v. |
| a | 5 | Kontroll-SPM | 25,5 | 2000 | 2 | i.p. |
| b | 5 | CHF-F-037 | 25,9 | 2000 | 2 | i.p. |

Im Vorfeld des eigentlichen Versuchs wurden die insgesamt 35 Tiere am Tag -5 (vor Studienbeginn) an die Laborbedingungen akklimatisiert und am Tag -2 einzeln in metabolische Käfige bei 22 ± 3°C und 30-70% Luftfeuchtigkeit gesetzt. Die Tiere hatten zu jeder Zeit ad libitum Zugang zu sterilem Wasser und Trockenfutter (engl. *R*/*M-standard diet, autoclaved*; Ssniff Spezialdiäten GmbH, Soest). Am Tag -1 wurde von einem Tier jeder Gruppe der Wasserkonsum für die Zeitintervalle 0-6 h und 6-24 h, das Urinvolumen für die Zeitintervalle 0-2 h, 2-4 h, 4-6 h, und 6-24 h gemessen.

Am Tag 0 wurden die Substanzen den Tieren der Gruppen A bis E intravenös (i.v.) in die Schwanzvene als Bolus in einem Volumen von 2 ml/kg Körpergewicht appliziert. Den Tieren der Gruppen a und b wurden die Substanzen intraperitoneal (i.p.) in einem Volumen von 2 ml/kg Körpergewicht injiziert. Alle Tiere wurden direkt vor der Injektion gewogen und das entsprechende Injektionsvolumen bestimmt. Nach der Administration der Testsubstanzen (Tag 0) wurde von allen Tieren der Wasserkonsum für die Zeitintervalle 0-6 h und 6-24 h und das Urinvolumen für die Zeitintervalle 0-2 h, 2-4 h, 4-6 h, und 6-24 h gemessen. Der gesammelte Urin wurde bei 2-8°C gelagert und seine Osmolalität bzw. der Natriumgehalt wurde ebenfalls für die Intervalle 0-2 h, 2-4 h, 4-6 h, und 6-24 h bestimmt. Das experimentelle Design wurde von den lokalen Behörden überprüft und genehmigt; Registriemummer 209.1/211-2531.2-14/02.

### B. Ergebnisse

### Diurese und Wasserverbrauch

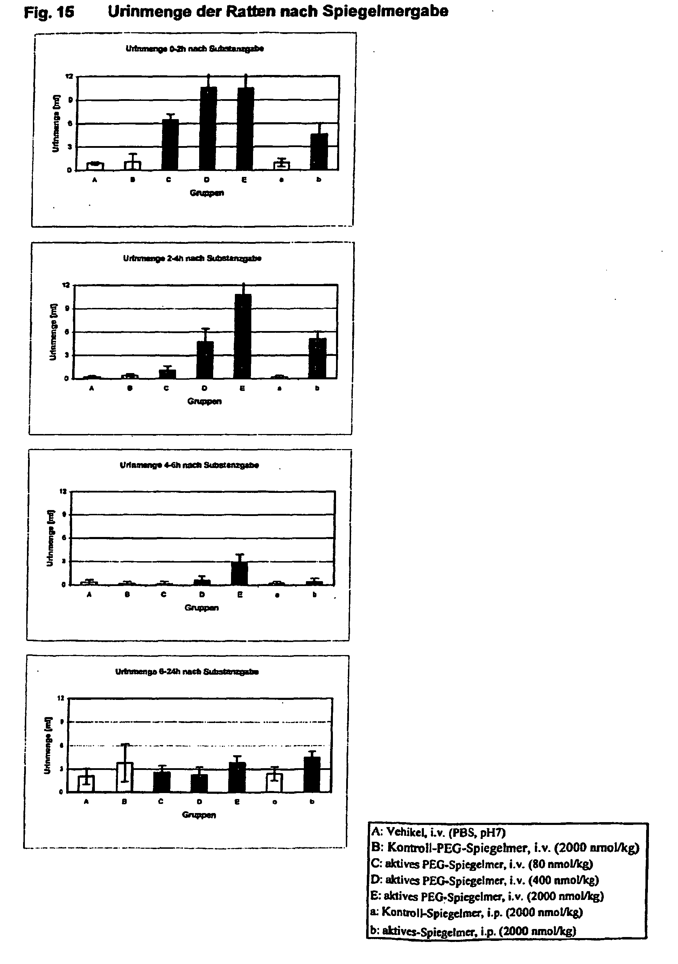

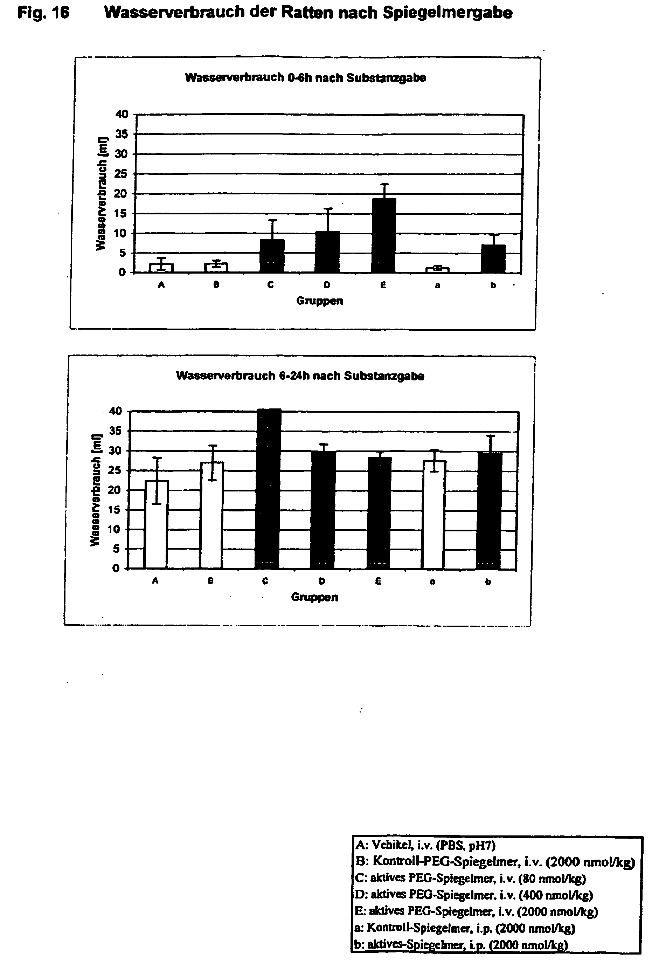

Intravenös administriertes CHF-F-037-3'PEG zeigte eine dosisabhängige Steigerung des Urinvolumens für die Gruppen C, D, und E; im Vergleich zu den Gruppen A (nur Vehikel) und B (inaktives Spiegelmer zu 2000 nmol/kg), wie in Fig. 15 dargestellt. Der Effekt setzte sofort nach der Gabe im Intervall 0-2 h ein und hielt bei den höher dosierten Gruppen (400 und 2000 nmol/kg) auch in den folgenden Intervallen (2-4 h und 4-6 h) an. Intraperitoneale Gabe von nicht-PEGyliertem Spiegelmer CHF-F-037 (Gruppe b, 2000 nmol/kg) sorgte während der ersten beiden Intervalle ebenfalls für ein gesteigertes Urinvolumen. Der Wasserverbrauch der Tiere im Intervall 0-6 h korrelierte weitgehend mit dem diuretischen Effekt; d.h. die Ratten tranken in diesem Intervall je mehr Wasser, desto größer das gemessene Urinvolumen war, wie in Fig. 16 dargestellt. Bezüglich der Parameter Urinvolumen und Wasserverbrauch waren in dem folgenden Zeitintervall 6-24 h keine signifikanten Unterschiede mehr zwischen den einzelnen Gruppen auszumachen

### Osmolalität und Na-Gehalt des Urins

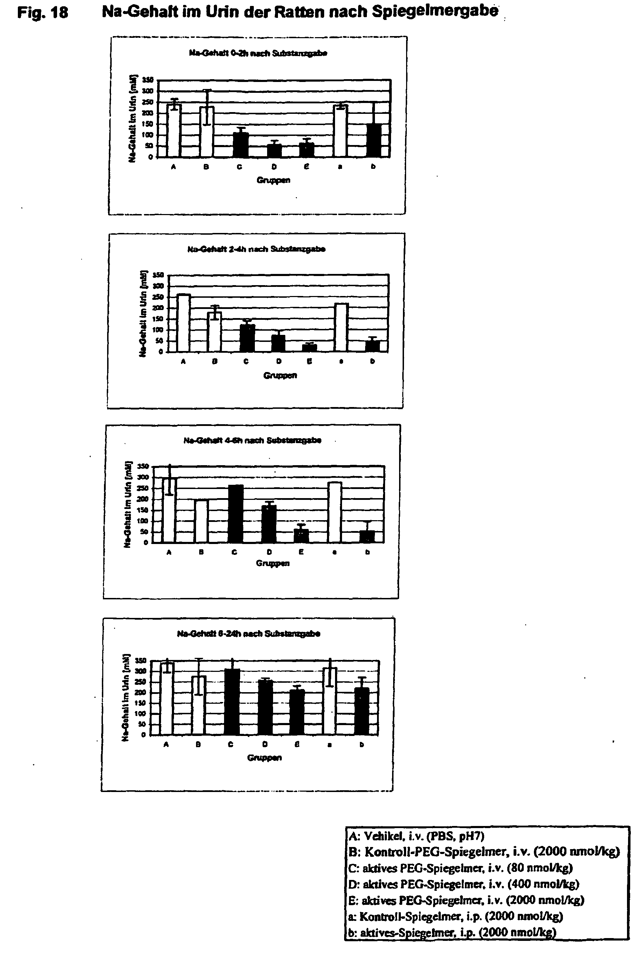

Die Osmolalität sowie der Na-Gehalt des Urins ist bei den Tieren, die aktives PEGyliertes Spiegelmer i.v. bzw. aktives nicht-PEGyliertes Spiegelmer i.p. erhalten haben, dosisabhängig gegenüber den Tieren der Kontrollgruppen deutlich erniedrigt, wie in Figs. 18 und 19 dargestellt. Hier ist selbst noch im Intervall von 6-24 h bei der höchsten Dosierung bei PEGyliertem Spiegelmer nach i.v. Applikation sowie bei nicht-PEGyliertem Spiegelmer nach i.p. Gabe ein Unterschied zu den Kontrolltieren zu sehen. Die Abnahme der Osmolalität und des Na-Gehalts ist ein Beweis für die spezifische Wirkung der Spiegelmere. Die Inhibition der AVP-V₂-Rezeptor-Interaktion führt zur Abnahme der Wasserresorption in den Sammelrohren der Nieren. Als Folge davon wird mehr Wasser ausgeschieden. Da jedoch nicht mehr Elektrolyte ausgeschieden werden, ist der Urin nach Behandlung mit dem aktiven Spiegelmer dosisabhängig deutlich verdünnter. Die AVP-bindenden Spiegelmere wirken also, genauso wie die V₂-Rezeptorantagonisten, nicht diuretisch sondem aquaretisch.

## Patentansprüche

1. Vasopressin bindende L-Nukleinsäure, **dadurch gekennzeichnet, dass** die Nukleinsäure einen Abschnitt Box1, einen Abschnitt Box2, einen Abschnitt Helix1 und einen Abschnitt Helix2 umfasst,
wobei Box1 die Sequenz GUGGW aufweist und W = A oder U ist und
wobei Box2 die Sequenz GGGGUAGGGMUUGGAHGGGHA aufweist,
wobei
M jeweils und unabhängig A oder C ist,
H jeweils und unabhängig A, C oder U ist, und
wobei der Abschnitt Helix1 und der Abschnitt Helix2 zueinander komplementär sind und der Abschnitt Helix1 und der Abschnitt Helix2 jeweils 5 bis 9 Nukleotide umfasst.

2. Vasopressin bindende Nukleinsäure nach Anspruch 1, **dadurch gekennzeichnet, dass** W = U ist.

3. Vasopressin bindende Nukleinsäure nach Anspruch 1 oder 2, **dadurch gekennzeichnet, dass** U oder C, bevorzugterweise U an den Positionen von H vorhanden ist.

4. Vasopressin bindende Nukleinsäure nach einem der Ansprüche 1 bis 3, **dadurch gekennzeichnet, dass** der Abschnitt Helix1 und der Abschnitt Helix2 jeweils 6 bis 7 Nukleotide und bevorzugtererweise 7 Nukleotide umfasst und die Abschnitte Helix1 und Helix2 bevorzugterweise eine doppelsträngige Helix ausbilden.

5. Vasopressin bindende Nukleinsäure nach Anspruch 4, **dadurch gekennzeichnet, dass** die doppelsträngige Helix terminal ausgebildet ist.

6. Vasopressin bindende Nukleinsäure nach einem der Ansprüche 1 bis 5, **dadurch gekennzeichnet, dass** die Nukleinsäuren einen W-Abschnitt umfasst, wobei der W-Abschnitt 0 bis 10, bevorzugterweise 6 bis 9 Nukleotide, oder alternativ 0 bis 7 Nukleotide umfasst.

7. Vasopressin bindende Nukleinsäure nach Anspruch 6, **dadurch gekennzeichnet, dass** der W-Abschnitt entweder (a) nur aus einem oder mehreren von A und/oder U besteht, oder (b) aus einem oder mehreren von A und/oder U und einem G besteht.

8. Vasopressin bindende Nukleinsäure nach Anspruch 6 oder 7, **dadurch gekennzeichnet, dass** der W-Abschnitt eine PEG-Gruppe umfasst.

9. Vasopressin bindende Nukleinsäure nach Anspruch 8, **dadurch gekennzeichnet, dass** die PEG-Gruppe am 5'-Ende des W-Abschnittes oder am 3'-Ende des W-Abschnittes oder zwischen zwei Nukleotiden des W-Abschnittes angeordnet ist.

10. Vasopressin bindende Nukleinsäuren nach einem der Ansprüche 1 bis 9, insbesondere nach einem der Ansprüche 1 bis 6, **dadurch gekennzeichnet, dass** die Nukleinsäure eine PEG-Gruppe enthält.

11. Vasopressin bindende Nukleinsäurenach einem der Ansprüche 1 bis 10, gemäß Formel **(I)** 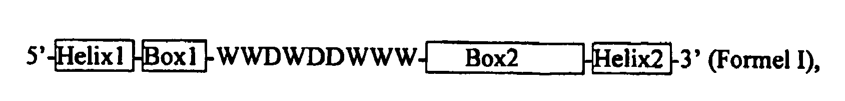 wobei
Helix 17 Nukleotide umfasst,
Helix2 7 Nukleotide umfasst,
Helix1 und Helix2 zueinander komplementär sind und eine terminale doppelsträngige Helix bilden,
der W-Abschnitt WWDWDDWWW umfasst, wobei
D einzeln und unabhängig A, G oder U sein kann, und wobei bevorzugterweise
der W-Abschnitt entweder (a) nur aus einem oder mehreren von A und/oder U besteht, oder (b) aus einem oder mehreren von A und/oder U und einem G besteht.

12. Vasopressin bindende Nukleinsäuren nach einem der Ansprüche 1 bis 10, gemäß Formel (II) 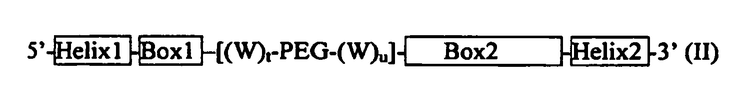 wobei -PEG- für eine PEG-Gruppe steht,
t und u einzeln und unabhängig voneinander 0, 1,2,3,4 oder 5 ist, wobei t + u 0, 1, 2, 3, 4 oder 5 ist; und
wobei
Helix1, Helix2, Box und Box2 wie in Anspruch 11 definiert sind; und
W = A oder U ist.

13. Vasopressin bindende Nukleinsäure nach einem der Ansprüche 1 bis 10, gemäß Formel (III) 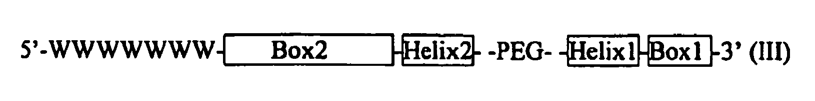 wobei -PEG- für eine PEG-Gruppe steht, und
wobei
Helix1 und Helix2 jeweils 6 oder 7 Nukleotide, bevorzugterweise 6 Nukleotide, umfasst, Helix1 und Helix2 zueinander komplementär sind und eine doppelsträngige Helix bilden,
Box1 und Box2 wie in Anspruch 1 definiert sind, wobei der W-Abschnitt WWWWWWW aus 0 bis 7 Ws besteht, wobei W = A oder U ist, bevorzugterweise kein W in Formel (III) vorhanden ist und
die PEG-Gruppe in 5-3'-Richtung zwischen Helix2 und Helix1 angeordnet ist.

14. Vasopressin bindende Nukleinsäure nach einem der Ansprüche 1 bis 13, insbesondere 10 bis 12, **dadurch gekennzeichnet, dass** die PEG-Gruppe ein Molekulargewicht von etwa 172 - 688 Da, bevorzugterweise etwa 344 aufweist.

15. Vasopressin bindende Nukleinsäure, nach einem der Ansprüche 1 bis 14, mit einer Nukleinsäuresequenz, wobei die Sequenz ausgewählt ist aus der Gruppe von Sequenzen, die SEQ. ID. NO. 2 bis SEQ. ID. NO. 50 umfasst.

16. Vasopressin bindende Nukleinsäure nach einem der vorangehenden Ansprüche, **dadurch gekennzeichnet, dass** das Vasopressin humanes Vasopressin ist.

17. Vasopressin bindende Nukleinsäure nach einem der Ansprüche 1 bis 16, **dadurch gekennzeichnet, dass** das Vasopressin eine Aminosäuresequenz gemäß SEQ. ID. NO. 1 aufweist.

18. Vasopressin bindende Nukleinsäure nach einem der Ansprüche 1 bis 17, **dadurch gekennzeichnet, dass** die Nukleinsäure eine Modifikation aufweist.

19. Vasopressin bindende Nukleinsäure nach Anspruch 18, **dadurch gekennzeichnet, dass** die Modifikation ausgewählt ist aus der Gruppe, die HESylierung und PEGylierung umfasst.

20. Vasopressin bindende Nukleinsäure nach Anspruch 19, **dadurch gekennzeichnet, dass** die PEGylierung durch ein geradkettiges oder verzweigtkettiges PEG erfolgt ist, wobei das Molekulargewicht des PEG etwa 20 bis 120 kDa, bevorzugterweise etwa 30 bis 80 kDa und bevorzugtererweise etwa 40 kDa ist.

21. Vasopressin bindende Nukleinsäure nach Anspruch 19, **dadurch gekennzeichnet, dass** die HESylierung durch ein HES erfolgt ist, wobei das Molekulargewicht der HES etwa 10 bis 130 kDa, bevorzugterweise etwa 30 bis 80 kDa und bevorzugtererweise etwa 50 kDa ist.

22. Vasopressin bindende Nukleinsäure nach einem der Ansprüche 1 bis 21, **dadurch gekennzeichnet, dass** die Nukleinsäure vollständig aus L-Nukleotiden besteht.

23. Pharmazeutische Zusammensetzung umfassend eine Nukleinsäure nach einem der Ansprüche 1 bis 22 und optional einem weiteren Bestandteil, wobei der weitere Bestandteil aus der Gruppe ausgewählt ist, die pharmazeutisch akzeptable Trägermittel umfasst.

24. Verwendung einer Nukleinsäure nach einem der Ansprüche 1 bis 22 zur Herstellung eines Medikamentes.

25. Verwendung einer Nukleinsäure nach einem der Ansprüche 1 bis 22 zur Herstellung eines diagnostischen Mittels.

26. Verwendung nach Anspruch 24, wobei das Medikament für die Behandlung und/oder Prävention von kongestiver Herzinsuffizienz ist, bevorzugterweise für die kurzzeitige Therapie, bevorzugtererweise zur Behandlung und/oder Prävention akuter dekompensierter kongestiver Herzinsuffizienz.

27. Verwendung nach Anspruch 24, wobei das Medikament für die Behandlung und/oder Prävention einer Erkrankung ist, wobei die Erkrankung eine Folgeerkrankung einer Krankheit ist, die ausgewählt ist aus der Gruppe, die Bluthochdruck, koronare Herzerkrankung, Herzinfarkt und Angina pectoris umfasst.

28. Verwendung nach einem der Ansprüche 24 bis 27, wobei die Patienten ältere Menschen oder Patienten mit einem vorhergegangenen Herzinfarkt sind.

29. Verwendung nach Anspruch 24, wobei das Medikament ein Medikament zur Prävention und/oder Behandlung von Bluthochdruck ist.

30. Verwendung nach Anspruch 24, wobei das Medikament ein Medikament zur Behandlung und/oder Prävention von Hypertrophie des Herzmuskels ist, bevorzugterweise durch (Arginin⁸)-Vasopressin Hyptertrophierung des Herzmuskels vermittelte.

31. Verwendung nach Anspruch 24, wobei das Medikament ein Medikament zur Behandlung und/oder Prävention von Ödemen ist.

32. Verwendung nach Anspruch 24, wobei das Medikament ein Medikament zur Verringerung des Gewichts bei Patienten mit erhöhter Wasserlast ist.

33. Verwendung nach Anspruch 24, wobei das Medikament ein Medikament zur Behandlung von Personen mit Hyponatriämie oder zur Prävention von Hyponatriämie ist.

34. Verwendung nach Anspruch 24, wobei das Medikament ein Medikament zur Behandlung und/oder Prävention des Syndroms der inadäquaten ADH-Sekretion ist.

35. Verwendung nach Anspruch 34, wobei das Syndroms der inadäquaten ADH-Sekretion einhergeht mit mindestens einem weiteren Symptom, wobei das weitere Symptom ausgewählt ist aus der Gruppe, die Antidiurese, Hyponatriämie und Hypoosmolarität umfasst.

36. Verwendung nach Anspruch 34 oder 35, wobei das Syndrom der inadäquaten ADH-Sekretion auf mindestens einer Ursache beruht, wobei die Ursache ausgewählt ist aus der Gruppe, die ektope ADH-Sekretion durch Tumore, medikamentöse Induktion von ADH-Sekretion, Erkrankungen der Lunge und Veränderung der zentralen Osmorezeptoren nach Schädel-Hirn-Trauma oder nach Meningitis umfasst.

37. Verwendung nach Anspruch 24, wobei das Medikament ein Medikament für die Prävention und/oder Behandlung der mit Leberzirrhose einhergehenden Hyponatriämie ist.

38. Verwendung nach Anspruch 24, wobei das Medikament ein Medikament zur Behandlung und/oder Prävention von Hirnödem, insbesondere nach Schädel-Hirn-Trauma oder nach Schlaganfall ist.

39. Verwendung nach Anspruch 24, **dadurch gekennzeichnet, dass** das Medikament für die Behandlung und/oder Prävention von Schädel-Hirn-Trauma und/oder Schlaganfall ist.

40. Verwendung nach Anspruch 24, wobei das Medikament ein Medikament zur Behandlung und/oder Prävention eines Tumors ist.

41. Verwendung nach Anspruch 40, wobei zumindest einige der den Tumor ausbildenden Zellen mindestens einen Rezeptor exprimieren, wobei der Rezeptor ausgewählt ist aus der Gruppe, die V₁-Rezeptoren, V₂-Rezeptoren und V₃-Rezeptoren umfasst.

42. Verwendung nach Anspruch 40 oder 41, wobei der Tumor ausgewählt ist aus der Gruppe, die ACTH sekretierende Tumoren, kleinzelliges Bronchialkarzinom und Mammakarzinom umfasst.

43. Verwendung nach Anspruch 24, wobei das Medikament ein Medikament zur Verhinderung von Frühgeburten ist.

44. Verwendung nach Anspruch 24, wobei das Medikament zur Prävention und/oder Behandlung von primärer Dysmenorrhö ist.

45. Komplex umfassend Vasopressin und eine Nukleinsäure nach einem der Ansprüche 1 bis 22.

46. Verfahren zum Screenen von Vasopressin-Antagonisten oder von Vasopressin-Agonisten umfassend die folgenden Schritte:
- Bereitstellen eines Kandidaten-Vasopressin-Antagonisten und/oder eines Kandidaten-Vasopressin-Agonisten,
- Bereitstellen einer Nukleinsäuren nach einem der Ansprüche 1 bis 22,
- Bereitstellen eines Testsystems, welches eine Signaländerung in Gegenwart eines Vasopressin-Antagonisten und/oder eines Vasopressin-Agonisten ergibt, und
- Bestimmen, ob der Kandidaten-Vasopressin-Antagonist ein Vasopressin-Antagonist ist, und/oder ob der Kandidaten-Vasopressein-Agonist ein Vasopressin-Agonist ist.

47. Verfahren nach Anspruch 46, wobei das Vasopressin (Arginin⁸)-Vasopressin ist.

48. Verfahren zum Screenen von Vasopressin-Agonisten und/oder Vasopressin-Antagonisten umfassend die folgenden Schritte:
- Bereitstellen von Vasopressin an Phase, bevorzugterweise einer festen Phase
- Bereitstellen einer Nukleinsäure nach einem der Ansprüche 1 bis 22, bevorzugterweise einer markierten Nukleinsäure nach einem der Ansprüche 1 bis 22,
- Zugabe eines Kandidaten-Vasopressin-Agonisten und/oder eines Kandidaten-Vasopressin-Antagonisten, und
- Bestimmen, ob der Kandidaten-Vasopressin-Agonist ein Vasopressin-Agonist ist und/oder ob der Kandidaten-Vasopressin-Antagonist ein Vasopressin-Antagonist ist.

49. Verfahren nach Anspruch 48, **dadurch gekennzeichnet, dass** das Bestimmen **dadurch** erfolgt, dass festgestellt wird, ob die Nukleinsäure durch den Kandidaten-Vasopressin-Agonisten verdrängt wird.

50. Kit für den Nachweis von Vasopressin umfassend eine Nukleinsäure nach einem der Ansprüche 1 bis 22.

51. Kit nach Anspruch 50, **dadurch gekennzeichnet, dass** Vasopressin Arginin-Vasopressin ist.

## Claims

1. A vasopressin binding L-nucleic acid, **characterized in that** the nucleic acid comprises a stretch box1, a stretch box2, a stretch helix1 and a stretch helix2,
wherein box1 has the sequence GUGGW and W = A or U and
wherein box2 has the sequence GGGGUAGGGMUUGGAHGGGHA,
wherein
M is each and independently A or C,
H is each and independently A, C or U, and
wherein the stretch helix1 and the stretch helix2 are complementary to each other and the stretch helix1 and the stretch helix2 each comprise 5 to 9 nucleotides.

2. The vasopressin binding nucleic acid according to claim 1, wherein W = U.

3. The vasopressin binding nucleic acid according to claim 1 or 2, **characterized in that** U or C, preferably U is present at the positions of H.

4. The vasopressin binding nucleic acid according to any one of claims 1 to 3, **characterized in that** the stretch helix1 and the stretch helix2 each comprises 6 to 7 nucleotides, preferably 7 nucleotides, and that the stretches helix1 and helix2 preferably form a double-stranded helix.

5. The vasopressin binding nucleic acid according to claim 4, **characterized in that** the double-stranded helix is formed at a terminus.

6. The vasopressin binding nucleic acid according to any one of claims 1 to 5, **characterized in that** the nucleic acids comprise a stretch W, whereby the stretch W comprises 0 to 10, preferably 6 to 9 nucleotides, or alternatively 0 to 7 nucleotides.

7. The vasopressin binding nucleic acid according to claim 6, **characterized in that** the stretch W consists either (a) of one or several of A and/or U, or (b) of one or several of A and/or U and one G.

8. The vasopressin binding nucleic acid according to claim 6 or 7, **characterized in that** the stretch W comprises a PEG group.

9. The vasopressin binding nucleic acid according to claim 8, **characterized in that** the PEG group is located at the 5'-end of the stretch W or at 3'-end of the stretch W or between two nucleotides of the stretch W.

10. The vasopressin binding nucleic acid according to any one of claims 1 to 9, preferably according to any one of claims 1 to 6, **characterized in that** the nucleic acid contains a PEG group.

11. The vasopressin binding nucleic acid according to any one of claims 1 to 10 in accordance with formula (I) 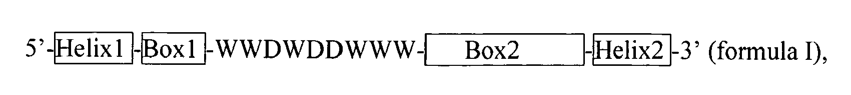 wherein
helix 1 comprises 7 nucleotides,
helix2 comprises 7 nucleotides,
helix1 and helix2 are complementary to each other and form a terminal double-stranded helix,
the stretch W comprises WWDWDDWWW, wherein
D may be individually and independently A, G or U and wherein preferably
the stretch W consists either (a) of only one or several of A and/or U, or (b) of one or several of A and/or U and one G.

12. The vasopressin binding nucleic acid according to any one of claims 1 to 10 in accordance with formula (II) 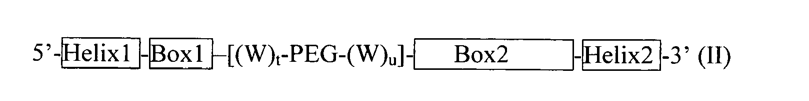 wherein -PEG- represents a PEG group,
t and u are individually and independently from each other 0, 1, 2, 3, 4 or 5, wherein t +uis0, 1,2, 3, 4 or 5; and
wherein
helix 1, helix2, box1 and box2 are as defined in claim 11; and
W=AorU.

13. The vasopressin binding nucleic acid according to any one of claims 1 to 10 in accordance with formula (III) 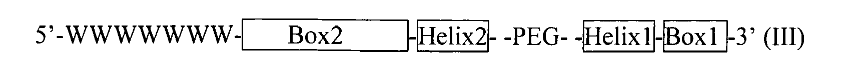 wherein -PEG- represents a PEG group, and
wherein
helix1 and helix2 each comprises 6 or 7 nucleotides, preferably 6 nucleotides, helix 1 and helix2 are complementary to each other and form a double-stranded helix,
box1 and box2 are as defined in claim 1, wherein the stretch WWWWWWW consists of 0 to 7 Ws, wherein W = A or U, preferably no W is contained in formula (III) and
the PEG group is located between helix2 and helix1 in 5'-3'-direction.

14. The vasopressin binding nucleic acid according to any one of claims 1 to 13, in particular 10 to 12, **characterized in that** the PEG group has a molecular weight of about 172-688 Da, preferably about 344.

15. The vasopressin binding nucleic acid according to any one of claims 1 to 14, having a nucleic acid sequence, whereby the sequence is selected from the group of sequences comprising SEQ. ID. NO. 2 to SEQ. ID. NO. 50.

16. The vasopressin binding nucleic acid according to any one of the preceding claims, **characterized in that** the vasopressin is human vasopressin.

17. The vasopressin binding nucleic acid according to any one of claims 1 to 16, **characterized in that** the vasopressin has an amino acid sequence according to SEQ. ID. NO. 1.

18. The vasopressin binding nucleic acid according to any one of claims 1 to 17, **characterized in that** the nucleic acid has a modification.

19. The vasopressin binding nucleic acid according to claim 18, **characterized in that** the modification is selected from the group comprising HESylation and PEGylation.

20. The vasopressin binding nucleic acid according to claim 19, **characterized in that** the PEGylation has occurred through a PEG having a linear or branched chain, whereby the molecular weight of the PEG is about 20 to 120 kDa, preferably 30 to 80 kDa and more preferably about 40 kDa.

21. The vasopressin binding nucleic acid according to claim 19, **characterized in that** the HESylation occurs by a HES, whereby the molecular weight of HES is about 10 to 130 kDa, preferably about 30 to 80 kDa and more preferably about 50 kDa.

22. The vasopressin binding nucleic acid according to any one of claim 1 to 21, **characterized in that** the nucleic acid completely consists of L-nucleotides.

23. A pharmaceutical composition comprising a nucleic acid according to any one of claims 1 to 22 and optionally a further constituent, whereby the further constituent is selected from the group comprising pharmaceutically acceptable carriers.

24. Use of a nucleic acid according to any one of claims 1 to 22 for the manufacture of a medicament.

25. Use of a nucleic acid according to any one of claims 1 to 22 for the manufacture of a diagnostic agent.

26. Use according to claim 24, wherein the medicament is for the treatment and/or prevention of congestive heart failure, preferably for the short-term therapy, more preferably for the treatment and/or prevention of acute decompensated congestive heart failure.

27. Use according to claim 24, wherein the medicament is for the treatment and/or prevention of a disease, whereby the disease is a secondary disease of a disease which is selected from the group comprising hypertension, coronary heart disease, heart infarction and angina pectoris.

28. Use according to any one of claims 24 to 27, wherein the patients are elderly people or patients with a previous heart infarction.

29. Use according to claim 24, wherein the medicament is a medicament for the prevention and/or treatment of hypertension.

30. Use according to claim 24, wherein the medicament is a medicament for the treatment and/or prevention of the hypertrophy of the heart muscle, preferably a hypertrophy of the heart muscle which is mediated by (arginine⁸)-vasopressin.

31. Use according to claim 24, wherein the medicament is a medicament for the treatment and/or prevention of edema.

32. Use according to claim 24, wherein the medicament is a medicament for reducing the weight of a patient with increased water load.

33. Use according to claim 24, wherein the medicament is a medicament for the treatment of people with hyponatraemia or for prevention of hyponatraemia.

34. Use according to claim 24, wherein the medicament is a medicament for the treatment and/or prevention of the syndrome of inadequate ADH secretion.

35. Use according to claim 34, wherein the syndrome of inadequate ADH secretion goes along with at least one further symptom, wherein the further symptom is selected from the group comprising antidiuresis, hyponatraemia and hypo-osmolarity.

36. Use according to claim 34 or 35, wherein the syndrome of inadequate ADH secretion is based on at least one cause, wherein said cause is selected from the group comprising ectopic ADH secretion by tumours, ADH secretion induced by medicaments, disorders of the lung and changes of the central osmoreceptors after craniocerebral injury or after meningitis.

37. Use according to claim 24, wherein the medicament is a medicament for the prevention and/or treatment of hyponatraemia going along with liver cirrhosis.

38. Use according to claim 24, wherein the medicament is a medicament for the treatment and/or prevention of cerebral oedema, more particularly after craniocerebral injury or after stroke.

39. Use according to claim 24, **characterized in that** the medicament is for the treatment and/or prevention of craniocerebral injury and/or stroke.

40. Use according to claim 24, wherein the medicament is a medicament for the treatment and/or prevention of a tumour.

41. Use according to claim 40, wherein at least some of the tumour forming cells express at least one receptor, wherein the receptor is selected from the group comprising V₁-receptors, V₂-receptors and V₃-receptors.

42. Use according to claim 40 or 41, wherein the tumour selected from the group comprising ACTH secreting tumours, small-cell bronchiolar carcinoma and mammary carcinoma.

43. Use according to claim 24, wherein the medicament is a medicament for the prevention of premature delivery.

44. Use according to claim 24, wherein the medicament is for the prevention and/or treatment of primary dysmenorrhoea.

45. A complex comprising vasopressin and a nucleic acid according to any one of claims 1 to 22.

46. A method for the screening of vasopressin antagonists or of vasopressin agonists comprising the following steps:
- providing a candidate vasopressin antagonist and/or a candidate vasopressin agonist
- providing a nucleic acid according to any one of claims 1 to 22,
- providing a test system which provides a change in signal in the presence of a vasopressin antagonist and/or a vasopressin agonist, and
- determining whether the candidate vasopressin antagonist is a vasopressin antagonist and/or whether the candidate vasopressin agonist is a vasopressin agonist.

47. The method according to claim 46, wherein the vasopressin is (arginine⁸)-vasopressin.

48. A method for the screening of vasopressin agonists and/or vasopressin antagonists comprising the following steps:
- providing vasopressin at a phase, preferably a solid phase,
- providing a nucleic acid according to any one of claims 1 to 22, preferably a labelled nucleic acid according to any one of claims 1 to 22,
- adding a candidate vasopressin agonist and/or a candidate vasopressin antagonist, and
- determining whether the candidate vasopressin agonist is a vasopressin agonist and/or whether the candidate vasopressin antagonist is a vasopressin antagonist.

49. The method according to claim 48, **characterized in that** the determining is done by assessing whether the nucleic acid is replaced by the candidate vasopressin agonist.

50. A kit for the detection of vasopressin comprising a nucleic acid according to any one of claims 1 to 22.

51. A kit according to claim 50, **characterized in that** vasopressin is arginine-vasopressin.

## Revendications

1. Acide nucléique L se fixant à la vasopressine, **caractérisé en ce que** l'acide nucléique comprend un enchaînement boîte1, un enchaînement boîte2, un enchaînement hélice1 et un enchaînement hélice2,
dans lequel boîte1 a la séquence GUGGW et W = A ou U et
dans lequel boîte2 a la séquence GGGGUAGGGMUUGGAHGGGHA,
dans laquelle
M est à chaque fois et indépendamment A ou C,
H est à chaque fois et indépendamment A, C ou U et
dans lequel l'enchaînement hélice1 et l'enchaînement hélice2 sont complémentaires l'un à l'autre et l'enchaînement hélice1 et l'enchaînement hélice2 comprennent chacun 5 à 9 nucléotides.

2. Acide nucléique se fixant à la vasopressine selon la revendication 1, dans lequel W = U.

3. Acide nucléique se fixant à la vasopressine selon la revendication 1 ou 2, **caractérisé en ce que** U ou C, de préférence U est présent au niveau des positions de H.

4. Acide nucléique se fixant à la vasopressine selon l'une quelconque des revendications 1 à 3, **caractérisé en ce que** l'enchaînement hélice1 et l'enchaînement hélice2 comprennent chacun 6 à 7 nucléotides, de préférence 7 nucléotides, et **en ce que** les enchaînements hélice1 et hélice2 forment de préférence une double hélice.

5. Acide nucléique se fixant à la vasopressine selon la revendication 4, **caractérisé en ce que** la double hélice est formée à une extrémité.

6. Acide nucléique se fixant à la vasopressine selon l'une quelconque des revendications 1 à 5, **caractérisé en ce que** les acides nucléiques comprennent un enchaînement W, l'enchaînement W comprenant 0 à 10, de préférence 6 à 9 nucléotides, ou en variante 0 à 7 nucléotides.

7. Acide nucléique se fixant à la vasopressine selon la revendication 6, **caractérisé en ce que** l'enchaînement W est constitué soit (a) d'un ou plusieurs éléments parmi A et/ou U, soit (b) d'un ou plusieurs éléments parmi A et/ou U et d'un G.

8. Acide nucléique se fixant à la vasopressine selon la revendication 6 ou 7, **caractérisé en ce que** l'enchaînement W comprend un groupe PEG.

9. Acide nucléique se fixant à la vasopressine selon la revendication 8, **caractérisé en ce que** le groupe PEG est situé à l'extrémité 5' de l'enchaînement W ou à l'extrémité 3' de enchaînement W ou entre deux nucléotides de l'enchaînement W.

10. Acide nucléique se fixant à la vasopressine selon l'une quelconque des revendications 1 à 9, de préférence selon l'une quelconque des revendications 1 à 6, **caractérisé en ce que** l'acide nucléique contient un groupe PEG.

11. Acide nucléique se fixant à la vasopressine selon l'une quelconque des revendications 1 à 10 répondant à la formule (I) 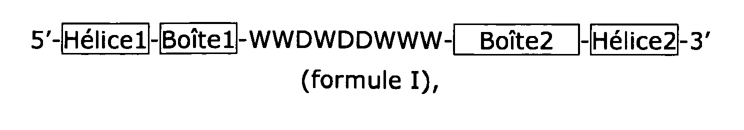 dans laquelle
hélice1 comprend 7 nucléotides,
hélice2 comprend 7 nucléotides,
hélice1 et hélice2 sont complémentaires l'un à l'autre et forment une double hélice terminale,
l'enchaînement W comprend WWDWDDWWW, dans lequel D peut être individuellement et indépendamment A, G ou U et dans lequel de préférence
l'enchaînement W est constitué soit (a) d'un seul ou de plusieurs éléments parmi A et/ou U, soit (b) d'un ou plusieurs éléments parmi A et/ou U et d'un G.

12. Acide nucléique se fixant à la vasopressine selon l'une quelconque des revendications 1 à 10 répondant à la formule (II) 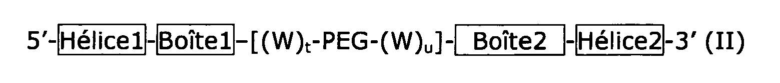 dans laquelle -PEG- représente un groupe PEG,
t et u valent individuellement et indépendamment l'un de l'autre 0, 1, 2, 3, 4 ou 5, t + u valant 0, 1, 2, 3, 4 ou 5 ; et dans laquelle
hélice1, hélice 2, boÎte1 et boîte2 sont tels que définis dans la revendication 11 ; et
W = A ou U.

13. Acide nucléique se fixant à la vasopressine selon l'une quelconque des revendications 1 à 10 répondant à la formule (III) 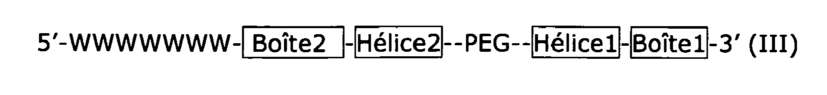 dans laquelle -PEG- représente un groupe PEG et dans laquelle
hélice1 et hélice2 comprennent chacun 6 ou 7 nucléotides, de préférence 6 nucléotides, hélice1 et hélice2 sont complémentaires l'un à l'autre et forment une double hélice, boÎte1 et boîte2 sont tels que définis dans la revendication 1, dans laquelle l'enchaînement WWWWWWW est constitué de 0 à 7 W, dans laquelle W = A ou U, de préférence aucun W n'est contenu dans la formule (III) etle groupe PEG est situé entre hélice2 et hélice1 dans le sens 5'-3'.

14. Acide nucléique se fixant à la vasopressine selon l'une quelconque des revendications 1 à 13, en particulier 10 à 12, **caractérisé en ce que** le groupe PEG a une masse moléculaire d'environ 172-688 Da, de préférence d'environ 344.

15. Acide nucléique se fixant à la vasopressine selon l'une quelconque des revendications 1 à 14, ayant une séquence d'acide nucléique, la séquence étant choisie dans le groupe de séquences comprenant SEQ. ID. NO. 2 à SEQ. ID. NO. 50.

16. Acide nucléique se fixant à la vasopressine selon l'une quelconque des revendications précédentes, **caractérisé en ce que** la vasopressine est la vasopressine humaine.

17. Acide nucléique se fixant à la vasopressine selon l'une quelconque des revendications 1 à 16, **caractérisé en ce que** la vasopressine a une séquence d'acides aminés selon SEQ. ID. NO. 1.

18. Acide nucléique se fixant à la vasopressine selon l'une quelconque des revendications 1 à 17, **caractérisé en ce que** l'acide nucléique a une modification.

19. Acide nucléique se fixant à la vasopressine selon la revendication 18, **caractérisé en ce que** la modification est choisie dans le groupe comprenant l'HESylation et la PEGylation.

20. Acide nucléique se fixant à la vasopressine selon la revendication 19, **caractérisé en ce que** la PEGylation a eu lieu par l'intermédiaire d'un PEG ayant une chaîne linéaire ou ramifiée, la masse moléculaire du PEG étant d'environ 20 à 120 kDa, de préférence de 30 à 80 kDa et de façon davantage préférable d'environ 40 kDa.

21. Acide nucléique se fixant à la vasopressine selon la revendication 19, **caractérisé en ce que** l'HESylation a lieu par un HES, la masse moléculaire du HES étant d'environ 10 à 130 kDa, de préférence d'environ 30 à 80 kDa et de façon davantage préférable d'environ 50 kDa.

22. Acide nucléique se fixant à la vasopressine selon l'une quelconque des revendications 1 à 21, **caractérisé en ce que** l'acide nucléique est constitué totalement de nucléotides L.

23. Composition pharmaceutique comprenant un acide nucléique selon l'une quelconque des revendications 1 à 22 et facultativement un autre constituant, l'autre constituant étant choisi dans le groupe comprenant les véhicules pharmaceutiquement acceptables.

24. Utilisation d'un acide nucléique selon l'une quelconque des revendications 1 à 22 pour la fabrication d'un médicament.

25. Utilisation d'un acide nucléique selon l'une quelconque des revendications 1 à 22 pour la fabrication d'un agent de diagnostic.

26. Utilisation selon la revendication 24, dans laquelle le médicament est pour le traitement et/ou la prévention d'une insuffisance cardiaque congestive, de préférence pour la thérapie à court terme, de façon davantage préférable pour le traitement et/ou la prévention d'une insuffisance cardiaque congestive aiguë décompensée.

27. Utilisation selon la revendication 24, dans laquelle le médicament est pour le traitement et/ou la prévention d'une maladie, la maladie étant une maladie secondaire d'une maladie qui est choisie dans le groupe comprenant l'hypertension, une maladie coronarienne, l'infarctus du myocarde et l'angine de poitrine.

28. Utilisation selon l'une quelconque des revendications 24 à 27, dans laquelle les patients sont des personnes âgées ou des patients présentant un infarctus du myocarde antérieur.

29. Utilisation selon la revendication 24, dans laquelle le médicament est un médicament pour la prévention et/ou le traitement de l'hypertension.

30. Utilisation selon la revendication 24, dans laquelle le médicament est un médicament pour le traitement et/ou la prévention de l'hypertrophie du muscle cardiaque, de préférence d'une hypertrophie du muscle cardiaque qui est à médiée par 1'(arginine⁸)-vasopressine.

31. Utilisation selon la revendication 24, dans laquelle le médicament est un médicament pour le traitement et/ou la prévention d'un oedème.

32. Utilisation selon la revendication 24, dans laquelle le médicament est un médicament pour la réduction du poids d'un patient avec une charge hydrique accrue.

33. Utilisation selon la revendication 24, dans laquelle le médicament est un médicament pour le traitement de personnes présentant une hyponatrémie ou pour la prévention de l'hyponatrémie.

34. Utilisation selon la revendication 24, dans laquelle le médicament est un médicament pour le traitement et/ou la prévention du syndrome de sécrétion inappropriée d'ADH.

35. Utilisation selon la revendication 34, dans laquelle le syndrome de sécrétion inappropriée d'ADH s'accompagne d'au moins un autre symptôme, l'autre symptôme étant choisi dans le groupe comprenant l'antidiurèse, l'hyponatrémie et l'hypo-osmolarité.

36. Utilisation selon la revendication 34 ou 35, dans laquelle le syndrome de sécrétion inappropriée d'ADH est fondé sur au moins une cause, ladite cause étant choisie dans le groupe comprenant la sécrétion ectopique d'ADH par des tumeurs, la sécrétion d'ADH induite par des médicaments, des troubles du poumon et des changements des osmorécepteurs centraux après une lésion crânio-cérébrale ou après une méningite.

37. Utilisation selon la revendication 24, dans laquelle le médicament est un médicament pour la prévention et/ou le traitement de l'hyponatrémie accompagnant une cirrhose du foie.

38. Utilisation selon la revendication 24, dans laquelle le médicament est un médicament pour le traitement et/ou la prévention d'un oedème cérébral, plus particulièrement après une lésion crânio-cérébrale ou après un accident vasculaire cérébral.

39. Utilisation selon la revendication 24, **caractérisée en ce que** le médicament est pour le traitement et/ou la prévention d'une lésion crânio-cérébrale et/ou d'un accident vasculaire cérébral.

40. Utilisation selon la revendication 24, dans laquelle le médicament est un médicament pour le traitement et/ou la prévention d'une tumeur.

41. Utilisation selon la revendication 40, dans laquelle au moins certaines des cellules formant une tumeur expriment au moins un récepteur, le récepteur étant choisi dans le groupe comprenant les récepteurs V₁, les récepteurs V₂ et les récepteurs V₃.

42. Utilisation selon la revendication 40 ou 41, dans laquelle la tumeur est choisie dans le groupe comprenant les tumeurs sécrétant de l'ACTH, le carcinome bronchiolaire à petites cellules et le carcinome mammaire.

43. Utilisation selon la revendication 24, dans laquelle le médicament est un médicament pour la prévention d'un accouchement prématuré.

44. Utilisation selon la revendication 24, dans laquelle le médicament est pour la prévention et/ou le traitement de la dysménorrhée primaire.

45. Complexe comprenant la vasopressine et un acide nucléique selon l'une quelconque des revendications 1 à 22.

46. Procédé pour la recherche par criblage d'antagonistes de la vasopressine ou d'agonistes de la vasopressine comprenant les étapes suivantes consistant à :
- se procurer un antagoniste de la vasopressine candidat et/ou un agoniste de la vasopressine candidat
- se procurer un acide nucléique selon l'une quelconque des revendications 1 à 22,
- se procurer un système de test qui fournit une variation de signal en présence d'un antagoniste de la vasopressine et/ou d'un agoniste de la vasopressine et
- déterminer si l'antagoniste de la vasopressine candidat est un antagoniste de la vasopressine et/ou si l'agoniste de la vasopressine candidat est un agoniste de la vasopressine.

47. Procédé selon la revendication 46, dans lequel la vasopressine est l'(arginine⁸)-vasopressine.

48. Procédé pour la recherche par criblage d'agonistes de la vasopressine et/ou d'antagonistes de la vasopressine comprenant les étapes suivantes consistant à :
- se procurer de la vasopressine sous forme d'une phase, de préférence d'une phase solide,
- se procurer un acide nucléique selon l'une quelconque des revendications 1 à 22, de préférence un acide nucléique marqué selon l'une quelconque des revendications 1 à 22,
- ajouter un agoniste de la vasopressine candidat et/ou un antagoniste de la vasopressine candidat et
- déterminer si l'agoniste de la vasopressine candidat est un agoniste de la vasopressine et/ou si l'antagoniste de la vasopressine candidat est un antagoniste de la vasopressine.

49. Procédé selon la revendication 48, **caractérisé en ce que** la détermination est effectuée en évaluant si l'acide nucléique est remplacé par l'agoniste de la vasopressine candidat.

50. Kit pour la détection de vasopressine comprenant un acide nucléique selon l'une quelconque des revendications 1 à 22.

51. Kit selon la revendication 50, **caractérisé en ce que** la vasopressine est l'arginine-vasopressine.


### SEQUENCE LISTING

<110> NOXXON Pharma AG
<120> Vasopressin bindende L-Nukleinsäuren
<130> N 10045 PCT
<160> 50
<170> Patent In version 3.1
<210> 1
   <211> 9
   <212> PRT
   <213> Homo sapiens
<220>
   <221> MISC_FEATURE
   <223> Vasopressin
<400> 1
   Cys Tyr Phe Gln Asn Cys Pro Arg Gly
   1 5
<210> 2
   <211> 48
   <212> RNA
   <213> Artificial
<220>
   <221> misc_feature
   <223> Binder
<400> 2
   agcgugcgug gaaauuauau gggguagggc uuggaagggu aguacgcu 48
<210> 3
   <211> 49
   <212> RNA
   <213> Artificial
<220>
   <221> misc_feature
   <223> Binder
<400> 3
   aguacgcgug guaaauugaa ugggguaggg cuuggacggg uaguguacu 49
<210> 4
   <211> 48
   <212> RNA
   <213> Artificial
<220>
   <221> misc_feature
   <223> Binder
<400> 4
   aggacgcgug guaauuauau gggguagggc uuggaugggu aguguccu 48
<210> 5
   <211> 49
   <212> RNA
   <213> Artificial
<220>
   <221> misc_feature
   <223> Binder
<400> 5
   aguaugcgug guaaauuaaa ugggguaggg cuuggacggg uaguguacu 49
<210> 6
   <211> 48
   <212> RNA
   <213> Artificial
<220>
   <221> misc_feature
   <223> Binder
<400> 6
   aguaugcgug guaaaugauu gggguagggc uuggaugggu aguguacu 48
<210> 7
   <211> 48
   <212> RNA
   <213> Artificial
<220>
   <221> misc_feature
   <223> Binder
<400> 7
   aguaugcgug guaaaugauu gggguagggc uugaauuggu aguguacu 48
<210> 8
   <211> 47
   <212> RNA
   <213> Artificial
<220>
   <221> misc_feature
   <223> Binder
<400> 8
   aguaugcgug guauauaaug ggguagggcu uggaugggua guguacu 47
<210> 9
   <211> 47
   <212> RNA
   <213> Artificial
<220>
   <221> misc_feature
   <223> Binder
<400> 9
   aguacgcgug guuuaaaaug ggguagggau uggaugggua guguacu 47
<210> 10
   <211> 48
   <212> RNA
   <213> Artificial
<220>
   <221> misc_feature
   <223> Binder
<400> 10
   agucugcgug guuuuuuaau gggguagggc uuggaugggu aguagacu 48
<210> 11
   <211> 48
   <212> RNA
   <213> Artificial
<220>
   <221> misc_feature
   <223> Binder
<400> 11
   agucugcgug guuuuuuuau gggguagggc uuggaugggu aguagacu 48
<210> 12
   <211> 49
   <212> RNA
   <213> Artificial
<220>
   <221> misc_feature
   <223> Binder
<400> 12
   agucugcgug guuuuuaaau ugggguaggg cuuggauggg uaguagacu 49
<210> 13
   <211> 47
   <212> RNA
   <213> Artificial
<220>
   <221> misc_feature
   <223> Binder
<400> 13
   aguaugcgug guuuuuaaug ggguagggcu uggaugggua guauacu 47
<210> 14
   <211> 46
   <212> RNA
   <213> Artificial
<220>
   <221> misc_feature
   <223> Binder
<400> 14
agugugcgug guuagaaugg gguagggcuu ggauggguag uacacu 46
<210> 15
   <211> 47
   <212> RNA
   <213> Artificial
<220>
   <221> misc_feature
   <223> Binder
<400> 15
   agugugcgug guuaauaaug ggguagggcu uggaugggua guacacu 47
<210> 16
   <211> 47
   <212> RNA
   <213> Artificial
<220>
   <221> misc_feature
   <223> Binder
<400> 16
   agugugggug guuaauaaug ggguagggcu uggaugggua ccacacu 47
<210> 17
   <211> 47
   <212> RNA
   <213> Artificial
<220>
   <221> misc_feature
   <223> Binder
<400> 17
aguaugggug guuauuaaug ggguagggau uggaugggua ccauacu 47
<210> 18
   <211> 46
   <212> RNA
   <213> Artificial
<220>
   <221> misc_feature
   <223> Binder
<400> 18
aguaaccgug guuuaaaugg gguagggauu ggaugggcag gauacu 46
<210> 19
   <211> 46
   <212> RNA
   <213> Artificial
<220>
   <221> misc_feature
   <223> Binder
<400> 19
aguagccgug guuuaaaugg gguagggauu ggaugggcag gauacu 46
<210> 20
   <211> 47
   <212> RNA
   <213> Artificial
<220>
   <221> misc_feature
   <223> Binder
<400> 20
agugaccgug guaaauaaug ggguagggau uggaugggca ggacacu 47
<210> 21
   <211> 47
   <212> RNA
   <213> Artificial
<220>
   <221> misc_feature
   <223> Binder
<400> 21
agugaccgug guaaaugaug ggguagggau uggaugggcu ggacacu 47
<210> 22
   <211> 47
   <212> RNA
   <213> Artificial
<220>
   <221> misc_feature
   <223> Binder
<400> 22
agugaccgug guaaauauug ggguagggau uggaugggca ggacacu 47
<210> 23
   <211> 47
   <212> RNA
   <213> Artificial
<220>
   <221> misc_feature
   <223> Binder
<400> 23
aguaugcgug guaaauagug ggguagggau uggaugggca guauacu 47
<210> 24
   <211> 47
   <212> RNA
   <213> Artificial
<220>
   <221> misc_feature
   <223> Binder
<400> 24
agugugcgug guuaauaaug ggguagggcu uggaugggua guacacu 47
<210> 25
   <211> 44
   <212> RNA
   <213> Artificial
<220>
   <221> misc_feature
   <223> Binder
<400> 25
agugugcgug gauaaugggg uagggcuugg auggguagua cacu 44
<210> 26
   <211> 44
   <212> RNA
   <213> Artificial
<220>
   <221> misc_feature
   <223> Binder
<400> 26
agugugcgug guuaaugggg uagggcuugg auggguagua cacu 44
<210> 27
   <211> 44
   <212> RNA
   <213> Artificial
<220>
   <221> misc_feature
   <223> Binder
<400> 27
agugugcgug guuaaugggg uagggcuugg auggguagua cacu 44
<210> 28
   <211> 44
   <212> RNA
   <213> Artificial
<220>
   <221> misc_feature
   <223> Binder
<400> 28
agugugcgug guuaaugggg uagggcuugg auggguagua cacu 44
<210> 29
   <211> 44
   <212> RNA
   <213> Artificial
<220>
   <221> misc_feature
   <223> Binder
<400> 29
agugugcgug guuaaugggg uagggcuugg auggguagua cacu 44
<210> 30
   <211> 44
   <212> RNA
   <213> Artificial
<220>
   <221> misc_feature
   <223> Binder
<400> 30
   agugugcgug guuaaugggg uagggcuugg auggguagua cacu 44
<210> 31
   <211> 41
   <212> RNA
   <213> Artificial
<220>
   <221> misc_feature
   <223> Binder
<400> 31
agugugcgug guugggguag ggcuuggaug gguaguacac u 41
<210> 32
   <211> 43
   <212> RNA
   <213> Artificial
<220>
   <221> misc_feature
   <223> Binder
<400> 32
   agugugcgug guaauggggu agggcuugga uggguaguac acu 43
<210> 33
   <211> 42
   <212> RNA
   <213> Artificial
<220>
   <221> misc_feature
   <223> Binder
<400> 33
   agugugcgug guauggggua gggcuuggau ggguaguaca cu 42
<210> 34
   <211> 41
   <212> RNA
   <213> Artificial
<220>
   <221> misc_feature
   <223> Binder
<400> 34
agugugcgug guugggguag ggcuuggaug gguaguacac u 41
<210> 35
   <211> 40
   <212> RNA
   <213> Artificial
<220>
   <221> misc_feature
   <223> Binder
<400> 35
agugugcgug gugggguagg gcuuggaugg guaguacacu 40
<210> 36
   <211> 44
   <212> RNA
   <213> Artificial
<220>
   <221> misc_feature
   <223> Binder
<400> 36
agugugcgug guuaaugggg uagggcuugg auggguagua cacu 44
<210> 37
   <211> 43
   <212> RNA
   <213> Artificial
<220>
   <221> misc_feature
   <223> Binder
<400> 37
agugugcgug guaauggggu agggcuugga uggguaguac acu 43
<210> 38
   <211> 47
   <212> RNA
   <213> Artificial
<220>
   <221> misc_feature
   <223> Binder
<400> 38
aauaaugggg uagggcuugg auggguagua cacuagugug cgugguu 47
<210> 39
   <211> 47
   <212> RNA
   <213> Artificial
<220>
   <221> misc_feature
   <223> Binder
<400> 39
uaauaauggg guagggcuug gauggguagu acacuagugu gcguggu 47
<210> 40
   <211> 44
   <212> RNA
   <213> Artificial
<220>
   <221> misc_feature
   <223> Binder
<400> 40
uaauggggua gggcuuggau ggguaguaca cuagugugcg uggu 44
<210> 41
   <211> 43
   <212> RNA
   <213> Artificial
<220>
   <221> misc_feature
   <223> Binder
<400> 41
aaugggguag ggcuuggaug gguaguacac uagugugcgu ggu 43
<210> 42
   <211> 42
   <212> RNA
   <213> Artificial
<220>
   <221> misc_feature
   <223> Binder
<400> 42
   augggguagg gcuuggaugg guaguacacu agugugcgug gu 42
<210> 43
   <211> 41
   <212> RNA
   <213> Artificial
<220>
   <221> misc_feature
   <223> Binder
<400> 43
ugggguaggg cuuggauggg uaguacacua gugugcgugg u 41
<210> 44
   <211> 40
   <212> DNA
   <213> Artificial
<220>
   <221> misc_feature
   <223> Binder
<400> 44
ugggguaggg cuuggauggg uaguacacua gugugcgugg 40
<210> 45
   <211> 40
   <212> RNA
   <213> Artificial
<220>
   <221> misc_feature
   <223> Binder
<400> 45
gggguagggc uuggaugggu aguacacuag ugugcguggu 40
<210> 46
   <211> 39
   <212> RNA
   <213> Artificial
<220>
   <221> misc_feature
   <223> Binder
<400> 46
gggguagggc uuggaugggu aguacacuag ugugcgugg 39
<210> 47
   <211> 39
   <212> RNA
   <213> Artificial
<220>
   <221> misc_feature
   <223> Binder
<400> 47
ugggguaggg cuuggauggg uaguacacgu gugcguggu 39
<210> 48
   <211> 38
   <212> RNA
   <213> Artificial
<220>
   <221> misc_feature
   <223> Binder
<400> 48
   ugggguaggg cuuggauggg uaguacacgu gugcgugg 38
<210> 49
   <211> 38
   <212> RNA
   <213> Artificial
<220>
   <221> misc_feature
   <223> Binder
<400> 49
   gggguagggc uuggaugggu aguacacgug ugcguggu 38
<210> 50
   <211> 37
   <212> RNA
   <213> Artificial
<220>
   <221> misc_feature
   <223> Binder
<400> 50
   gggguagggc uuggaugggu aguacacgug ugcgugg 37